(19) Europäisches Patentamt
European Patent Office
Office européen des brevets

(11) Publication number: **0 309 400 B1**

(12) **EUROPEAN PATENT SPECIFICATION**

(45) Date of publication of patent specification: **02.03.94**

(51) Int. Cl.[5]: **C09D 7/12**, C08K 5/34, C07D 211/00, C07D 401/00, C07D 403/00

(21) Application number: **88810621.8**

(22) Date of filing: **13.09.88**

The file contains technical information submitted after the application was filed and not included in this specification

(54) **N-acyloxy hindered amine stabilizers.**

(30) Priority: **21.09.87 US 99419**
**21.09.87 US 99420**
**21.09.87 US 99411**

(43) Date of publication of application:
**29.03.89 Bulletin 89/13**

(45) Publication of the grant of the patent:
**02.03.94 Bulletin 94/09**

(84) Designated Contracting States:
**BE DE ES FR GB IT NL SE**

(56) References cited:
**EP-A- 0 130 945**

**JOURNAL OF POLYMER SCIENCE, POLYMER CHEMISTRY EDITION, vol. 22, no. 1, January 1984, pages 277-281, John Wiley & Sons; T. KURUMADA et al.: "The effect of N-substituents of hindered amine on photo-oxidation of polypropylene"**

(73) Proprietor: **CIBA-GEIGY AG**
**Klybeckstrasse 141**
**CH-4002 Basel(CH)**

(72) Inventor: **Malherbe, Roger, François, Dr.**
**Bleichestrasse 7**
**CH-4058 Basle(CH)**
Inventor: **Winter, Roland, A.E.**
**23 Banksville Road**
**Armonk New York 10504(US)**
Inventor: **Galbo, James P.**
**119 East Hartsdale Avenue**
**Hartsdale New York 10530(US)**
Inventor: **Behrens, Rudolf A.**
**P.O. Box 8213**
**New Fairfield Connecticut 06812(US)**
Inventor: **Mar, Andrew**
**97 Beacon Street**
**Norwalk Connecticut 06851(US)**
Inventor: **Schirmann, Peter J.**
**89 Eunice Avenue**
**Fairfield Connecticut 06430(US)**
Inventor: **Seltzer, Raymond**
**11 Angus Lane**
**New City New York 10956(US)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services
(3.10/3.09/3.3.3)

## Description

The instant invention relates to the stabilization of a wide variety of ambient temperature curable and of acid catalyzed thermosetting coating compositions by the incorporation therein of N-acyloxy-substituted hindered amine light stabilizers as well as to new N-acyloxy hindered amines.

Hindered amine light stabilizers are well known to be effective in stabilizing a host of organic substrates including polymers against the deleterious effects of oxygen and light. Such hindered amine light stabilizers have been used in the stabilization of hot-crosslinkable alkyd or acrylic metallic stoving lacquers (US 4,426,472) and in stabilizing acid-catalyzed stoving lacquers based on hot-crosslinkable acrylic polyester or alkyd resins (US 4,344,876 and 4,426,471). The hindered amine light stabilizers of these patents do not possess structures having an acyloxy group substituted directly on the hindered N-atom of the compound.

EP-A-I3O,945 describes some N-acyloxy substituted hindered amines as light stabilizers for a variety of organic materials including stoving lacquers.

Related hindered amine stabilizers have been utilized individually and in combination with ultra-violet light absorbers to improve the performance characteristics of coating systems. Notwithstanding such improvements, there still exists a need to further retard the photooxidation and photodegradation of such coating systems and thereby provide increased effectiveness by maintaining the physical integrity of the coatings. Such effectiveness can be manifested by prevention of embrittlement, cracking, corrosion, erosion, loss of gloss, chalking and yellowing of the coating.

It has now been determined that the aforementioned improvements can be achieved by substitution on the hindered N-atom of the hindered amines with acyloxy groups and the utilization of such derivatives in ambient temperature curable and acid catalyzed thermosetting coating systems. In particular, the physical integrity of the coatings is maintained to a higher degree with significant reduction in loss of gloss and yellowing. Accordingly, the present invention relates to a stabilized ambient temperature curable or acid catalyzed thermosetting coating composition containing an effective stabilizing amount of a hindered amine compound containing the group

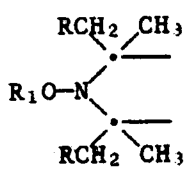

wherein R is hydrogen or methyl and $R_1$ is a group

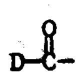 ,

wherein D is $C_1$-$C_{18}$ alkyl, $C_1$-$C_{18}$ alkoxy, phenyl, phenyl substituted by hydroxy, alkyl or alkoxy, or D is amino or amino mono- or disubstituted by alkyl or phenyl.

More particularly, the instant invention relates to the use of a compound having one of formulae A to N

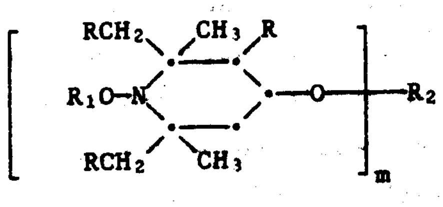 (A)

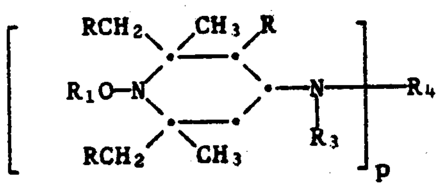 (B)

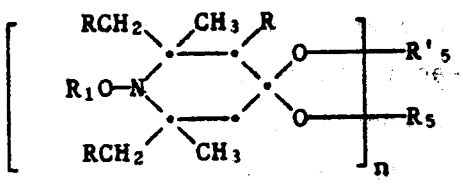 (C)

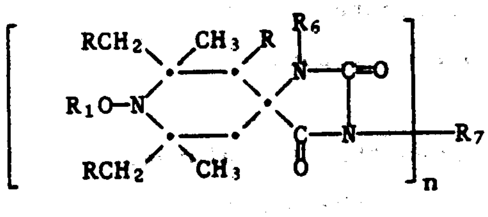 (D)

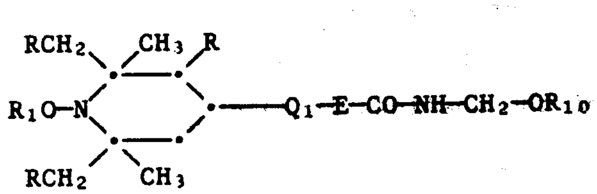 (E)

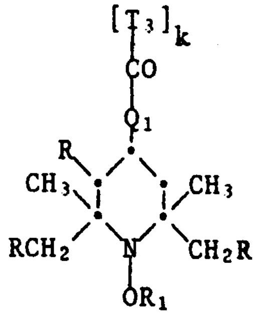

(F)

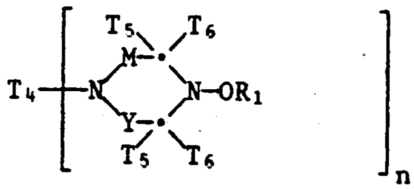

(G)

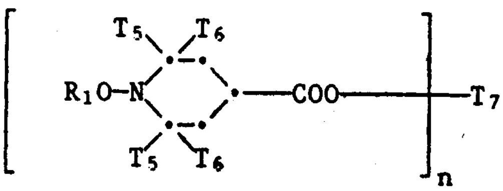

(H)

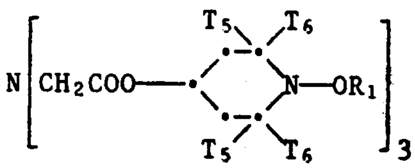

(I)

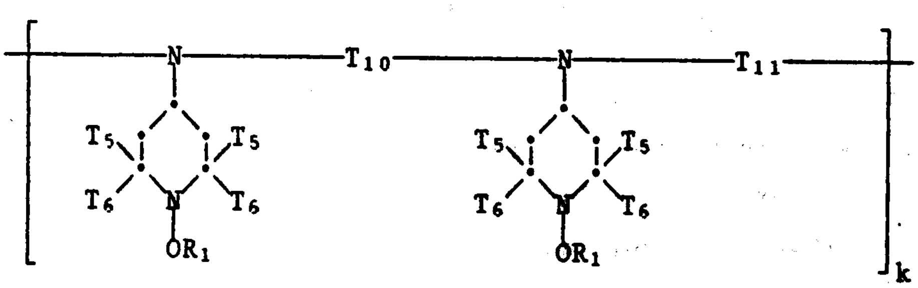

(J)

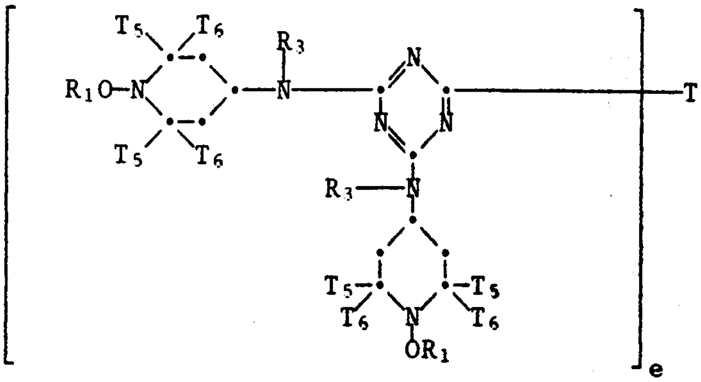

(K)

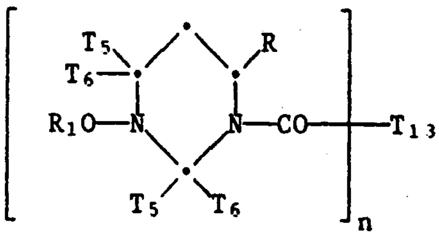

(L)

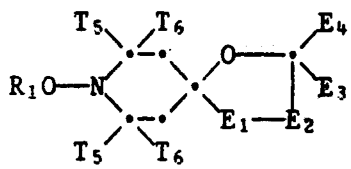

(M)

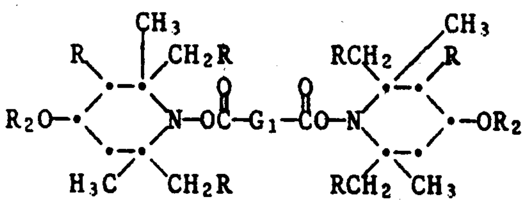

(N)

wherein
R is hydrogen or methyl,
$R_1$ is a group D-CO- , wherein D is $C_1$-$C_{18}$ alkyl, $C_1$-$C_{18}$ alkoxy, phenyl, phenyl substituted by hydroxy, $C_1$-$C_4$ alkyl or $C_1$-$C_4$ alkoxy, or amino or amino mono- or disubstituted by $C_1$-$C_{12}$ alkyl or phenyl;
m is 1-4,
when m is 1,
$R_2$ is hydrogen, $C_1$-$C_{18}$ alkyl optionally interrupted by one or more oxygen atoms, $C_2$-$C_{12}$ alkenyl, $C_6$-$C_{10}$ aryl, $C_7$-$C_{18}$ aralkyl, glycidyl, a monovalent acyl radical of an aliphatic, cycloaliphatic, araliphatic or aromatic carboxylic acid, or of a carbamic acid, preferably an acyl radical of an aliphatic carboxylic acid having 2-18 C atoms, of a cycloaliphatic carboxylic acid having 6-12 C atoms or of an aromatic carboxylic acid having 7-15 C atoms, or $R_2$ is a group

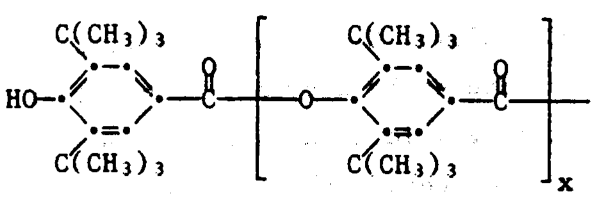

wherein x is 0 or 1, or is a group

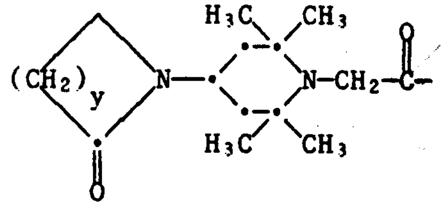

wherein y is 2-4;
when m is 2,
$R_2$ is $C_1$-$C_{12}$ alkylene, $C_4$-$C_{12}$ alkenylene, xylylene, a divalent acyl radical of an aliphatic, cycloaliphatic, araliphatic or aromatic dicarboxylic acid or of a dicarbamic acid, preferably an acyl radical of an aliphatic dicarboxylic acid having having 2-18 C atoms, of an cycloaliphatic or aromatic dicarboxylic acid having 8-14 C atoms, or of an aliphatic, cycloaliphtic or aromatic dicarbamic acid having 8-14 C atoms; or $R_2$ is a group

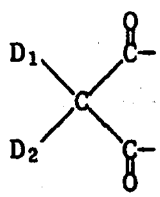

or

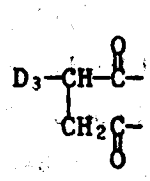

wherein $D_1$ and $D_2$ independently are hydrogen, an alkyl radical containing up to 8 carbon atoms, phenyl, benzyl or 3,5-di-t-butyl-4-hydroxybenzyl, $D_3$ is an alkyl or alkenyl radical containing up to 18 carbon atoms; when m is 3, $R_2$ is a trivalent acyl radical of an aliphatic, cycloaliphatic, or aromatic tricarboxylic acid; when m is 4, $R_2$ is a tetravalent acyl radical of a saturated or unsaturated aliphatic or aromatic tetracarboxylic acid including 1,2,3,4-butanetetracarboxylic acid, 1,2,3,4-but-2-enetetracarboxylic acid, and 1,2,3,5- and 1,2,4,5-pentanetetracarboxylic acid;
p is 1, 2 or 3,
$R_3$ is hydrogen, $C_1$-$C_{12}$ alkyl, $C_5$-$C_7$ cycloalkyl, $C_7$-$C_9$ aralkyl, $C_2$-$C_{18}$ alkanoyl, $C_3$-$C_5$ alkenoyl or benzoyl;
when p is 1,
$R_4$ is hydrogen, $C_1$-$C_{18}$ alkyl, $C_5$-$C_7$ cycloalkyl, $C_2$-$C_8$ alkenyl unsubstituted or substituted by a cyano, carbonyl or carbamide group, or it is aryl, aralkyl, glycidyl, a group of the formula $-CH_2-CH(OH)-Z$ or $-CONH-Z$ wherein Z is hydrogen, methyl or phenyl;
or $R_4$ is a group of the formula I

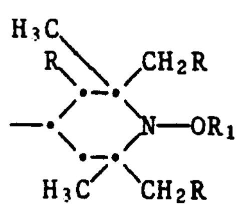

(I) or

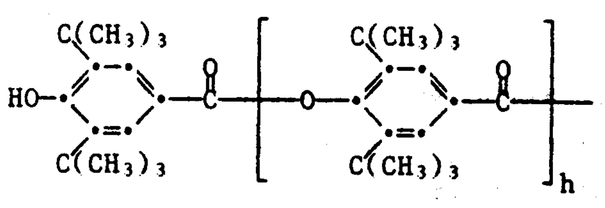

with h as 0 or 1;

or $R_3$ and $R_4$ together are alkylene of 4 to 6 carbon atoms or 1-oxo alkylene or the divalent acyl radical of an aliphatic or aromatic 1,2- or 1,3-dicarboxylic acid;
when p is 2,
$R_4$ is $C_1$-$C_{12}$ alkylene, $C_6$-$C_{12}$ arylene, xylylene, a $-CH_2CH(OH)-CH_2$ group, or a group $-CH_2-CH(OH)-CH_2-O-X-O-CH_2-CH(OH)-CH_2-$ wherein X is $C_2$-$C_{10}$ alkylene, $C_6$-$C_{15}$ arylene or $C_6$-$C_{12}$ cycloalkylene; or, provided that $R_3$ is not alkanoyl, alkenoyl or benzoyl, $R_4$ can also be a divalent acyl radical of an aliphatic, cycloaliphatic or aromatic dicarboxylic or dicarbamic acid, or can be the group -CO-, or $R_4$ is a group of formula II

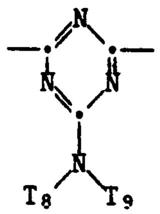

(II)

where $T_8$ and $T_9$ are independently hydrogen, alkyl of 1 to 18 carbon atoms, or $T_8$ and $T_9$ together are alkylene of 4 to 6 carbon atoms or 3-oxapentamethylene, preferably $T_8$ and $T_9$ together are 3-oxapentamethylene;
when p is 3, $R_4$ is 2,4,6-triazinetriyl;
n is 1 or 2 and
when n is 1,
$R_5$ and $R'_5$ are independently $C_1$-$C_{12}$ alkyl, $C_2$-$C_{12}$ alkenyl, $C_7$-$C_{12}$ aralkyl, or $R_5$ is also hydrogen, or $R_5$ and $R'_5$ together are $C_2$-$C_8$ alkylene or hydroxyalkylene or $C_4$-$C_{22}$ acyloxyalkylene;
when n is 2,
$R_5$ and $R'_5$ together are $(-CH_2)_2C(CH_2-)_2$;
$R_6$ is hydrogen, $C_1$-$C_{12}$ alkyl, allyl, benzyl, glycidyl or $C_2$-$C_6$ alkoxyalkyl;
when n is 1,
$R_7$ is hydrogen, $C_1$-$C_{12}$ alkyl, $C_3$-$C_5$ alkenyl, $C_7$-$C_9$ aralkyl, $C_5$-$C_7$ cycloalkyl, $C_2$-$C_4$ hydroxyalkyl, $C_2$-$C_6$ alkoxyalkyl, $C_6$-$C_{10}$ aryl, glycidyl, a group of the formula $-(CH_2)_t-COO-Q$ or of the formula $-(CH_2)_t-O-CO-Q$ wherein t is 1 or 2, and Q is $C_1$-$C_4$ alkyl or phenyl; or
when n is 2,
$R_7$ is $C_2$-$C_{12}$ alkylene, $C_6$-$C_{12}$ arylene, a group $-CH_2CH(OH)-CH_2-O-X-O-CH_2-CH(OH)-CH_2-$ wherein X is $C_2$-$C_{10}$ alkylene, $C_6$-$C_{15}$ arylene or $C_6$-$C_{12}$ cycloalkylene, or a group $-CH_2CH(OZ')CH_2-(OCH_2-CH(OZ')-CH_2)_2-$ wherein Z' is hydrogen, $C_1$-$C_{18}$ alkyl, allyl, benzyl, $C_2$-$C_{12}$ alkanoyl or benzoyl;
$Q_1$ is $-N(R_8)-$ or -O-;
E is $C_1$-$C_3$ alkylene, the group $-CH_2-CH(R_9)-O-$ wherein $R_9$ is hydrogen, methyl or phenyl, or E is the group $-(CH_2)_3-NH-$ or a direct bond;
$R_{10}$ is hydrogen or $C_1$-$C_{18}$ alkyl;
$R_8$ is hydrogen, $C_1$-$C_{18}$ alkyl, $C_5$-$C_7$ cycloalkyl, $C_7$-$C_{12}$ aralkyl, cyanoethyl, $C_6$-$C_{10}$ aryl, the group $-CH_2-CH(R_9)-OH$ wherein $R_9$ has the meaning defined above; a group of the formula I
or a group of the formula

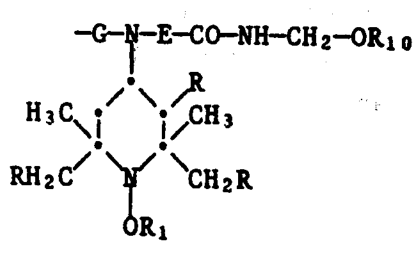

wherein G is $C_2$-$C_6$ alkylene or $C_6$-$C_{12}$ arylene; or $R_8$ is a group $-E-CO-NH-CH_2-OR_{10}$;
Formula F denotes a recurring structural unit of a polymer where $T_3$ is ethylene or 1,2-propylene, or is the repeating structural unit derived from an alpha-olefin copolymer with an alkyl acrylate or methacrylate; preferably a copolymer of ethylene and ethyl acrylate, and where k is 2 to 100;
$T_4$ has the same meaning as $R_4$ when p is 1 or 2,

$T_5$ is methyl,
$T_6$ is methyl or ethyl, preferably $T_5$ and $T_6$ are each methyl,
M and Y are independently methylene or carbonyl, preferably M is methylene and Y is carbonyl, and $T_4$ is ethylene where n is 2;
$T_7$ is the same as $R_7$, and $T_7$ preferably octamethylene where n is 2;
$T_{10}$ and $T_{11}$ are independently alkylene of 2 to 12 carbon atoms, or $T_{11}$ is a group of formula II;
e is 2, 3 or 4 and
$T_{12}$ is a group $-N(R_5)-(CH_2)_d-N(R_5)-$ or

$$-NH(CH_2)_a-\overset{|}{N}(CH_2)_b-\overset{|}{N}[(CH_2)_c-\overset{|}{N}]_fH$$

where a, b and c are independently 2 or 3, d is 2 to 10 and f is 0 or 1, preferably a and c are each 3, b is 2 and f is 1;
$T_{13}$ is the same as $R_4$ with the proviso that $T_{13}$ cannot be hydrogen when n is 1;
$E_1$ and $E_2$, being different, each are -CO- or $-N(E_5)-$, where $E_5$ is hydrogen, $C_1$-$C_{12}$ alkyl or alkoxycarbonylalkyl of 4 to 22 carbon atoms, preferably $E_1$ is -CO- and $E_2$ is $-N(E_5)-$ ;
$E_3$ is hydrogen, alkyl of 1 to 30 carbon atoms, phenyl, naphthyl, said phenyl or said naphthyl substituted by chlorine or by alkyl of 1 to 4 carbon atoms, or phenylalkyl of 7 to 12 carbon atoms, or said phenylalkyl substituted by alkyl of 1 to 4 carbon atoms,
$E_4$ is hydrogen, alkyl of 1 to 30 carbon atoms, phenyl, naphthyl or phenylalkyl of 7 to 12 carbon atoms, or
$E_3$ and $E_4$ together are polymethylene of 4 to 17 carbon atoms, or said polymethylene substituted by up to four alkyl groups of 1 to 4 carbon atoms, preferably methyl, and
$R_2$ of formula (N) is as previously defined when m is 1, $G_1$ is a direct bond, $C_1$-$C_{12}$ alkylene, phenylene or -NH-G'-NH wherein G' is $C_1$-$C_{12}$ alkylene.

In the structures A to N, if any substituents are $C_1$-$C_{18}$ alkyl, they are for example methyl, ethyl, n-propyl, n-butyl, sec-butyl, tert-butyl, n-hexyl, n-octyl, 2-ethylhexyl, n-nonyl, n-decyl, n-undecyl, n-dodecyl, n-tridecyl, n-tetradecyl, n-hexadecyl or n-octadecyl.

If $R_2$ is a monovalent acyl radical of a carboxylic acid, it is for example an acyl radical of acetic acid, stearic acid, salicylic acid, methacrylic acid, benzoic acid or $\beta$-(3,5-di-tert-butyl-4-hydroxyphenyl)-propionic acid.

If $R_2$ is a divalent acyl radical of a dicarboxylic acid, it is for example an acyl radical of adipic acid, succinic acid, suberic acid sebacic acid, phthalic acid, maleic acid, dibutylmalonic acid, dibenzylmalonic acid or butyl-(3,5-di-tert-butyl-4-hydroxybenzyl)-malonic acid, or bicycloheptenedicarboxylic acid.

If $R_2$ is a divalent acyl radical of a dicarbamic acid, it is for example an acyl radical of hexamethylenedicarbamic acid or of 2,4-toluylenedicarbamic acid.

As $C_7$-$C_9$ aralkyl, $R_3$ is particularly phenetyl or above all benzyl.

As $C_2$-$C_{18}$ alkanoyl, $R_3$ is for example propionyl, butyryl, octanoyl, dodecanoyl, hexadecanoyl, octadecanoyl, but preferably acetyl; and a $C_3$-$C_5$ alkenoyl, $R_3$ is in particular acryloyl.

If $R_4$ is $C_2$-$C_8$ alkenyl unsubstituted or substituted by a cyano, carbonyl or carbamide group, it is for example 1-propenyl, allyl, methallyl, 2-butenyl, 2-pentenyl, 2-hexenyl, 2-octenyl, 2,2-dicyanovinyl, 1-methyl-2-cyano-2-methoxycarbonyl-vinyl or 2,2-diacetylaminovinyl.

If any substituents are $C_2$-$C_{12}$ alkylene, they are for example ethylene, propylene, 2,2-dimethylpropylene, tetramethylene, hexamethylene, octamethylene, decamethylene or dodecamethylene.

If any substituents are $C_6$-$C_{15}$ arylene, they are for example o-, m- or p-phenylene, 1,4-naphthylene or 4,4'-diphenylene.

As $C_6$-$C_{12}$ cycloalkylene, X is especially cyclohexylene.

If $R_5$ is $C_2$-$C_8$ alkylene or hydroxyalkylene, it is for example ethylene, 1-methyl-ethylene, propylene, 2-ethylpropylene or 2-ethyl-2-hydroxymethylpropylene.

As $C_4$-$C_{22}$ acyloxyalkylene, $R_5$ is for example 2-ethyl-2-acetoxymethylpropylene.

If any substituents are $C_2$-$C_6$ alkoxyalkyl, they are for example methoxymethyl, ethoxymethyl, propoxymethyl, tert-butoxyethyl, ethoxyethyl, ethoxypropyl, n-butoxyethyl, tert-butoxyethyl, isopropoxyethyl or propoxypropyl.

If $R_7$ is $C_3$-$C_5$ alkenyl, it is for example 1-propenyl, allyl, methallyl, 2-butenyl or 2-pentenyl.

As $C_7$-$C_9$ aralkyl, $R_7$ is in particular phenethyl or above all benzyl; and as $C_5$-$C_7$ cycloalkyl, $R_7$ is especially cyclohexyl.

If $R_7$ is $C_2$-$C_4$ hydroxyalkyl, it is for example 2-hydroxyethyl, 2-hydroxypropyl, 3-hydroxypropyl, 2-hydroxybutyl or 4-hydroxybutyl.

As $C_6$-$C_{10}$ aryl, $R_7$ is in particular phenyl, or alpha- or $\beta$-naphthyl which is unsubstituted or substituted by halogen or $C_1$-$C_4$ alkyl.

If $R_7$ is $C_2$-$C_{12}$ alkylene, it is for example ethylene, propylene 2,2-dimethylpropylene, tetramethylene, hexamethylene, octamethylene, decamethylene or dodecamethylene.

If $R_7$ is $C_6$-$C_{12}$ arylene, it is for example o-, m- or p-phenylene, 1,4-naphthylene or 4,4'-diphenylene.

If Z' is $C_2$-$C_{12}$ alkanoyl, it is for example propionyl, butyryl, octanoyl, dodecanoyl or preferably acetyl.

As $C_5$-$C_7$ cycloalkyl, $R_8$ is in particular cyclohexyl.

As $C_6$-$C_{10}$ aryl, $R_8$ is particularly phenyl, or alpha- or $\beta$-naphthyl which is unsubstituted or substituted with halogen or $C_1$-$C_4$ alkyl.

As $C_1$-$C_3$ alkylene, E is for example methylene, ethylene or propylene.

As $C_2$-$C_6$ alkylene, G is for example ethylene, propylene, 2,2-dimethylpropylene, tetramethylene or hexamethylene; and as $C_6$-$C_{12}$ arylene, G is o-, m- or p-phenylene, 1,4-naphthylene or 4,4'-diphenylene.

Preferred hindered amine compounds are the compounds of formulae A, B, D, J, K or M wherein R is hydrogen and $T_5$ and $T_6$ are methyl.

Further preferred are the compounds of formula A wherein R is hydrogen,
$R_1$ is a group D-CO- and D is $C_1$-$C_{12}$ alkyl, $C_1$-$C_4$ alkoxy, phenyl, amino or amino monosubstituted by $C_1$-$C_{12}$ alkyl or phenyl,
m is 1, 2 or 4 and
when m is 1,
$R_2$ is an acyl radical of an aliphatic $C_2$-$C_{18}$ carboxylic acid, of a cycloaliphatic $C_6$-$C_{12}$ carboxylic acid or of an aromatic $C_7$-$C_{15}$ carboxylic acid and when m is 2, $R_2$ is a divalent acyl radical of an aliphatic $C_2$-$C_{18}$ dicarboxylic acid or of a cycloaliphatic or aromatic $C_8$-$C_{14}$ dicarboxylic acid, or of an aliphatic, cycloaliphatic or aromatic $C_8$-$C_{14}$ dicarbamic acid, or $R_2$ is a group

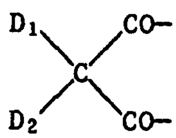

wherein $D_1$ is $C_1$-$C_8$ alkyl or 3,5-di-tert.-butyl-4-hydroxybenzyl and $D_2$ is $D_1$ or hydrogen and when m is 4, $R_2$ is a tetravalent acyl radical of a butane- or pentane-tetracarboxylic acid.

Further preferred are the compounds of formula B wherein R is hydrogen,
$R_1$ is a group D-CO- and D is $C_1$-$C_{12}$ alkyl, $C_1$-$C_4$ alkoxy, phenyl, amino or amino monosubstituted by $C_1$-$C_{12}$ alkyl or phenyl,
p is 1 or 2, $R_3$ is hydrogen, $C_1$-$C_{12}$ alkyl or $C_2$-$C_{12}$ alkanoyl and when p is 1, $R_4$ is $C_1$-$C_{12}$ alkyl, $C_5$-$C_7$ cycloalkyl or a group of formula I, and when p is 2, $R_4$ is $C_2$-$C_8$ alkylene, phenylene or xylylene, and if $R_3$ is not alkanoyl, $R_4$ may also be a divalent acyl residue of an aliphatic $C_2$-$C_{10}$ dicarboxylic acid or of an aromatic $C_6$ dicarboxylic acid or of an aliphatic or aromatic $C_8$-$C_{15}$ dicarbamic acid.

The following compounds are examples of hindered amine derivatives applicable for use in the invention:

1. di-(1-acetoxy-2,2,6,6-tetramethylpiperidin-4-yl) phthalate
2. di-(1-acetoxy-2,2,6,6-tetramethylpiperidin-4-yl) isophthalate
3. di-(1-acetoxy-2,2,6,6-tetramethylpiperidin-4-yl) sebacate
4. di-(1-acetoxy-2,2,6,6-tetramethylpiperidin-4-yl) succinate
5. di-(1-acetoxy-2,2,6,6-tetramethylpiperidin-4-yl) adipate
6. di-(1-acetoxy-2,2,6,6-tetramethylpiperidin-4-yl) n-butylmalonate
7. di-(1-acetoxy-2,2,6,6-tetramethylpiperidin-4-yl) 2,2-diethylmalonate
8. poly-{[6-[(1,1,3,3-tetramethylbutyl)-imino]-1,3,5-triazine-2,4-diyl]-[2-(1-acetoxy-2,2,6,6-tetramethylpiperidyl)-imino]-hexamethylene-[4-(1-acetoxy-2,2,6,6-tetramethylpiperidyl)-imino]}
9. 1,4-diacetoxy-2,2,6,6-tetramethylpiperidine
10. 1,4-acetoxy-4-hydroxy-2,2,6,6-tetramethylpiperidine
11. 1,4-acetoxy-4-acryloxy-2,2,6,6-tetramethylpiperidine
12. polymer of 11
13. (1-acetoxy-2,2,6,6-tetramethylpiperidin-4-yl)4-hydroxy-3,5-di-tert.butylbenzoate
14. di-(1-acetoxy-2,2,6,6-tetramethylpiperidin-4-yl) 2-(4-hydroxy-3,5-di-tert.butylbenzyl)-n-butylmalonate

15. N-(1-acetoxy-2,2,6,6-tetramethylpiperidin-4-yl)-N-(n-butyl)-4-(4-hydroxy-3,5-di-tert.butylbenzoyloxy)-3,4-di-tert.butylbenzamide
16. 1,6-di-[N-acetyl-N-(1-acetoxy-2,2,6,6-tetramethylpiperidin-4-yl)-amino]hexane
17. di-(1-acetoxy-2,2,6,6-tetramethylpiperidin-4-yloxy)-hexane-1,6-dicarbamate
18. 1-acetoxy-4-(N-acetyl-N-n-dodecylamino)-2,2,6,6-tetramethylpiperidine
19. di-(1-propionoxy-2,2,6,6-tetramethylpiperidin-4-yl) succinate
20. di-(4-n-octadecanoyloxy-2,2,6,6-tetramethylpiperazin-1-yl) oxalate
21. 1,4-di-(2-ethylhexanoyloxy)-2,2,6,6-tetramethylpiperidine
22. di-(1-benzoyloxy)-2,2,6,6-tetramethylpiperidin-4-yl) sebacate
23. 1-benzoyloxy-4-(N-n-butyl-N-benzoylamino)-2,2,6,6-tetramethylpiperidine
24. 1-(1-benzoyloxy)-2,2,6,6-tetramethylpiperidin-4-yl)-azepin-2-one
25. [1-benzoyloxy-1′-benzyloxy-di-(2,2,6,6-tetramethylpiperidin-4-yl)]-isophthalate
26. 1,4-di-(4-hydroxy-3,5-di-tert-butylbenzoyloxy)-2,2,6,6-tetramethylpiperidine
27. n-butyl-(4-benzoyloxy-2,2,6,6-tetramethylpiperidin-4-yl) carbonate
28. 1-carbamoyloxy-4-benzoyloxy-2,2,6,6-tetramethylpiperidine
29. di(1-carbamoyloxy-2,2,6,6-tetramethylpiperidin-4-yl) sebacate
30. di-(1-n-butylcarbamoyloxy-2,2,6,6-tetramethylpiperidin-4-yl)- 2,2-diethylmalonate
31. di-(4-benzoyloxy-2,2,6,6-tetramethylpiperidin-1-yl)-2,4,4-trimethylhexane-1,6-dicarbamate
32. di-(1-carbamoyloxy-2,2,6,6-tetramethylpiperidin-4-yl) n-butylmalonate
33. di-(1-n-butylcarbamoyloxy-2,2,6,6,-tetramethylpiperidin-4-yl) phthalate
34. di-(1-n-butylcarbamoyloxy-2,2,6,6-tetramethylpiperidin-4-yl) butylmalonate
35. di-(1-phenylcarbamoyloxy-2,2,6,6-tetramethylpiperidin-4-yl) sebacate.

The coating compositions of this invention may be ambient curable or acid catalyzed hot curable systems, depending from the type of binder resins of the composition.

Resins for ambient curable coatings may be, for example, alkyd resins, thermoplastic acrylic resins, acrylic alkyd resins, polyurethane resins, or polyester resins, said resins may be modified with silicons, isocyanates, epoxides, isocyanurates, ketimines or oxazolidines. The resins may be esters of cellulose such as nitrocellulose or cellulose acetobutyrate or the resins may be epoxide resins hardenable with polyamines or other hardeners.

Applicable alkyd, acrylic, polyester und epoxide resins are described in S. Paul's "Surface Coatings: Science and Technology" (1985) at pages 70-310. The unmodified and modified alkyd resins which can be stabilized in accordance with the invention, are the conventional resins which are used in trade sales, maintenance and automative refinish coatings. For example, such coatings are based on alkyd resins, alkyd/acrylic resins and alkyd/silicon resins optionally crosslinked by isocyanates or epoxy resins.

In addition various acrylic lacquer coating compositions are disclosed in U.S. 4,168,249. Other acrylic/alkyd resins with polyisocyanate additives are disclosed in U.S. 4,471,083; and acrylic resins containing either pendant amino ester groups or glycidyl groups are described in U.S. 4,525,521.

Resins for acid catalyzed thermosetting coatings may be, for example, hot crosslinkable acrylic, polyester, polyurethane, polyamide or alkyd resins. This implies mixtures of those resins or mixtures with cross-linking agents such as melamine resins.

The acrylic resin lacquers are the conventional acrylic resin stoving lacquers or thermosetting resins including acrylic/melamine systems which are described, for example, in H. Kittel's "Lehrbuch der Lacke und Beschichtungen", Vol. 1, Part 2, on pages 735 and 742 (Berlin 1972), "Lackkunstharze" (1977), by H. Wagner und H.F. Sarx, on pages 229-238, and in S. Paul's "Surface Coatings: Science and Technology" (1985).

The polyester lacquers are the conventional stoving lacquers described e.g. in H. Wagner and H.F. Sarx, op. cit., on pages 86-99.

The alkyd resin lacquers which can be stabilized in accordance with the invention, are the conventional stoving lacquers which are used in particular for coating automobiles (automobile finishing lacquers), for example lacquers based on alkyd/melamine resins and alkyd/acrylic/melamine resins (see H. Wagner and H.F. Sarx, op. cit., pages 99-123). Other crosslinking agents include glycoluril resins, blocked isocyanates or epoxy resins.

In their industrial uses, enamels with high solids content based on crosslinkable acrylic, polyester, urethane or alkyd resins are cured with an additional acid catalyst. Light stabilizers containing a basic nitrogen group are generally less than satisfactory in this application. Formation of a salt between the acid catalyst and the light stabilizer leads to incompatibility or insolubility and precipitation of the salt and to a reduced level of cure and to reduced light protective action and poor resistance to moisture.

The ambient temperature curable coatings are well as the acid catalyzed hot curable coatings stabilized in accordance with the invention are suitable both for metal finish coatings and solid shade finishes, especially in the case of retouching finishes. The lacquers stabilized in accordance with the invention are preferably applied in the conventional manner by two methods, either by the single-coat method or by the two-coat method. In the latter method, the pigment-containing base coat is applied first and a covering coat of clear lacquer applied over it.

The amount of hindered amine derivative employed is 0.1 to 10 % by weight, based on the solvent-free binder, preferably 0.5 to 5 % by weight. The binders can be dissolved or dispersed in customary organic solvents or in water or can be solvent-free.

When used in two-coat finishes, the hindered amine derivative can be incorporated in the clear coat or both in the clear coat and in the pigmented base coat. In the manufacture of acrylic modified alkyd resins or acrylic resins, polymerizable hindered amine derivatives can be polymerized into the resin. The incorporation into the lacquer binder can also, however, be effected via polycondensation in the manufacture of the alkyd or polyester resins. In these cases, there is the additional advantage that the light stabilizers cannot be removed by extraction or migration so that their action is very prolonged.

To attain maximum light stability, the concurrent use of other conventional light stabilizers can be advantageous. Examples are UV absorbers of the benzophenone, benztriazole, acrylic acid derivative, or oxalanilide type, or aryl-s-triazines or metal-containing light stabilizers, for example organic nickel compounds. In two-coat systems, these additional light stabilizers can be added to the clear coat and/or the pigmented base coat.

If such combinations are employed, the sum of all light stabilizers is 0.2 to 20 % by weight, preferably 0.5 to 5 % by weight, based on the film-forming resin.

Examples of the UV absorbers which may be used in the instant compositions in conjunction with the aforementioned hindered amine compounds are:

(a) 2-(2′-Hydroxyphenyl)-benzotriazoles, for example the 5′-methyl-, 3′,5′-di-tert-butyl-, 5′-tert-butyl-, 5′-(1,1,3,3-tetramethylbutyl)-5-chloro-3′,5′-di-tert-butyl-, 5-chloro-3′-tert-butyl-5′-methyl-, 3′-sec-butyl-5′-tert-butyl-, 4′-octoxy-, 3′,5′-di-tert-amyl derivative.

(b) 2-Hydroxy-benzophenones, for example, the 4-hydroxy-, 4-methoxy-4-octoxy-, 4-decyloxy-, 4-dodecyloxy-, 4-benzyloxy, 4,2′,4′-trihydroxy-and 2′-hydroxy-4,4′-dimethoxy derivative.

(c) Acrylates, for example, alpha-cyano-β,β-diphenyl-acrylic acid ethyl ester or isoctyl ester, alpha-carbomethoxy-cinnamic acid methyl ester, alpha-cyano-β-methyl-p-methoxy-cinnamic acid methyl ester or butyl ester, alpha-carbomethoxy-p-methoxy-cinnamic acid methyl ester, N-(β-carbomethoxy-β-cyanovinyl)-2-methyl-indoline.

(d) Nickel compounds, for example, nickel complexes of 2,2′-thiobis-[4-(1,1,3,3-tetramethylbutyl)-phenol], such as the 1:1 or 1:2 complex, optionally with additional ligands such as n-butylamine, triethanolamine or N-cyclohexyl-di-ethanolamine, nickel dibutyldithiocarbamate, nickel salts of 4-hydroxy-3,5-di-tert-butylbenzylphosphonic acid monoalkyl esters, such as of the methyl, ethyl or butyl ester, nickel complexes of ketoximes such as of 2-hydroxy-4-methyl-phenyl undecyl ketonoxime, nickel complexes of 1-phenyl-4-lauroyl-5-hydroxy-pyrazol, optionally with additional ligands.

(e) Oxalic acid diamides, for example 4,4′-di-octyl-oxyoxanilide, 2,2′-di-octyloxy-5,5′-di-tert-butyl-oxanilide, 2,2′-di-dodecyloxy-5,5′-di-tert-butyl-oxanilide, 2-ethoxy-2′-ethyl-oxanilide, N-N′-bis-(3-dimethylaminopropyl)-oxalamide, 2-ethoxy-5-tert-butyl-2′-ethyl-oxanilide and its mixture with 2-ethoxy-2′-ethyl-5,4′-di-tert-butyl-oxanilide, and the mixtures of ortho- and paramethoxy- as well as of o- and p-ethoxy-disubstituted oxanilides.

(f) Hydroxyphenyl-5-triazines such as 2,6-bis-(2,4-di-methylphenyl)-4-(2-hydroxy-4-octyloxyphenyl)-s-triazine or the corresponding 4-(2,4-dihydroxyphenyl) derivatives.

Of particular value in the instant compositions are the benzotriazoles of high molecular weight and low volatility such as 2-[2-hydroxy-3,5-di(alpha,alpha-dimethylbenzyl)-phenyl]-benzotriazole, 2-(2-hydroxy-3,5-di-tert-octylphenyl]-benzotriazole, 2-(2-hydroxy-3-alpha,alpha-dimethylbenzyl-5-tert-octylphenyl)-benzotriazole, 2-(2-hydroxy-3-tert-octyl-5,alpha,alpha-dimethylbenzylphenyl)-benzotriazole, 2-(2-hydroxy-3,5-di-tert-amylphenyl)-benzotriazole, 2-[2-hydroxy-3-tert.butyl-5-(2-omega-hydroxy-octa(ethyleneoxy)-carbonyl)-ethylphenyl]-benzotriazole, 2-[2-hydroxy-3-tert.butyl-5-(2-octyloxycarbonyl-ethyl)-phenyl]-benzotriazole, 2-(2-hydroxy-3-sec.dodecyl-5-methyl-phenyl)-benzotriazole, hexamethylene di[β-(3-tert.butyl-4-hydroxy-5-[2-benzotriazolyl]-phenyl)-propionate] and the 5-chloro compounds corresponding to each of the above named benzotriazoles.

Most preferably the benzotriazoles useful in the instant compositions are 2-[2-hydroxy-3,5-di-(alpha,alpha-dimethylbenzyl)-phenyl]-benzotriazoleand 2-[2-hydroxy-3-tert.butyl-5-(2-omega-hydroxy-octa-(ethyleneoxy)-carbonyl)-ethylphenyl]-benzotriazole.

Further ingredients which the enamels or coatings can contain are antioxidants, for example those of the sterically hindered phenol derivative type, phosphorus compounds, such as phosphites, phosphines or phosphonites, conventional hindered amine light stabilizers, plasticizers, levelling assistants, hardening catalysts, thickeners, dispersants or adhesion promoters.

The stabilizers are needed to impart greater retention of durability to the cured enamels (as measured by 20° gloss, distinction of image, cracking or chalking); the enamel should not yellow on curing and further color change on exposure to light should be minimized; the stabilizers should be soluble in the organic solvents normally used in coating applications such as methyl amyl ketone, xylene, n-hexyl acetate, alcohol and the like.

The instant hindered amine light stabilizers substituted on the hindered N-atom by a acyloxy group fulfill each of these requirements and provide individually or in combination with a UV-absorber outstanding light stabilization protection to the cured coatings.

The following examples describe the inventive use of substituted hindered amine derivatives in various ambient curable and acid catalyzed hot curable resin systems. Parts and percentages are by weight.

Example 1 Stabilization of a Tung Oil Phenolic Varnish

Pieces of 1.27 cm x 30.32 cm x 30.48 cm western red cedar panels having a fine radial cut are used to test a commercially available tung oil phenolic varnish (supplied by McCloskey). One half of each panel is coated with two coats of the unstabilized varnish. An equal amount of varnish containing 5 % (by weight based on resins solids) of light stabilizers is applied to the other half of the panel in two coats. After storage for 2 weeks at ambient temperature, the wood panels are exposed outdoors at an angle of 45°S for a period of 8 months. The 60° gloss of each half of the panel is measured at the top, middle, and bottom portion of the panel and averaged (ASTM D 523). Due to the lack of homogeneity of wood substrates, the gloss retention of the same varnish tends to differ slightly from panel to panel. Thus, the application of an unstabilized control varnish to every panel allows for a more meaningful measurement of the improvement in gloss due to the presence of the light stabilizer.

| Compound | 60° Gloss Retention (%) | | |
|---|---|---|---|
| | Unstabilized | Stabilized | Gloss Improvement |
| 3 | 24.2 | 46.7 | 22.5 |
| A | 28.9 | 39.0 | 10.1 |
| A = bis(1,2,2,6,6-pentamethyl-4-piperidinyl)sebacate | | | |

Example 2 Stabilization of a White Two-Component Polyester Urethane Gloss Enamel

A white polyurethane was formulated as shown below.

| Component I | Parts |
|---|---|
| Desmophen® 670-90 (polyester polyol from Mobay Corp.) | 132.4 |
| Titanium Dioxide | 198.6 |
| Cellosolve Acetate | 98.9 |
| Sand Mill | |
| Desmophen® 670-90 | 94.98 |
| Flow Aid | 0.28 |
| Tertiary Amine | 0.015 |
| Cellosolve Acetate | 332.6 |
| **Component II** | |
| Desmodur® N-100 (polyisocyanate from Mobay Corp.) | 93.9 |
| Cellosolve Acetate | 58.7 |

This material is spray applied at a dry film thickness of 40-50 µm onto Bonderite 40 cold rolled steel panels that have been previously primed with a commercial epoxy polyamide maintenance primer (Sherwin-Williams Tile Clad II). Prior to application, the indicated amounts of hindered amine derivatives (based on resin solids) are added to the paint. After ambient storage for a period of two weeks, three panels of each formulation are exposed outdoors at an angle of 45°S for a period of 9 months. Thereafter, the 20° gloss retention is determined (ASTM D 523-80) at the top, middle and bottom portions of each panel. Thus, the average values for nine gloss retention measurements for each triplicate set of panels are reported below.

| Additive | Conc. (% by weight) | 20° Gloss Retention (%) |
|---|---|---|
| A | 1 | 55 |
| 3 | 1 | 67 |
| 26 | 1 | 69 |

Example 3 Stabilization of a Medium Oil Alkyd Enamel

A medium oil alkyd enamel pigmented with non-leafing aluminum pigment and tinted light blue is stabilized with the indicated amounts of ultraviolet light absorber and hindered amine derivative, and then spray applied onto cold rolled steel panels primed with an epoxy primer. After the coating is allowed to cure at room temperature for 2 weeks, the panels are exposed for accelerated weathering in a Xenon Arc Weatherometer for 840 hours. The 20° gloss values of the panels are determined before and after exposure and indicated below in terms of % gloss retention.

| Additive | Conc. (% by weight) | 20° Gloss Retention (%) |
|---|---|---|
| B/A | 3/2 | 18.9 |
| B/1 | 3/2 | 26.4 |
| B = 2-[2-hydroxy-3-tert.butyl-5-(2-(omega-hydroxy-octa-(ethyleneoxy)-carbonyl-ethylphenyl]-benzotriazole | | |

Example 4 Stabilization of an Acrylic Alkyd Crosslinked with an Aliphatic Isocyanate Refinish Enamel

A silver metallic acrylic alkyd enamel hardened with an aliphatic isocyanate is stabilized with the indicated amounts of ultraviolet light absorber and hindered amine derivative (by weight on total resin solids) and then spray applied onto Bonderite 40 panels primed with a black alkyd primer. After the coatings are aged at ambient temperature for 2 weeks, the panels are exposed in a Xenon Arc Weatherometer for 1550 hours. The 20° gloss values of the unexposed and exposed panels are determined and reported below in terms of % gloss retention.

| Additive | Conc. (% by weight) | 20° Gloss Retention (%) |
|---|---|---|
| B/A | 3/2 | 20.7 |
| B/3 | 3/2 | 25.7 |
| B/22 | 3/2 | 24.3 |

Example 5

The following alkyd paint formulations are prepared.

| | White(W) | Yellow(Y) | Blue(B) |
|---|---|---|---|
| Aroplaz® 1445 M-50 (alkyd resin from NL Industries) | 45.0 | 57.73 | 58.27 |
| $TiO_2$ | 45.0 | 20.82 | 4.26 |
| Irgalite® GS (yellow pigment from CIBA-GEIGY) | - | 7.3 | - |
| Phthalocyanine blue | - | - | 1.41 |
| Ketoxime (antiskinning agent) | 0.17 | 0.07 | 0.014 |
| Ionic antifloat compound | - | - | 0.05 |
| 24 % Pb as naphthenate | 0.94 | 1.30 | 1.30 |
| 6 % Co as naphthenate | 0.36 | 0.50 | 0.50 |
| 6 % Mn as naphthenate | 0.45 | 0.60 | 0.60 |
| Xylene | 35.0 | 48.4 | 45.1 |
| Mineral Spirits | 60.0 | 64.49 | 64.11 |

The formulations are stabilized with the indicated materials in the indicated concentrations (by weight on total resin solids) and sprayed onto cold rolled steel panels primed with an electrocoated epoxy primer. The coating is allowed to cure overnight at room temperature and the panels are then exposed in Florida at an angle of 45° South. 60° gloss, distinciton of image (DI) (Hunter Associates Apparatus) and color change based on Yellowness Index values are determined and tabulated below.

| Additive | Conc.(% by wt.) | Paint | Florida Exposure (Months) 60° Gloss 0 | 9 | 12 | 15 | DI 0 | 9 | 12 | 15 | Color Change 9 | 12 | 15 |
|---|---|---|---|---|---|---|---|---|---|---|---|---|---|
| - | - | W | 61 | 30 | 29 | 30 | 63 | 6 | 18 | 25 | 3.0 | 2.7 | 4.0 |
| B/A | 1.5/1.5 | W | 61 | 28 | 27 | 35 | 55 | 8 | 7 | 19 | 4.2 | 4.4 | 5.4 |
| B/3 | 1.5/1.5 | W | 63 | 43 | 34 | 43 | 80 | 52 | 24 | 38 | 3.5 | 3.9 | 4.8 |
| - | - | Y | 81 | 30 | 15 | 14 | 84 | 78 | 12 | 7 | 11.0 | 12.0 | 13.0 |
| B/A | 1.5/1.5 | Y | 83 | 41 | 25 | 21 | 78 | 58 | 36 | 12 | 8.9 | 11.0 | 12.0 |
| B/3 | 1.5/1.5 | Y | 83 | 50 | 31 | 25 | 82 | 73 | 60 | 24 | 8.0 | 10.0 | 12.0 |
| - | - | B | 86 | 33 | 15 | 11* | 84 | 61 | 10 | 0 | 7.6 | 11.0 | 10.0 |
| B/A | 1.5/1.5 | B | 83 | 63 | 58 | 44 | 80 | 69 | 68 | 59 | 2.0 | 2.6 | 3.3 |
| B/3 | 1.5/1.5 | B | 85 | 63 | 54 | 45 | 86 | 79 | 78 | 64 | 2.0 | 4.0 | 4.3 |

* = cracking observed

Example 6 Stabilization of a Thermoplastic Acrylic Lacquer

A commercially available light blue metallic thermoplastic acrylic lacquer is stabilized with 2 % each of UV absorber and hindered amine (by weight on total resin solids) and then spray applied onto Bonderite 40 panels primed with an alkyd primer. After storage at ambient temperature for 2 weeks, the panels are exposed in an Xenon Arc Weatherometer for 1250 hours. The 20° gloss retention of the panels are reported below.

| Additive | 20° Gloss Retention (%) |
|---|---|
| C/A | 11 |
| C/3 | 29 |
| C/19 | 35 |
| C/29 | 40 |
| C = 2-(2-hydroxy-3,5-di(alpha,alpha-dimethylbenzyl)phenyl)-benzotriazole | |

Example 7: Stabilization of a High Solids Acid-catalyzed, Thermoset Acrylic Resin Enamel

A high solids (50 % by weight) thermoset acrylic resin enamel, catalyzed by 0.5 % by weight of p-toluenesulfonic acid, based on the film-forming resin, is stabilized by the addition of various derivatives of the instant invention.

The high solids thermoset acrylic resin enamel formulation (Acryloid® AT 400 from Rohm and Haas) is based on hydroxyethyl methacrylate, methyl methacrylate, styrene, butyl acrylate and butyl methacrylate and a melamine curing agent.

Pieces of steel sheeting 4" x 12" (9.16 cm x 30.48 cm), coated with a primer based on polyester/epoxy resin, are then coated with a $TiO_2$-pigmented base coat on a binder of 70 % of monomers such as hydroxyethyl acrylate, styrene, acrylonitrile, butyl acrylate and acrylic acid with 30 % of a melamine resin and an acid catalyst and finally with the clear finishing enamel. The base coat is sprayed onto the sheet to a thickness of about 0.8 mil (0.02 mm), air dried for 3 minutes and baked at 121°C for 10 minutes. The clear finishing enamel coat is then wire-coated onto the sheet to a thickness of about 0.06 mm. After 15 minutes air-drying, the coated sheets are baked for 30 minutes at 82°C.

The stabilizers under test are added to the thermoset acrylic resin finishing enamel in concentrations of 2 % by weight, before the enamel is coated onto the base coated sheet.

Gardner yellowness index color values are determined on the baked sample and on the sample after having been subjected to an additional 30 minutes baking at 82°C.

In addition, after storage for 3 weeks in an air-conditioned room (23°C/50 % relative humidity), the coated sheets are subjected to weathering for 150 hours according to test method ASTM G-53/77 in a QUV exposure apparatus. In this apparatus, the samples are subjected to weathering in repreated cycles for 4 hours in a humid atmosphere at 50°C and then for 8 hours under UV light at 70°C. Equivalent samples are also subjected to accelerated weathering for 2625 hours in a Xenon arc (6500 watt) Weatherometer. The samples are exposed to repeated cycles for 102 minutes to the light source, then for 18 minutes to a water spray and light at 60°C. The Gardner color value is also determined for each sample after QUV and Zenon exposure.

| Compound | Orig. Color | Color Change | | |
|---|---|---|---|---|
| | | Overbake | QUV | Xenon |
| Unstabilized | 1.0 | 0.8 | 23.0 | 24.5 |
| 3 | 1.2 | 0.8 | 13.1 | 3.9 |
| 9 | 1.2 | 0.6 | 15.0 | 4.6 |
| 12 | 1.1 | 0.3 | 18.1 | 4.8 |
| 26 | 3.3 | 0.8 | 12.4 | 1.8 |
| 28 | 1.0 | 0.3 | 15.3 | 3.8 |

Example 8

The samples of Example 7 are also evaluated on the basis of Knoop Hardness (ASTM D-1474-68) on the baked and overbaked samples; distinction of image (DI), Hunter Associates Apparatus; 20° gloss (ASTM D-523-80); and cracking based on visual observation on a scale of 0-8 with "0" reflecting absence of cracking and "8" relecting excessive cracking and "a", "b", "c" designations for crack depth with "a" relecting shallow cracks and "c" relecting deepest cracks.

| Compound | Knoop Hardness | | QUV Exposure | | | | | | | |
|---|---|---|---|---|---|---|---|---|---|---|
| | | | 20° Gloss | | | DI | | | Cracks | |
| | Bake | Overbake | 575 hrs. | 750 | 900 | 575 | 750 | 900 | 750 | 900 |
| Unstabilized | 5.5 | 8.0 | 13 | 11 | - | 9 | 4 | - | 8c | - |
| 3 | 5.5 | 8.0 | 84 | 70 | 29 | 90 | 85 | 12 | 0 | 3a |
| 9 | 5.5 | 8.0 | 85 | 56 | 12 | 90 | 62 | 7 | 5a | 6a |
| 12 | 4.0 | 7.0 | 70 | 55 | 10 | 42 | 13 | 8 | 3a | 4a |
| 26 | 5.5 | 8.0 | 85 | 44 | 8 | 92 | 51 | 12 | 7a | 7a |
| 28 | 4.0 | 6.5 | 90 | 66 | 17 | 95 | 77 | 3 | 0 | 7a |

These data illustrate a pattern of greater retention of gloss and DI and longer absence of severe cracking after the indicated exposure conditions.

Example 9

The thermoset acrylic enamel of Example 7 is formulated to include 2 % by weight of a benzotriazole UV-absorber and 1.5 %, by weight, of the hindered amine light stabilizer. The enamel is coated over a white base coat or over a silver metallic base coat. Baking is conducted for 30 minutes at 121°C.

The coated panels are exposed in the QUV exposure apparatus and 20° gloss and distinction of image (DI) values are determined.

A) With white base coat

| Compound | Hours of QUV Exposure | | | | | |
|---|---|---|---|---|---|---|
| | 20° Gloss | | | DI | | |
| | 0 | 1421 | 2106 | 0 | 1421 | 2106 |
| Unstabilized | 93 | (655 hrs)* | - | 88 | (655 hrs)* | - |
| Compound C | 94 | 9 | 3* | 89 | 15 | 3* |
| C + 3 | 93 | 64 | 16* | 89 | 81 | 47* |
| C + 10 | 93 | 102 | 90* | 84 | 82 | 59* |
| C + 11 | 93 | 80* | - | 91 | 70* | - |
| C + 12 | 93 | 73 | 27* | 92 | 88 | 57* |
| C + 16 | 93 | 92 | 62* | 88 | 81 | 58* |
| C + 17 | 92 | 67 | 12* | 91 | 85 | 45* |
| C + 18 | 94 | 55 | 7* | 89 | 80 | 21* |
| C + 21 | 92 | 69* | - | 82 | 57* | - |

Compound C = 2-[2-hydroxy-3,5-di-(alpha,alpha-dimethylbenzyl)phenyl]-benzotriazole

* = cracking observed

B) With silver metallic base coat

| Compound | Hours of QUV Exposure | | | | | | | | | |
|---|---|---|---|---|---|---|---|---|---|---|
| | 20° Gloss | | | | | DI | | | | |
| | 0 | 674 | 916 | 1118 | 1377 | 0 | 674 | 916 | 1118 | 1377 |
| Unstabilized | 92 | 2* | - | - | - | 87 | 2 | - | - | - |
| Compound C | 90 | 51 | 5 | 3 | 2.2* | 84 | 65 | 6 | 5 | -2.5 |
| C + 3 | 95 | 86 | 53 | 18 | 4.8* | 88 | 79 | 78 | 48 | 28 |
| C + 10 | 94 | 85 | 44 | 22* | - | 88 | 78 | 48 | 13 | - |
| C + 11 | 92 | 82 | 43 | 27* | - | 87 | 77 | 36 | 13 | - |
| C + 12 | 95 | 83 | 59 | 21 | 3* | 88 | 87 | 80 | 46 | -1.8 |
| C + 16 | 92 | 93 | 72 | 32* | - | 90 | 88 | 75 | 28 | - |
| C + 17 | 93 | 82 | 60 | 33 | 8* | 85 | 83 | 80 | 59 | 29 |
| C + 18 | 93 | 85 | 40 | 9 | 3* | 89 | 86 | 67 | 44 | 20 |
| C + 21 | 91 | 89 | 64 | 29* | - | 71 | 68 | 54 | 28 | - |

Example 10

Two thermoset acrylic enamels are formulated to include 2 %, by weight, of a benzotriazole UV-absorber and 1 %, by weight of a hindered amine light stabilizer as described in Example 9,

The thermoset acrylic enamels are based on a binder of 70 % of a copolymer from hydroxyethyl acrylate, styrene, acrylonitrile, butyl acrylate and acrylic acid and 30 % of a melamine resin and an acid catalyst (0.5 % of p-toluenesulfonic acid).

Pieces of steel sheeting (9 x 30 cm), coated with a primer based on polyester/epoxy resin, are coated with the white base coat and finally with the clear finishing enamel. The base coat is sprayed onto the sheet to a thickness of about 0.02 mm and air dried for 3 minutes. The clear finishing enamel coat is then

sprayed onto the sheet to a thickness of about 0.038 mm. After 15 minutes air-drying, the coated sheets are baked for 30 minutes at 121 ° C. QUV evaluations are then conducted.

| Compound | Test | Hours of QUV Exposure | | | | | |
|---|---|---|---|---|---|---|---|
| | | 0 | 1010 | 1270 | 1594 | 1826 | 2036 |
| Unstabilized | 20 ° Gloss<br>DI | 89<br>92 | 25<br>5 | 23*<br>4 | -<br>- | -<br>- | -<br>- |
| C + 14 | 20 ° Gloss<br>DI | 91<br>98 | 91<br>90 | 89<br>86 | 82<br>87 | 48<br>47 | 34*<br>45 |

Example 11

A white polyester/melamine based oil-free alkyd coil coating is utilized in this example. The fully formulated point is applied over a primed steel sheet using a wire wound rod to give a 0.015-0.020 mm dry film. The panels are baked for 90 seconds at 218 ° C. The baked panels are removed from the oven and are immediately quenched in water. The coated panels are exposed in a Carbon Arc Weatherometer, a Xenon Arc Weatherometer, and in South Florida at an angle of 45 ° S to the sun. 20 ° gloss values are determined.

| | Exposure Time | Unstabilized | 2 %, by weight Compound |
|---|---|---|---|
| Carbon Arc Weatherometer | 0 (Hrs.)<br>112<br>262<br>337<br>447 | 78<br>69<br>15<br>11<br>14 | 81<br>78<br>45<br>37<br>31 |
| Xenon Arc Weatherometer | 0 (Hrs.)<br>150<br>378<br>709<br>954 | 78<br>75<br>63<br>11<br>5 | 81<br>83<br>75<br>23<br>12 |
| Florida 45 ° S Direct Weather | 0 (Mos.)<br>6<br>12 | 78<br>76<br>39 | 81<br>79<br>60 |

Example 12

The thermoset acrylic enamel of Example 10 including 0.5 % of p-toluenesulfonic acid is formulated to include varying concentrations of a benzotriazole UV-absorber and a hindered amine light stabilizer of the invention. The enamel is coated over a silver metallic base coat pursuant to the procedure in Example 10 and baking is conducted for 30 minutes at 121 ° C bake temperature.

The coated panels are exposed in the QUV exposure apparatus and the time to 50 % loss of 20 ° gloss is determined.

| Compound | Conc. (%, by weight) | Time to 50 % loss of 20° Gloss (hours) |
|---|---|---|
| unstabilized | - | 900 |
| B/1 | 3.5/1.5 | 3600 |
| B/2 | 3.5/1.5 | 4200 |
| B/23 | 3.5/1.5 | 3200 |
| B/26 | 3.5/1.5 | 3700 |
| B/32 | 3.5/1.5 | 3900 |
| B/33 | 3.5/1.5 | 4200 |
| B/35 | 3.5/1.5 | 4800 |
| unstabilized | - | 1000 |
| B/1 | 3/1 | 3800 |
| B/2 | 3/1 | 3400 |
| B/4 | 3/1 | 3950 |
| B/19 | 3/1 | 4000 |
| B/22 | 3/1 | 3700 |
| B/25 | 3/1 | 3700 |
| Compound B = 2-[2-hydroxy-3-tert.butyl-5-(2-(omega-hydroxy-octa(ethyleneoxy)carbonyl)-ethylphenyl)]-benzotriazole | | |

The date in these examples clearly illustrate the beneficial performance characteristics of the compositions of this invention.

Various N-acyloxy hindered amine derivatives have been disclosed. N-Acetoxy derivatives prepared from the N-hydroxy starting material are described in Kurumada et al., J.Polym.Sci., Polym.Chem. Ed. 22, 277-81 (1984). Felder et al., Helv.Chim.Acta. 63, 132 (1980) teach the preparation of an N-phenylacetoxy derivative. The bis(N-acetoxypiperidyl) sebacate has been disclosed in Carlsson et al, Polym.Science Technol. 26, 35-47 (1984). Finally US 4,472,547 discloses various N-piperidyl lactam compounds such as an N-benzoyloxy hindered amine containing a 4-(2-oxo-hexamethyleneimine) substituent as light stabilizers for polyolefins and other organic polymers.

A further object of the invention are the new N-acyloxy hindered amine compounds having one of formulae A′ to N′

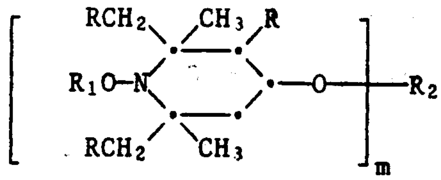

(A')

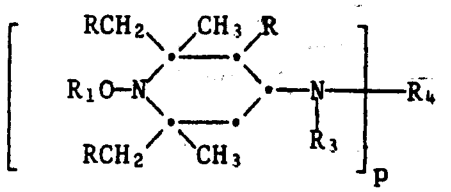

(B')

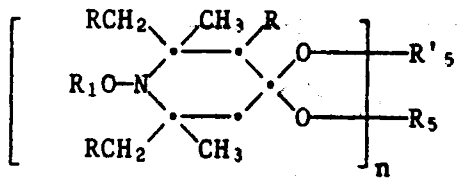

(C')

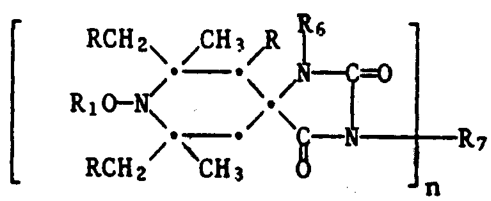

(D')

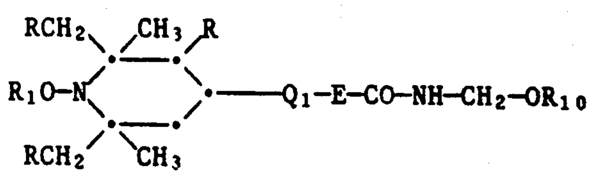

(E')

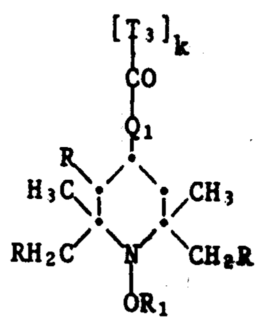

(F')

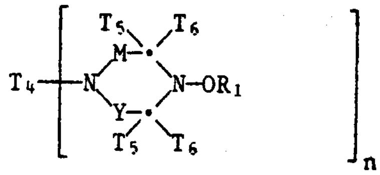

(G')

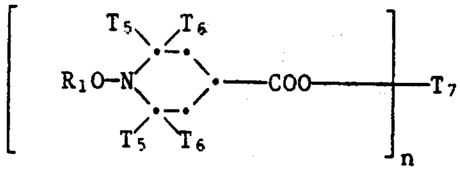

(H')

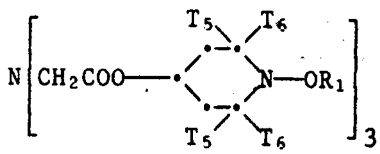

(I')

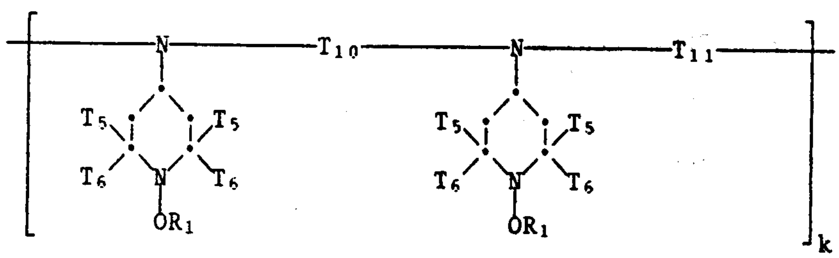

(J')

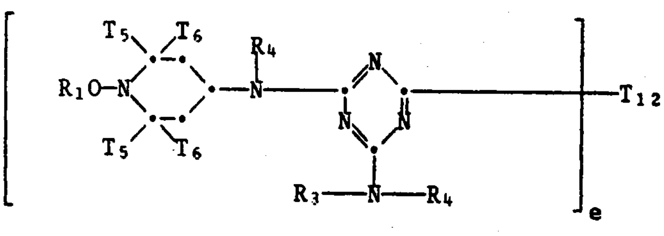

(K')

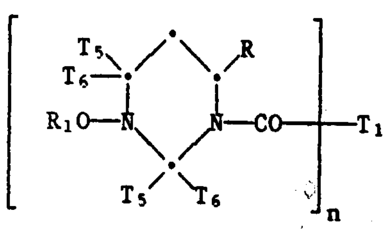

(L')

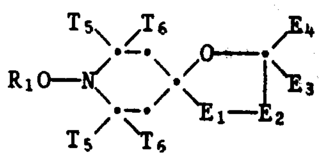

(M')

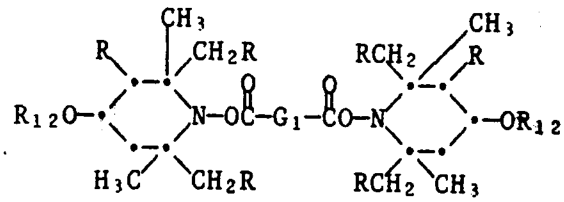

(N')

wherein
R is hydrogen or methyl, preferably hydrogen;
$R_1$ is a group

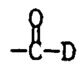

wherein D is $C_1$-$C_{18}$ alkyl, $C_1$-$C_{18}$ alkoxy, phenyl, phenyl substituted by hydroxy, $C_1$-$C_4$ alkyl or $C_1$-$C_4$ alkoxy, or amino or amino mono- or disubstituted by $C_1$-$C_{12}$ alkyl or phenyl; preferably D is $C_1$-$C_{12}$ alkyl, $C_1$-$C_4$ alkoxy, phenyl, amino, $C_1$-$C_{12}$ alkylamino or phenylamino;
m is 1-4,
when m is 1,
$R_2$ is a group

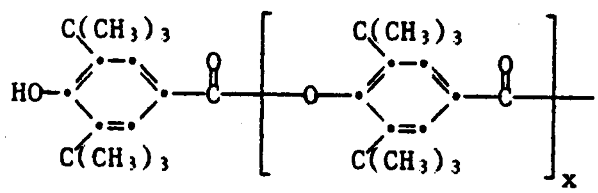

wherein x is 0 or 1, or $R_2$ is a group

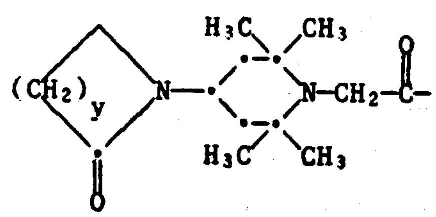

wherein y is 2-4;
when m is 2 and D is alkyl,
$R_2$ is $C_1$-$C_{12}$ alkylene, $C_4$-$C_{12}$ alkenylene, xylylene, a divalent acyl radical of a cycloaliphatic, araliphatic or aromatic dicarboxylic acid having 8-14 C atoms or of an aliphatic, cycloaliphatic or aromatic dicarbamic acid having 8-14 C atoms; or $R_2$ is a group

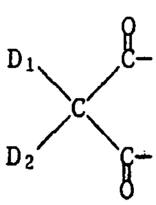

or

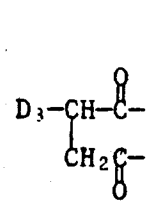

wherein $D_1$ is $C_1$-$C_8$ alkyl, benzyl or 3,5-di-t-butyl-4-hydroxybenzyl, $D_2$ is $D_1$ or hydrogen, $D_3$ is $C_1$-$C_{18}$ alkyl or $C_2$-$C_{18}$ alkenyl;

when m is 2 and D is phenyl or substituted phenyl, amino, substituted amino or alkoxy,

$R_2$ is $C_1$-$C_{12}$ alkylene, $C_4$-$C_{12}$ alkenylene, xylylene, a divalent acyl radical of an aliphatic, cycloaliphatic, araliphatic or aromatic dicarboxylic or dicarbamic acid having up to 14 C atoms;

preferably an acyl radical of an aliphatic dicarboxylic acid having 2-18 C atoms, of a cycloaliphatic or aromatic dicarboxylic acid having 8-14 C atoms, or of an aliphatic, cycloaliphatic or aromatic dicarbamic acid having 8-14 C atoms; or $R_2$ is a group

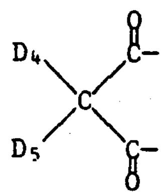

or

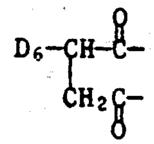

wherein $D_4$ and $D_5$ are independently hydrogen, $C_1$-$C_8$ alkyl, benzyl or 3,5-di-t-butyl-4-hydroxybenzyl und $D_6$ is $C_1$-$C_{18}$ alkyl or $C_2$-$C_{18}$ alkenyl;

when m is 3, $R_2$ is a trivalent acyl radical of an aliphatic, cycloaliphatic, or aromatic tricarboxylic acid having up to 12 C atoms;

when m is 4, $R_2$ is a tetravalent acyl radical of a saturated or unsaturated aliphatic or aromatic tetracarboxylic acid having up to 18 C atoms including 1,2,3,4-butanetetracarboxylic acid, 1,2,3,4-but-2-enetetracarboxylic acid, and 1,2,3,5-and 1,2,4,5-pentanetetracarboxylic acid;

p is 1, 2 or 3,

$R_3$ is hydrogen, $C_1$-$C_{12}$ alkyl, $C_5$-$C_7$ cycloalkyl, $C_7$-$C_9$ aralkyl, $C_2$-$C_{18}$ alkanoyl, $C_3$-$C_5$ alkenoyl or benzoyl;

when p is 1,

$R_4$ is hydrogen, $C_1$-$C_{18}$ alkyl, $C_5$-$C_7$ cycloalkyl, $C_2$-$C_8$ alkenyl unsubstituted or substituted by a cyano, carbonyl or carbamide group, or it is aryl, aralkyl, glycidyl, a group of the formula $-CH_2-CH(OH)-Z$ or $-CONH-Z$ wherein Z is hydrogen, methyl or phenyl;

or $R_4$ is a group of the formula

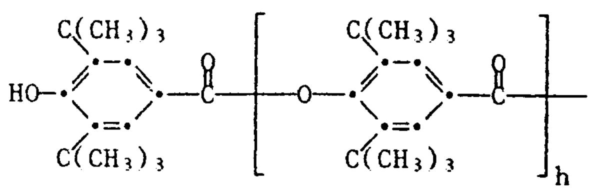

with h as 0 or 1;

or a group of the formula I

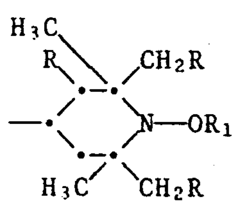

(I);

or $R_3$ and $R_4$ together are alkylene of 4 to 6 carbon atoms or the divalent acyl radical of an aliphatic or aromatic 1,2- or 1,3-dicarboxylic acid;

when p is 2,

$R_4$ is $C_1$-$C_{12}$ alkylene, $C_6$-$C_{12}$ arylene, xylylene, a $-CH_2CH(OH)-CH_2-$ group, or a group $-CH_2-CH(OH)-CH_2-O-X-O-CH_2-CH(OH)-CH_2-$ wherein X is $C_2$-$C_{10}$ alkylene, $C_6$-$C_{15}$ arylene or $C_6$-$C_{12}$ cycloalkylene; or, provided that $R_3$ is not alkanoyl, alkenoyl or benzoyl, $R_4$ can also be a divalent acyl radical of an aliphatic, cycloaliphatic or aromatic dicarboxylic or dicarbamic acid having up to 14 C atoms, or can be the group -CO- or a group of formula II

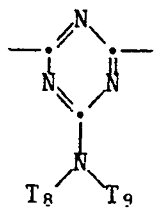

(II)

where $T_8$ and $T_9$ are independently hydrogen, alkyl of 1 to 18 carbon atoms, or $T_8$ and $T_9$ together are alkylene of 4 to 6 carbon atoms or 3-oxapentamethylene, preferably $T_8$ and $T_9$ together are 3-oxapentamethylene;
when p is 3, $R_4$ is 2,4,6-triazinetriyl;
n is 1 or 2 and when n is 1,
$R_5$ and $R'_5$ are independently $C_1$-$C_{12}$ alkyl, $C_2$-$C_{12}$ alkenyl, $C_7$-$C_{12}$ aralkyl, or $R_5$ is also hydrogen, or $R_5$ and $R'_5$ together are $C_2$-$C_8$ alkylene or hydroxyalkylene or $C_4$-$C_{22}$ acyloxyalkylene;
and when n is 2,
$R_5$ and $R'_5$ together are $(-CH_2)_2C(CH_2-)_2$;
$R_6$ is hydrogen, $C_1$-$C_{12}$ alkyl, allyl, benzyl, glycidyl or $C_2$-$C_6$ alkoxyalkyl;
when n is 1,
$R_7$ is hydrogen, $C_1$-$C_{12}$ alkyl, $C_3$-$C_5$ alkenyl, $C_7$-$C_9$ aralkyl, $C_5$-$C_7$ cycloalkyl, $C_2$-$C_4$ hydroxyalkyl, $C_2$-$C_6$ alkoxyalkyl, $C_6$-$C_{10}$ aryl, glycidyl, a group of the formula $-(CH_2)_t$-COO-Q or of the formula $-(CH_2)_t$-O-CO-Q wherein t is 1 or 2, and Q is $C_1$-$C_4$ alkyl or phenyl; and
when n is 2,
$R_7$ is $C_2$-$C_{12}$ alkylene, $C_6$-$C_{12}$ arylene, a group $-CH_2CH(OH)-CH_2-O-X-O-CH_2-CH(OH)-CH_2-$ wherein X is $C_2$-$C_{10}$ alkylene, $C_6$-$C_{15}$ arylene or $C_6$-$C_{12}$ cycloalkylene, or a group $-CH_2CH(OZ')CH_2-(OCH_2-CH(OZ')-CH_2)_2-$ wherein Z' is hydrogen, $C_1$-$C_{18}$ alkyl, allyl benzyl, $C_2$-$C_{12}$ alkanoyl or benzoyl;
$Q_1$ is $-N(R_8)-$ or -O-;
E is $C_1$-$C_3$ alkylene, the group $-CH_2-CH(R_9)-O-$ wherein $R_9$ is hydrogen, methyl or phenyl, or E is the group $-(CH_2)_3-NH-$ or a direct bond;
$R_{10}$ is hydrogen or $C_1$-$C_{18}$ alkyl, $R_8$ is hydrogen, $C_1$-$C_{18}$ alkyl, $C_5$-$C_7$ cycloalkyl, $C_7$-$C_{12}$ aralkyl, cyanoethyl, $C_6$-$C_{10}$ aryl, the group $-CH_2-CH(R_9)-OH$ wherein $R_9$ has the meaning defined above; a group of the formula I or a group of the formula

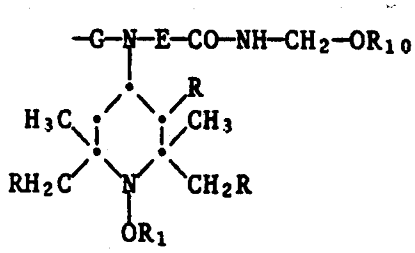

wherein G can be $C_2$-$C_6$ alkylene or $C_6$-$C_{12}$ arylene; or $R_8$ is a group $-E-CO-NH-CH_2-OR_{10}$;
Formula F denotes a recurring structural unit of a polymer where $T_3$ is ethylene or 1,2-propylene, or is the repeating structural unit derived from an alpha-olefin copolymer with an alkyl acrylate or methacrylate; preferably a copolymer of ethylene and ethyl acrylate, and where k is 2 to 100;
$T_4$ has the same meaning as $R_4$ when p is 1 or 2,
$T_5$ is methyl,
$T_6$ is methyl or ethyl, or $T_5$ and $T_6$ together are tetramethylene or pentamethylene, preferably $T_5$ and $T_6$ are each methyl,
M and Y are independently methylene or carbonyl, preferably M is methylene, Y is carbonyl and $T_4$ is ethylene where n is 2;
$T_7$ is the same as $R_7$, and $T_7$ is preferably octamethylene when n is 2;
$T_{10}$ and $T_{11}$ are independently alkylene of 2 to 12 carbon atoms, or $T_{11}$ is a group of formula II;
e is 2, 3 or 4,
$T_{12}$ is a group

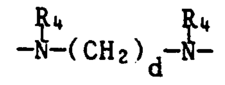

or 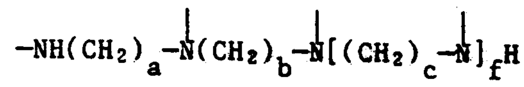

where a, b and c are independently 2 or 3, d is 2-10, and f is 0 or 1, preferably a and c are each 3, b is 2 and f is 1;

$T_{13}$ is the same as $R_4$ with the proviso that $T_{13}$ cannot be hydrogen when n is 1;

$E_1$ and $E_2$, being different, each are -CO- or -N($E_5$)-, where $E_5$ is hydrogen, $C_1$-$C_{12}$ alkyl or alkoxycarbonylalkyl of 4 to 22 carbon atoms, preferably $E_1$ is -CO- and $E_2$ is -N($E_5$);

$E_3$ is hydrogen, alkyl of 1 to 30 carbon atoms, phenyl, naphthyl, said phenyl or said naphthyl substituted by chlorine or by alkyl of 1 to 4 carbon atoms, or phenylalkyl of 7 to 12 carbon atoms, or said phenylalkyl substituted by alkyl of 1 to 4 carbon atoms,

$E_4$ is hydrogen, alkyl of 1 to 30 carbon atoms, phenyl, naphthyl or phenylalkyl of 7 to 12 carbon atoms, or

$E_3$ and $E_4$ together are polymethylene of 4 to 17 carbon atoms, or said polymethylene substituted by up to four alkyl groups of 1 to 4 carbon atoms, preferably methyl, and

$R_{12}$ is $C_2$-$C_{18}$ alkanoyl, $C_3$-$C_6$ alkenoyl, benzoyl or benzoyl substituted by $C_1$-$C_4$ alkyl, halogen or hydroxyl, and $G_1$ is a direct bond, $C_1$-$C_{12}$ alkylene, phenylene or -NH-G′-NH wherein G′ is $C_1$-$C_{12}$ alkylene.

In the structures A′ to N′, if any substituents are $C_1$-$C_{18}$ alkyl, they are for example methyl, ethyl, n-propyl, n-butyl, sec-butyl, tert-butyl, n-hexyl, n-octyl, 2-ethylhexyl, n-nonyl, n-decyl, n-undecyl, n-dodecyl, n-tridecyl, n-tetradecyl, n-hexadecyl or n-octadecyl. Typical cycloalkyl groups include cyclopentyl and cyclohexyl, while typical aralkyl groups include benzyl, alpha-methyl-benzyl, alpha,alpha-dimethylbenzyl or phenethyl.

If $R_2$ is a divalent acyl radical of a dicarboxylic acid, it is for example an acyl radical of adipic acid, succinic acid, suberic acid, sebacic acid, phthalic acid, dibutylmalonic acid, dibenzylmalonic acid or (3,5-di-tert-butyl-4-hydroxybenzyl)-malonic acid, or bicycloheptenedicarboxylic acid.

If $R_2$ is a divalent acyl radical of a dicarbamic acid, it is for example an acyl radical of hexamethylenedicarbamic acid or of 2,4-toluylenedicarbamic acid.

The following compounds are examples of polyalkylpiperidine starting materials useful in making hindered amine derivatives of formula A′. (Depending on the selected preparative procedure).

di-(2,2,6,6-tetramethylpiperidin-4-yl) phthalate
alpha,alpha′-(di-2,2,6,6-tetramethylpiperidyl-4-oxy)-p-xylene
1,4-dihydroxy-2,2,6,6-tetramethylpiperidine
1-acetoxy-4-hydroxy-2,2,6,6-tetramethylpiperidine
di-(1-oxyl-2,2,6,6-tetramethylpiperidin-4-yl) phthalate
1-oxyl-2,2,6,6-tetramethylpiperidin-4-one
(1-hydroxy-2,2,6,6-tetramethylpiperidin-4-yl) stearate
(2,2,6,6-tetramethylpiperidin-4-yl)-[4-(2-oxoazepin-1-yl)-2,2,6,6-tetramethylpiperidin-4-yl] acetate

As $C_2$-$C_{18}$ alkanoyl, $R_3$ is for example propionyl, butyryl, octanoyl, dodecanoyl, hexadecanoyl, octadecanoyl, but preferably acetyl; and as $C_3$-$C_5$ alkenoyl, $R_3$ is in particular acryloyl.

If $R_4$ is $C_2$-$C_8$ alkenyl unsubstituted or substituted by a cyano, carbonyl or carbamide group, it is for example 1-propenyl, allyl, methallyl, 2-butenyl, 2-pentenyl, 2-hexenyl, 2-octenyl, 2,2-dicyanovinyl, 1-methyl-2-cyano-2-methoxycarbonyl-vinyl or 2,2-diacetylaminovinyl.

If any substituents are $C_2$-$C_{12}$ alkylene, they are for example ethylene, propylene, 2,2-dimethylpropylene, tetramethylene, hexamethylene, octamethylene, decamethylene or dodecamethylene.

If any substituents are $C_6$-$C_{12}$ arylene, they are for example o-, m- or p-phenylene, 1,4-naphthylene or 4,4′-di-phenylene.

As $C_6$-$C_{12}$ cycloalkylene, X is especially cyclohexylene.

The following compounds are examples of polyalkylpiperidine starting materials useful in making compounds of formula B′.

N,N′-bis-(2,2,6,6-tetramethylpiperidin-4-yl)-hexamethylene-1,6-diamine,
N,N′-bis-(2,2,6,6-tetramethylpiperidin-4-yl)-hexamethylene-1,6-diacetamide,
4-benzylamino-2,2,6,6-tetramethylpiperidine,
N-n-butyl-N-(2,2,6,6-tetramethylpiperidine-4-yl)-4-hydroxy-3,5-di-tert.butylbenzamide,
N,N′-bis-(2,2,6,6-tetramethylpiperidin-4-yl)-N,N′-di-butyl-adipamide,

N,N′-bis-(2,2,6,6-tetramethylpiperidin-4-yl)-N,N′-dicyclohexyl-(2-hydroxypropylene),
N,N′-bis-(2,2,6,6-tetramethylpiperidin-4-yl)-p-xylylenediamine,
4-(3-methyl-4-hydroxy-5-tert-butyl-benzoyl acetamido)-2,2,6,6-tetramethylpiperidine,
alpha-cyano-β-methyl-β-[N-(2,2,6,6-tetramethylpiperidin-4-yl-amino]-acrylic acid methyl ester,
1-acetoxy-4-butylamino-2,2,6,6-tetramethylpiperidine,
1-oxyl-2,2,6,6-tetramethylpiperidin-4-one.

If $R_5$ and $R'_5$ together are $C_2$-$C_8$ alkylene or hydroxyalkylene or $C_4$-$C_{22}$ acyloxyalkylene, it is for example ethylene, 1-methyl-ethylene, propylene, 2-ethylpropylene, 2-ethyl-2-hydroxymethylpropylene or 2-ethyl-2-acetoxymethyl-propylene.

The following compounds are examples of polyalkylpiperidine starting materials useful in making the compounds of formula C′.
9-aza-8,8,10,10-tetramethyl-1,5-dioxaspiro[5.5]undecane,
9-aza-8,8,10,10-tetramethyl-3-ethyl-1,5-dioxaspiro[5.5]undecane,
2,2,6,6-tetramethylpiperidine-4-spiro-2′-(1′,3′-dioxane)-5′-spiro-5″-(1″,3″-dioxane)-2″-spiro-4‴-(2‴,2‴,6‴,6‴-tetramethylpiperidine).

If any substituents are $C_2$-$C_6$ alkoxyalkyl, they are for example methoxymethyl, ethoxymethyl, propoxymethyl, tert-butoxyethyl, ethoxyethyl, ethoxypropyl, n-butoxyethyl, tert-butoxyethyl, isopropoxyethyl or propoxypropyl.

If $R_7$ is $C_3$-$C_5$ alkenyl, it is for example 1-propenyl, allyl, methallyl, 2-butenyl or 2-pentenyl.

As $C_7$-$C_9$ aralkyl, $R_7$ is in particular phenethyl or above all benzyl; and as $C_5$-$C_7$ cycloalkyl, $R_7$ is especially cyclohexyl.

If $R_7$ is $C_2$-$C_4$ hydroxyalkyl, it is for example 2-hydroxyethyl, 2-hydroxypropyl, 3-hydroxypropyl, 2-hydroxybutyl or 4-hydroxybutyl.

As $C_6$-$C_{10}$ aryl, the substituents are in particular phenyl, or alpha- or β-naphthyl which is unsubstituted or substituted by halogen or $C_1$-$C_4$ alkyl.

If Z′ is $C_2$-$C_{12}$ alkanoyl, it is for example propionyl, butyryl, octanoyl, dodecanoyl or preferably acetyl.

The following compounds are examples of polyalkylpiperidine starting materials useful in making compounds of formula D′.
3-benzyl-1,3,8-triaza-7,7,9,9-tetramethylspiro[4.5]-decane-2,4-dione,
3-n-octyl-1,3,8-triaza-7,7,9,9-tetramethylspiro[4.5]-decane-2,4-dione,
3-allyl-1,3,8-triaza-1,7,7,9,9-pentamethylspiro[4.5]-decane-2,4-dione,
or the compounds of the following formulae:

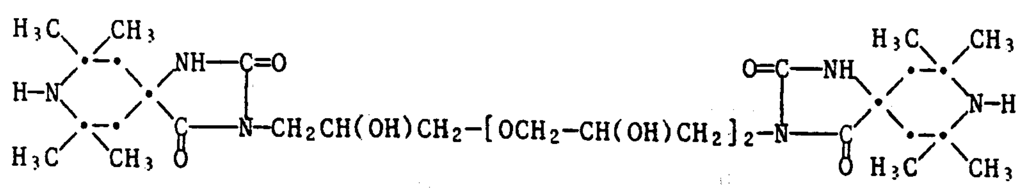

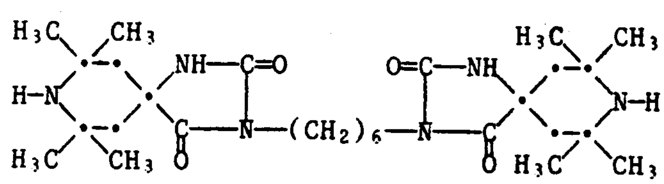

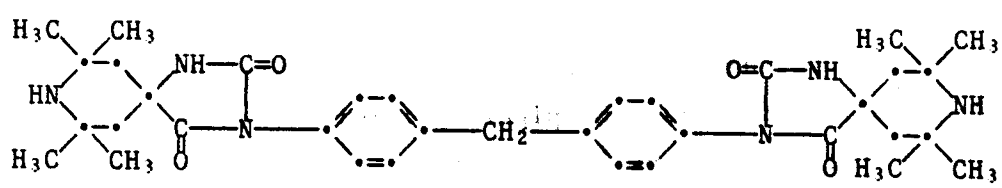

As $C_5$-$C_7$ cycloalkyl, $R_8$ is in particular cyclohexyl.

As $C_6$-$C_{10}$ aryl, $R_8$ is particularly phenyl, or alpha- or β-naphthyl which is unsubstituted or substituted with halogen or $C_1$-$C_4$ alkyl.

As $C_1$-$C_3$ alkylene, E is for example methylene, ethylene or propylene.

As $C_2$-$C_6$ alkylene, G is for example ethylene, propylene, 2,2-dimethylpropylene, tetramethylene or hexamethylene; and as $C_6$-$C_{12}$ arylene, G is o-, m- or p-phenylene, 1,4-naphthylene or 4,4′-diphenylene.

The following compounds are examples of polyalkylpiperidine starting materials useful in making the compounds of formula E′.

N-hydroxymethyl-N′-2,2,6,6-tetramethylpiperidin-4-yl-urea,
N-methoxymethyl-N′-2,2,6,6-tetramethylpiperidin-4-yl-urea,
N-methoxymethyl-N′-n-dodecyl-N′-2,2,6,6-tetramethylpiperidin-4-yl-urea, and
O-(2,2,6,6-tetramethylpiperidin-4-yl)-N-methoxymethyl-urethane.

When the instant hindered amine derivative is of formula F′, the following polymeric compounds are examples of starting materials useful in preparing said derivatives.

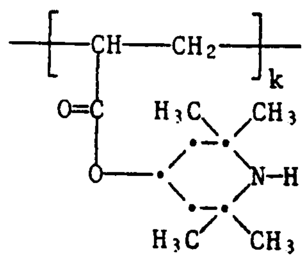

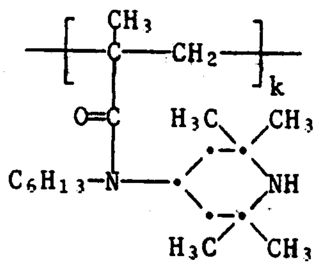

Additional starting hindered amine derivatives include for formula J′:
poly-{[6-[(1,1,3,3-tetramethylbutyl)-imino]-1,3,5-triazine-2,4-diyl][2-(1-oxyl-2,2,6,6-tetramethylpiperidyl)-imino]-hexamethylene-4[4-(1-oxyl-2,2,6,6-tetramethylpiperidyl]-imino]}.

Preferred compounds are the compounds of formulae A′, B′, D′, F′, J′, K′, M′ or N′, wherein R is hydrogen,
$R_1$ is -CO-D and D is $C_1$-$C_{12}$ alkyl, $C_1$-$C_4$ alkoxy, phenyl, amino, $C_1$-$C_{12}$ alkylamino or phenylamino,
m is 1, 2 or 4, and
when m is 1, $R_2$ is

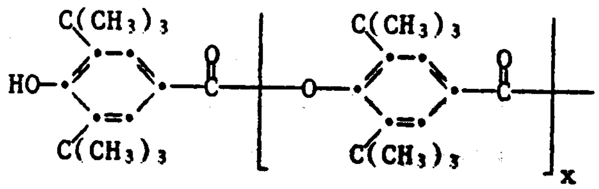

wherein x is 0 or 1,
when m is 2 and D is alkyl,
$R_2$ is a divalent acyl radical of a cycloaliphatic or aromatic dicarboxylic acid having 8-14 C atoms, or of an aliphatic, cycloaliphatic or aromatic dicarbamic acid having 8-14 C atoms; or $R_2$ is a group

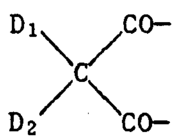

wherein $D_1$ is $C_1$-$C_4$ alkyl or 3,5-di-t-butyl-4-hydroxybenzyl and $D_2$ is $D_1$ or hydrogen, and when m is 2 and D is phenyl, amino, alkylamino, phenylamino or alkoxy, $R_2$ is a divalent acyl radical of an aliphatic, dicarboxylic acid having 2-8 C atoms or of a cycloaliphatic or aromatic dicarboxylic acid having 8-14 C atoms or of an aliphatic, cycloaliphatic or aromatic dicarbamic acid having 8-14 C atoms, or $R_2$ is a group

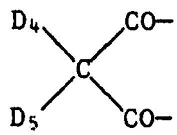

wherein $D_4$ and $D_5$ are independently hydrogen, $C_1$-$C_8$ alkyl, benzyl or 3,5-di-t-butyl-4-hydroxybenzyl, and when m is 4, $R_2$ is a tetravalent acyl radical of 1,2,3,4-butanetetracarboxylic acid, 1,2,3,4-but-2-enetetracarboxylic acid, 1,2,3,5- or 1,2,4,5-pentanetetracarboxylic acid;
p is 1, 2 or 3,
$R_3$ is hydrogen, $C_1$-$C_{12}$ alkyl, cyclohexyl, $C_7$-$C_9$ aralkyl, $C_2$-$C_{18}$ alkanoyl or benzoyl;
when p is 1,
$R_4$ is $C_1$-$C_{18}$ alkyl, cyclohexyl, allyl, benzyl or a group

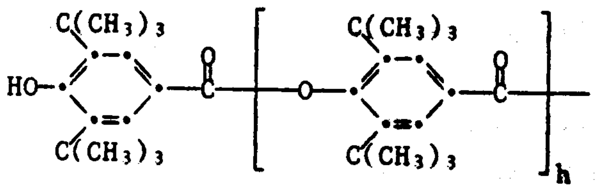

with h as 0 or 1, or is a group

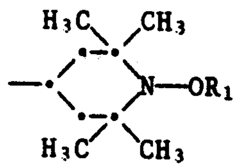

when p is 2,
$R_4$ is $C_1$-$C_{12}$ alkylene, $C_6$-$C_{12}$ arylene, xylylene, a group $-CH_2CH(OH)-CH_2-$, or provided that $R_3$ is not alkanoyl or benzoyl, $R_4$ can also be a divalent acyl radical of an aliphatic, cycloaliphatic or aromatic dicarboxylic acid or dicarbamic acid having 6-12 C atoms,
or $R_4$ is a group of formula II,
where $T_8$ and $T_9$ are independently hydrogen or $C_1$-$C_{12}$ alkyl or $T_8$ and $T_9$ together are $C_4$-$C_6$ alkylene or 3-oxapentamethylene,
when p is 3, $R_4$ is 2,4,6-triazinetriyl;
n is 1 or 2 and
$R_6$ is hydrogen, $C_1$-$C_{12}$ alkyl, allyl or benzyl,
when n is 1, $R_7$ is hydrogen, $C_1$-$C_{12}$ alkyl, allyl, benzyl or cyclohexyl, and when n is 2, $R_7$ is $C_2$-$C_{12}$ alkylene;
k is 5 to 50, $T_3$ is ethylene or propylene and $Q_1$ i NH or O;
$T_5$ and $T_6$ each are methyl,
$T_{10}$ and $T_{11}$ are independently $C_2$-$C_{12}$ alkylene or $T_{11}$ is a group of formula II,
e is 4 and
$T_{12}$ is

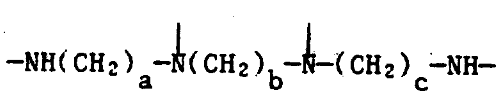

where a, b and c are independently 2 or 3;
$E_1$ is -CO- and $E_2$ is $-N(E_5)-$, $E_3$ and $E_4$ independently are $C_1$-$C_{12}$ alkyl or $E_3$ and $E_4$ together are $C_5$-$C_{12}$ polymethylene, and $E_5$ is hydrogen or $C_1$-$C_{12}$ alkyl;

$G_1$ is a direct bond, $C_2$-$C_8$ alkylene or -NH-G′-NH- wherein G′ is $C_2$-$C_8$ alkylene, and $R_{12}$ is $C_2$-$C_{18}$ alkanoyl or benzoyl.

Especially preferred are the compounds of formulae A′, B′, F′, J′ or N′, wherein R is hydrogen,
$R_1$ is -CO-D and D is $C_1$-$C_4$ alkyl, $C_1$-$C_4$ alkoxy, phenyl, amino, $C_1$-$C_4$ alkylamino or phenylamino;
m is 1 or 2 and
when m is 1, $R_2$ is a group

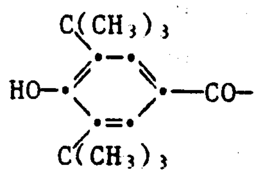

when m is 2 and D is alkyl, $R_2$ is a divalent acyl radical of a phenylenedicarboxylic acid or is a group -CO-C($D_1$)($D_2$)-CO- wherein $D_1$ is $C_1$-$C_4$ alkyl or 3,5-di-t-butyl-4-hydroxybenzyl and $D_2$ is $D_1$ or hydrogen and when m is 2 and D is alkoxy, phenyl, amino or alkylamino,
$R_2$ is a divalent acyl radical of an aliphatic or aromatic dicarboxylic acid having 8-10 C atoms or of an aliphatic dicarbamic acid having 8-10 C atoms; or $R_2$ is a group

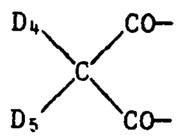

wherein $D_4$ is alkyl or 3,5-di-t-butyl-4-hydroxybenzyl and $D_5$ is $D_4$ or hydrogen;
p is 1 or 2, $R_3$ is $C_1$-$C_4$ alkyl, acetyl or benzoyl,
when p is 1, $R_4$ is $C_1$-$C_{12}$ alkyl or a group

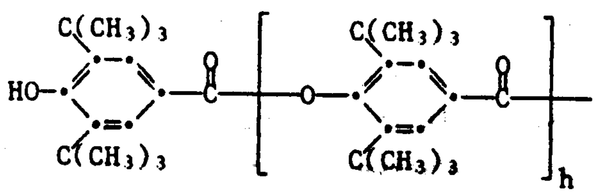

with h as 0 or 1,
and when p is 2, $R_4$ is $C_4$-$C_8$ alkylene;
k is 5 to 50, $T_3$ is ethylene and $Q_1$ is 0;
$T_5$ and $T_6$ are methyl,
$T_{10}$ is hexamethylene and $T_{11}$ is a group of formula II
wherein $T_8$ and $T_9$ are independently hydrogen or $C_1$-$C_{12}$ alkyl or $T_8$ and $T_9$ together are pentamethylene or 3-oxapentamethylene;
$R_{12}$ is acetyl or benzoyl and $G_1$ is a direct bond or -NH-$(CH_2)_6$-NH-.

The compounds of formula A′ are generally prepared by oxidizing the corresponding hindered amine with an appropriate peroxy compound such as hydrogen peroxide or tert-butyl hydroperoxide in the presence of a metal carbonyl or metal oxide catalyst followed by reduction of the oxyl intermediate formed to the desired N-hydroxy derivative, preferably by catalytic hydrogenation.

Thereafter, the N-acyloxy, N-carbamoyloxy and N-(alkoxyacyl)oxy derivatives are prepared by reacting the N-hydroxy hindered amine with the appropriate acid chloride, anhydride, cyanate, isocyanate or substituted chloroformate (for carbonates). The catalytic hydrogenation can also be conducted in acetic anhydride to prepare the N-acetoxy derivative.

The compounds of formula N′ can be prepared analogously by reacting the 1-hydroxy intermediates with an dicarboylic acid derivative or with a diisocyanate.

These reactions are generally conducted at temperatures ranging from 0 to 60 °C and in a variety of solvents including toluene and dichloromethane. The acylations with carboxylic acid chlorides are preferably

conducted in the presence of an acid acceptor such as triethylamine. The various hindered amine precursors are largely commercially available or can be prepared by methods known in the art.

The derivatives are particularly effective in stabilizing organic materials against the degradative effects of actinic stimuli. Such organic materials include polymeric materials such as the following polymers:

1. Polymers of monoolefins and diolefins, for example polypropylene, polyisobutylene, polybutene-1, polymethylpentene-1, polyisoprene or polybutadiene, as well as polymers of cycloolefins, for instance of cyclopentene or norbornene, polyethylene (which optioanlly can be crosslinked), for example high density polyethylene (HDPE), low density polyethylene (LDPE) and linear low density polyethylene (LLDPE).

2. Mixtures of the polymers mentioned under 1), for example mixtures of polypropylene with polyisobutylene, polypropylene with polyethylene (for example PP/HDPE, PP/LDPE) and mixtures of different types of polyethylene (for example LDPE/HDPE).

3. Copolymers of monoolefines and diolefines with each other or with other vinyl monomers, such as, for example, ethylene/propylene, linear low density polyethylene (LLDPE) and its mixtures with low density polyethylene (LDPE), propylene/butene-1, ethylene/hexene, ethylene/ethylpentene, ethylene/heptene, ethylene/octene, propylene/isobutylene, ethylene/butene-1, propylene/butadiene, isobutylene/isoprene, ethylene/alkyl acrylates, ethylene/alkyl methacrylates, ethylene/vinyl acetate or ethylene/ acrylic acid copolymers and their salts (ionomers) and terpolymers of ethylene with propylene and a diene, such as hexadiene, dicyclopentadiene or ethylidene-norbornene; as well as mixtures of such copolymers and their mixtures with polymers mentioned in 1) above, for example polypropylene/ethylene-propylene-copolymers, LDPE/EVA, LDPE/EAA, LLDPE/EVA and LLDPE/EAA.

3a. Hydrocarbon resins (for example $C_5$-$C_9$) and hydrogenated modifications thereof (for example tackyfiers).

4. Polystyrene, poly-(p-methylstyrene), poly-($\alpha$-methylstyrene).

5. Copolymers of styrene or $\alpha$-methylstyrene with dienes or acrylic derivatives, such as, for example, styrene/butadiene, styrene/ acrylonitrile, styrene/alkyl methacrylate, styrene/maleic anhydride, styrene/butadiene/ethyl acrylate, styrene/acrylonitrile/methyl acrylate; mixtures of high impact strength from styrene copolymers and another polymer, such as, for example, from a polyacrylate, a diene polymer or an ethylene/propylene/diene terpolymer; and block copolymers of styrene, such as, for example, styrene/butadiene/ styrene, styrene/ isoprene/styrene, styrene/ethylene/butylene/ styrene or styrene/ ethylene/propylene/styrene.

6. Graft copolymers of styrene or $\alpha$-methylstyrene such as, for example, styrene on polybutadiene, styrene on polybutadiene-styrene or polybutadiene-acrylonitrile; styrene and acrylonitrile (or methacrylonitrile) on polybutadiene; styrene and maleic anhydride or maleimide on polybutadiene; styrene, acrylonitrile and maleic anhydride or maleimide on polybutadiene; styrene, acrylonitrile and methyl methacrylate on polybutadiene, styrene and alkyl acrylates or methacrylates on polybutadiene, styrene and acrylonitrile on ethylene/propylene/diene terpolymers, styrene and acrylonitrile on polyacrylates or polymethacrylates, styrene and acrylonitrile on acrylate/butadiene copolymers, as well as mixtures thereof with the copolymers listed under 5), for instance the copolymer mixtures known as ABS-, MBS-, ASA- or AES-polymers.

7. Halogen-containing polymers, such as polychloroprene, chlorinated rubbers, chlorinated or sulfochlorinated polyethylene, epichlorohydrin homo- and copolymers, polymers from halogen-containing vinyl compounds, as for example, polyvinylchloride, polyvinylidene chloride, polyvinyl fluoride, polyvinylidene fluoride, as well as copolymers thereof, as for example, vinyl chloride/vinylidene chloride, vinyl chloride/vinyl acetate or vinylidene chloride/vinyl acetate copolymers.

8. Polymers which are derived from $\alpha,\beta$-unsaturated acids and derivatives thereof, such as polyacrylates and polymethacrylates, polyacrylamide and polyacrylonitrile.

9. Copolymers from the monomers mentioned under 8) with each other or with other unsaturated monomers, such as, for instance, acrylonitrile/butadiene, acrylonitrile/alkyl acrylate, acrylonitrile/ alkoxyalkyl acrylate or acrylonitrile/vinyl halogenide copolymers or acrylonitrile/alkyl methacrylate/butadiene terpolymers.

10. Polymers which are derived from unsaturated alcohols and amines, or acyl derivatives thereof or acetals thereof, such as polyvinyl alcohol, polyvinyl acetate, polyvinyl stearate, polyvinyl benzoate, polyvinyl maleate, polyvinyl butyral, polyallyl phthalate or polyallylmelamine; as well as their copolymers with olefins mentioned in 1) above.

11. Homopolymers and copolymers of cyclic ethers, such as polyalkylene glycols, polyethylene oxide, polypropylene oxide or copolymers thereof with bis-glycidyl ethers.

12. Polyacetals, such as polyoxymethylene and those polyoxymethylenes which contain ethylene oxide as a comonomer; polyacetals modified with thermoplastic polyurethanes, acrylates or MBS.
13. Polyphenylene oxides and sulfides, and mixtures of polyphenylene oxides with polystyrene or polyamides.
14. Polyurethanes which are derived from polyethers, polyesters or polybutadienes with terminal hydroxyl groups on the one side and aliphatic or aromatic polyisocyanates on the other side, as well as precursors thereof (polyisocyanates, polyols or prepolymers).
15. Polyamides and copolyamides which are derived from diamines and dicarboxylic acids and/or from aminocarboxylic acids or the corresponding lactams, such as polyamide 4, polyamide 6, polyamide 6/6, 6/10, 6/9, 6/12 and 4/6, polyamide 11, polyamide 12, aromatic polyamides obtained by condensation of m-xylene diamine and adipic acid; polyamides prepared from hexamethylenediamine and isophthalic or/and terephthalic acid and optionally an elastomer as modifier, for example poly-2,4,4,-trimethylhexamethylene terephthalamide or poly-m-phenylene isophthalamide. Further copolymers of the aforementioned polyamides with polyolefins, olefin copolymers, ionomers or chemically bonded or grafted elastomers; or with polyethers, such as for instance, with polyethylene glycols, polypropylene glycols or polytetramethylene glycols. Polyamides or copolyamides modified with EPDM or ABS. Polyamides condensed during processing (RIM-polyamide systems).
16. Polyureas, polyimides and polyamide-imides.
17. Polyesters which are derived from dicarboxylic acids and diols and/or from hydroxycarboxylic acids or the corresponding lactones, such as polyethylene terephthalate, polybutylene terephthalate, poly-1,4-dimethylolcyclohexane terephthalate, poly-[2,2,-(4-hydroxyphenyl)-propane] terephthalate and polyhydroxybenzoates as well as block-copolyether-esters derived from polyethers having hydroxyl end groups.
18. Polycarbonates and polyester-carbonates.
19. Polysulfones, polyether-sulfones and polyether-ketones.
20. Crosslinked polymers which are derived from aldehydes on the one hand and phenols, ureas and melamines on the other hand, such as phenol/formaldehyde resins, urea/formaldehyde resins and melamine/formaldehyde resins.
21. Drying and non-drying alkyd resins.
22. Unsaturated polyester resins which are derived from copolyesters of saturated and unsaturated dicarboxylic acids with polyhydric alcohols and vinyl compounds as crosslinking agents, and also halogen-containing modifications thereof of low inflammability.
23. Thermosetting acrylic resins, derived from substituted acrylic esters, such as epoxy-acrylates, urethane-acrylates or polyester-acrylates.
24. Alkyd resins, polyester resins or acrylate resins in admixture with melamine resins, urea resins, polyisocyanates or epoxide resins as crosslinking agents.
25. Crosslinked epoxide resins which are derived from polyepoxides, for example from bis-glycidyl ethers or from cycloaliphatic diepoxides.
26. Natural polymers, such as cellulose, rubber, gelatine and derivatives thereof which are chemically modified in a polymer-homologous manner, such as cellulose acetates, cellulose propionates and cellulose butyrates, or the cellulose ethers, such as methylcellulose; rosins and their derivatives.
27. Mixtures of polymers as mentioned above, for example PP/EPDM, Polyamide 6/EPDM or ABS, PVC/EVA, PVC/ABS, PVC/MBS, PC/ABS, PBTP/ABS, PC/ASA, PC/PBT, PVC/CPE, PVC/acrylates, POM/thermoplastic PUR, PC/thermoplastic PUR, POM/acrylate, POM/MBS, PPE/HIPS, PPE/PA 6.6 and copolymers, PA/HDPE, PA/PP, PA/PPE.

The instant compounds are added to the polymers in a concentration of 0.05 to 5 % by weight, calculated relative to the material to be stabilized. Preferably, 0.1 to 2.5 % by weight of the stabilizer calculated relative to the material to be stabilized, is incorporated into the latter.

Incorporation can be effected during the polymerization or after polymerization, for example by mixing the compounds and, if desired, further additives into the melt by the methods customary in the art, before or during shaping, or by applying the dissolved or dispersed compounds to the polymer.

Further additives used in combination with the instant compounds may be other stabilizers such as phenolic antioxidants, metal desactivators, phosphites, thiodipropionic diesters, fatty acid salts, UV-absorbers or nickel complex salts. Further additives may be pigments, fillers, plasticizers, flame retardants or antistatica.

In general, the stabilizers of this invention are employed from about 0.05 to about 5 % by weight of the stabilized composition, although this will vary with the particular substrate and application. An advantageous range is from about 0.1 to about 2.5 %.

The compounds are particularly active as light stabilizers in ambient cured and acid catalyzed thermoset coatings or enamels. Since these materials are considerably less basic than conventional hindered amines, they do not inhibit or interfere with cure as is encountered with the conventional hindered amines nor are they interacting. They likewise do not exhibit the color problems encountered with nitroxyl radicals and, unlike N-hydroxy hindered amines, tend to resist air oxidation during handling. Finally, the N-acyloxy hindered amines exhibit greater solubility in the solvents typically utilized in coatings. These areas are further described in a copending application.

The compounds alone or in combination with phenols can further be used in photographic layers as yellow dye light stabilizers, as cyan dye dark stabilizers, as antistain agents in magenta layers (especially for two-equivalent magenta couplers) and as thermal stabilizers for magenta couplers.

The following examples further illustrate the preparation of these compounds.

Example 13 Di-(1-acetoxy-2,2,6,6-tetramethylpiperidin-4-yl) Phthalate

A mixture of 20.0 g (42 mmol) of di-(1-oxyl-2,2,6,6-tetramethyl-piperidin-4-yl) phthalate, 100 ml of acetic anhydride, and 500 mg of 5 % Pd on C is hydrogenated in a Parr apparatus (50 psi, ambient temperature, 1 hour). Catalyst is filtered and solvent is evaporated. The residue is dissolved in 150 ml of ethyl acetate. The ethyl acetate solution is washed with 5 % aqueous ammonia (2 x 100 ml), water (100 ml), and saturated sodium chloride (100 ml), then dried over magnesium sulfate and concentrated to obtain a crude solid. The crude product is recrystallized from methanol to obtain 14.9 g (64 % yield) of a white crystalline solid, m.p. 172-175 °C.

| | | | |
|---|---|---|---|
| Anal. Calcd. for $C_{30}H_{44}N_2O_8$: | C, 64.3; | H, 7.9; | N, 5.0. |
| Found: | C, 64.5; | H, 8.2; | N, 5.1. |

Example 14 Di-(1-acetoxy-2,2,6,6-tetramethylpiperidin-4-yl) Isophthalate

The compound is prepared according to the procedure given for Example 13 utilizing the isophthalate in place of the phthalate, m.p. 98-101 °C.

| | | | |
|---|---|---|---|
| Anal. Calcd. for $C_{30}H_{44}N_2O_8$: | C, 64.3; | H, 7.9; | N, 5.0. |
| Found: | C, 62.4; | H, 8.0; | N, 5.0. |

Example 15 Poly-{[6-[(1,1,3,3-tetramethylbutyl)-imino]-1,3,5-triazine-2,4-diyl] [2-(1-acetoxy-2,2,6,6-tetramethylpiperidyl)-imino]-hexamethylene-[4-(1-acetoxy-2,2,6,6-tetramethylpiperidyl]-imino]}

The compound is prepared according to the procedure given for Example 13, utilizing the corresponding 1-oxyl compound
m.p. 110-120 °C (glass transition).

| | | | |
|---|---|---|---|
| Anal. Calcd. for $(C_{39}H_{70}N_8O_4)_n$: | C, 65.5; | H, 9.9; | N, 15.7. |
| Found: | C, 65.6; | H, 9.4; | N, 13.0. |

Example 16 Polymer of 1-acetoxy-4-acryloxy-2,2,6,6-tetramethylpiperidine

A solution of 20.0 g (74.3 mmol) of 1-acetoxy-4-acryloxy-2,2,6,6-tetramethylpiperidine and 0.19 g of azobisisobutyronitrile in 30 ml of dry toluene is added over 2 hours to 25 ml of dry toluene maintained at 100 °C. The reaction mixture is stirred an additional 15 minutes. A solution of 35 mg of n-dodecyl mercaptan in 2 ml of toluene is added, and the reaction mixture is then poured into cold hexane to yield a precipitate. The precipitate is dissolved in ether, and cold hexane is added to afford a white precipitate which is dried to give 16.4 g (82 % yield) of a brittle glass.
IR: 1755,1720 $cm^{-1}$.

| | | | |
|---|---|---|---|
| Anal. Calcd. for $(C_{14}H_{23}NO_4)_n$: | C, 62.4; | H, 8.6; | N, 5.2. |
| Found: | C, 62.8; | H, 8.6; | N, 5.1. |

Example 17 1-Acetoxy-2,2,6,6-tetramethylpiperidin-4-yl 4-Hydroxy-3,5-di-tert-butylbenzoate

A solution of 20.0 g (92.9 mmol) of 1-acetoxy-4-hydroxy-2,2,6,6-tetramethylpiperidine in 10.3 g (102 mmol) of triethylamine and 210 ml of dichloromethane is cooled below 0°C. To this solution is added, under nitrogen, a solution of 25.0 g (92.9 mmol) of 4-hydroxy-3,5-di-tert-butylbenzoyl chloride over a 30 minutes interval. During the addition the reaction temperature is maintained at -3 to 0°C. The reaction mixture is stirred at room temperature for 3 hours, then diluted with hexane (200 ml). Triethylamine hydrochloride is removed by filtration, and the filtrate is washed with 1N HCl (200 ml) and saturated sodium bicarbonate solution (200 ml). The solution is dried over magnesium sulfate and concentrated to an oil which is triturated in hexane to yield 1.5 g of a white solid impurity.

The mother liquor is concentrated and crystallized from methanoldichloromethane to yield 11.6 g (28 %) of a white solid, m.p. 154-156°C, which is the title compound.

| | | | |
|---|---|---|---|
| Anal. Calcd. for $C_{26}H_{41}NO_5$: | C, 69.7; | H, 9.2; | N, 3.1. |
| Found: | C, 69.7; | H, 9.4; | N, 3.1. |

Example 18 Di-(1-acetoxy-2,2,6,6-tetramethylpiperidin-4-yl) 2-(4-Hydroxy-3,5-di-tert-butylbenzyl) n-butylmalonate

To 10 mmol of lithium diisopropylamide in 25 ml of anhydrous tetrahydrofuran is added a solution of 27.6 g (50 mmol) of di-(1-acetoxy-2,2,6,6-tetramethylpiperidin-4-yl) n-butylmalonate in 90 ml of THF followed by a solution of 16.5 g (62.5 mmol) of 4-dimethylaminomethyl-2,6-di-tert-butylphenol in 50 ml THF. The reaction mixture is refluxed for 2 hours. The reaction mixture is concentrated under reduced pressure and the residue is dissolved in ether (300 ml). The ether solution is washed with 1N HCl (300 ml) and saturated sodium bicarbonate solution (150 ml), then dried over magnesium sulfate and concentrated. The resulting crude solid is recrystallized from hexane to give 25.7 g (66 % yield) of a white solid, m.p. 174-175°C.

| | | | |
|---|---|---|---|
| Anal. Calcd. for $C_{44}H_{72}N_2O_9$: | C, 68.4; | H, 9.4; | N, 3.6. |
| Found: | C, 68.1; | H, 9.4; | N, 3.8. |

Example 19 N-(1-Acetoxy-2,2,6,6-tetramethylpiperidin-4-yl)-N-(n-butyl)-4-(4-hydroxy-3,5-di-t-butylbenzoyloxy)-3,5-di-t-butylbenzamide

A solution of 20.9 g (77.6 mmol) of 4-hydroxy-3,5-di-tert-butylbenzoyl chloride in 100 ml of dichloromethane is added dropwise over 30 minutes under nitrogen to a chilled solution (0°C) of 20.0 g (64.7 mol) of 1-acetoxy-4-butylamino-2,2,6,6-tetramethylpiperidine hydrochloride in 19.6 g (194 mmol) of triethylamine and 100 ml of dichloromethane. The reaction temperature is maintained below 5°C during the addition. The reaction mixture is stirred for 3 hours at room temperature, then diluted with ether (200 ml) and filtered. The filtrate is washed with 1N HCl (2 x 100 ml) and sodium bicarbonate solution (200 ml). The solution is dried over magnesium sulfate, concentrated, and chromatographed on silica gel (3:1 hexane:ethyl acetate). The major reaction product is crystallized from methanol to yield 13.0 g of a white solid, m.p. 230-232°C.

| | | | |
|---|---|---|---|
| Anal. Calcd. for $C_{45}H_{70}N_2O_6$: | C, 73.5; | H, 9.6; | N, 3.8. |
| Found: | C, 72.6; | H, 9.4; | N, 3.7. |

Example 20 N-(1-Acetoxy-2,2,6,6-tetramethylpiperidin-4-yl)-N-n-butyl-4-hydroxy-3,5-di-t-butylbenzamide

A second recrystallization crop from Example 19 is recrystallized until a constant melting point is obtained. The yield is 4.4 g of a white solid, m.p. 161-164°C, with an analysis consistent with the title compound.

| | | | |
|---|---|---|---|
| Anal. Calcd. for $C_{30}H_{50}N_2O_4$: | C, 71.7; | H, 10.0; | N, 5.6. |
| Found: | C, 72.0; | H, 10.2; | N, 5.5. |

Example 21 1,6-Di-[N-acetyl-N-(1-acetoxy-2,2,6,6-tetramethylpiperidin-4-yl)]-aminohexane

A mixture of 52.8 g (310 mmol) of 1-oxyl-2,2,6,6-tetramethylpiperidin-4-one, 17.4 g (150 mmol) of 1,6-hexanediamine, methanol (10.0 ml), water (150 ml), and platinum oxide (500 mg) is hydrogenated in a Parr apparatus for 18 hours at 50 psi (ambient temperature). Chloroform (1000 ml) is added, and the catalyst is filtered. The organic phase is concentrated to obtain 44.4 g (69 %) of 1,6-di-[N-(1-hydroxy-2,2,6,6-tetramethylpiperidin-4-yl)]-aminohexane. The hydroxylamine (19.5 g, 45.7 mmol) is added to 150 ml of acetic anhydride over 5 minutes, with the reaction temperature reaching 50°C during the addition. The reaction mixture is refluxed for 30 minutes, then rehydrogenated (400 mg 5 % Pd on C, 50 psi, 3 hours) to reduce any remaining nitroxyl radical. The catalyst is removed by filtration, and solvent is evaporated. The residue is crystallized from 4:1 toluene:heptane to give 14:1 g (52 % yield) of a white solid, m.p. 169-170°C.

| | | | |
|---|---|---|---|
| Anal. Calcd. for $C_{32}H_{58}N_4O_6$: | C, 64.6; | H, 9.8; | N, 9.4. |
| Found: | C, 64.8; | H, 9.8; | N, 9.3. |

Example 22 Di-(1-acetoxy-2,2,6,6-tetramethylpiperidin-4-yl) N,N′-(1,6-Hexanediyl)dicarbamate

A mixture of 15.0 g (70 mmol) of 1-acetoxy-4-hydroxy-2,2,6,6-tetramethylpiperidine, 5.9 g (35 mmol) of 1,6-hexanediisocyanate, toluene (125 ml), and dibutyltin dilaurate (200 mg) is refluxed for 1 hour. The hot reaction mixture is poured into hexane (500 ml). The resulting precipitate is removed by filtration and triturated in ether to give 13.1 g (63 % yield) of a white solid, m.p. 158-163°C.

| | | | |
|---|---|---|---|
| Anal. Calcd. for $C_{30}H_{54}N_4O_8$: | C, 60.2; | H, 9.1; | N, 9.4. |
| Found: | C, 60.1; | H, 9.1; | N, 10.0. |

Example 23 1-Acetoxy-4-(N-acetyl-N-n-dodecylamino)-2,2,6,6-tetramethylpiperidine

A mixture of 42.6 g (250 mmol) of 1-oxyl-2,2,6,6-tetramethylpiperidin-4-one, 47.0 g (250 mmol) of n-dodecylamine, 600 mg of platinum oxide and 200 ml of toluene is hydrogenated in a Parr apparatus (50 psi, ambient temperature) for 3 hours. Acetic anhydride (77.9 g, 760 mmol) is added. The catalyst is removed by filtration, and the filtrate is refluxed for 3 hours. Solvent is evaporated at reduced pressure, and the residue is dissolved in 2:1 hexane:ether (500 ml). The solution is washed with 1N HCl (400 ml), saturated sodium bicarbonate solution (200 ml), and saturated sodium chloride solution (200 ml), then dried over magnesium sulfate and concentrated. The residue is chromatographed on silica gel (1:1 hexane:ethyl acetate) to obtain 42.4 g (40 % yield) of a yellow oil.

| | | | |
|---|---|---|---|
| Anal. Calcd. for $C_{25}H_{48}N_2O_3$: | C, 70.7; | H, 11.4; | N, 6.6. |
| Found: | C, 71.0; | H, 11.3; | N, 6.3. |

Example 24 Di-(4-n-octadecanoyloxy-2,2,6,6-tetramethylpiperidin-1-yl) Oxalate

A solution of 4.33 g (34.1 mmol) of oxalyl chloride in 50 ml of dichloromethane is added dropwise over 20 minutes to a solution of 30.0 g (68.2 mmol) of 1-hydroxy-2,2,6,6-tetramethylpiperidin-4-yl) stearate in 8.3 g (81.8 mmol) of triethylamine and 100 ml dichloromethane in a nitrogen atmosphere. The reaction tempeature rises from 20° to 35°C during the addition. The reaction mixture is stirred overnight at room temperature; the triethylamine hydrochloride formed is removed by filtration, and the filtrate is diluted to a total volume of 300 ml with dichloromethane. The solution is washed with 1N HCl (2 x 100 ml) and saturated sodium bicarbonate solution (200 ml), dried over magnesium sulfate, and concentrated to obtain a dark brown solid. The solid is suspended in methanol and filtered. The isolated solid is recrystallized twice from heptane (filtrol added to decolorize) to obtain 9.3 g (29 % yield) of white crystals, m.p. 104-105°C.

| | | | |
|---|---|---|---|
| Anal. Calcd. for $C_{56}H_{104}N_2O_8$: | C, 72.1; | H, 11.2; | N, 3.0. |
| Found: | C, 72.1; | H, 11.3; | N, 3.2. |

Example 25 Di-(4-benzoyloxy-2,2,6,6-tetramethylpiperidin-1-yl) Oxalate

The compound is prepared according to the procedure given for Example 24, utilizing the corresponding benzoate.
m.p. 244°C (dec).

| | | | |
|---|---|---|---|
| Anal. Calcd. for $C_{34}H_{44}N_2O_8$: | C, 67.1; | H, 7.3; | N, 4.6. |
| Found: | C, 66.8; | H, 7.4; | N, 4.5. |

Example 26 Di-(1-benzoyloxy-2,2,6,6-tetramethylpiperidin-4-yl) Sebacate

Benzoyl chloride (29.5 g, 210 mmol) is added dropwise over 30 minutes to a mixture of 51.3 g (100 mmol) of di-(1-hydroxy-2,2,6,6-tetramethylpiperidin-4-yl) sebacate, 250 ml of toluene, and 30.4 g (300 mmol) of triethylamine under nitrogen. The temperature rises from 22° to 48°C during the addition. The reaction mixture is then heated at 70°C for 45 minutes, cooled below 40°C, and diluted with 150 ml of toluene. Triethylamine hydrochloride is filtered off, and the filtrate is washed with 10 % aqueous ammonia (200 ml), warm water (3 x 400 ml), 1N HCl (2 x 200 ml), saturated sodium bicarbonate solution (200 ml) and saturated sodium chloride solution (200 ml). The solution is dried over magnesium sulfate and concentrated. The residue is recrystallized from methanol to obtain 51.5 g (71 % yield) of a white solid, m.p. 92-100°C.

| | | | |
|---|---|---|---|
| Anal. Calcd. for $C_{42}H_{60}N_2O_8$: | C, 70.0; | H, 8.4; | N, 3.9. |
| Found: | C, 69.8; | H, 8.6; | N, 3,9. |

Example 27 1-Benzoyloxy-4-(N-n-butylbenzyolamino)-2,2,6,6-tetramethylpiperidine

The compound is prepared from the reaction of 1-hydroxy-4-(N-n-butylamino)-2,2,6,6-tetramethylpiperidine with benzoyl chloride following a procedure similar to that used for Example 26.
m.p. 155-159°C.

| | | | |
|---|---|---|---|
| Anal. Calcd. for $C_{27}H_{36}N_2O_3$: | C, 74.3; | H, 8.3; | N, 6.4. |
| Found: | C, 74.0; | H, 8.4; | N, 6.3. |

Example 28 (1-Benzoyloxy-2,2,6,6-tetramethylpiperidin-4-yl)(1'-Benzyloxy-2,2,6,6-tetramethylpiperidin-4-yl) Isophthalate

A mixture of 35.0 g (78.7 mmol) of di-(2,2,6,6-tetramethylpiperidin-4-yl) isophthalate, 1.0 g of molybdenum hexacarbonyl, and 75 ml of toluene is heated to 90°C in a nitrogen atmosphere. A solution of 4.2 M t-butyl hydroperoxide in toluene (225 ml, 945 mmol) is added over 5 minutes and the reaction mixture turns red. After the addition, the reaction mixture is irradiated for 6 hours (internal temperature 85°C) with a UV lamp. Another 1.0 g portion of molybdenum hexacarbonyl is added, and the reaction mixture is irradiated for 16 hours. The mixture was then filtered and concentrated and the crude residue is chromatographed on silica gel (9:1 hexane:ethyl acetate). The more polar of the two major reaction products is recrystallized from ethanol to obtain 12.0 g (23 % yield) of the title compound, a white solid with m.p. 137-140°C.

| | | | |
|---|---|---|---|
| Anal. Calcd. for $C_{40}H_{50}N_2O_7$: | C, 71.6; | H, 7.5; | N, 4.2. |
| Found: | C, 71.7; | H, 7.8; | N, 4.4. |

Example 29 1,4-Di-(4-hydroxy-3,5-di-tert-butylbenzoyloxy)-2,2,6,6-tetramethylpiperidine

A solution of 31.2 g (116 mmol) of 4-hydroxy-3,5-di-tert-butylbenzoyl chloride in 100 ml of toluene is added dropwise over 3 minutes to a mixture of 10.0 g (58.8 mmol) of 1,4-dihydroxy-2,2,6,6-tetramethylpiperidine and 15.5 g (128 mmol) of N,N-dimethylaniline in 50 ml of toluene in a nitrogen atmosphere. The reaction mixture is heated at 80° for three hours, then diluted to 400 ml. The solution is washed with 1N HCl, saturated sodium bicarbonate solution, and saturated sodium chloride solution, then dried over magnesium sulfate and concentrated. The yellow residue is decolorized with DARCO G-60 in 2-propanol. Crystallization gives 14.0 g (38 % yield) of a white solid, m.p. 196°C(dec).

| | | | |
|---|---|---|---|
| Anal. Calcd. for $C_{39}H_{59}NO_6$: | C, 73.4; | H, 9.3; | N, 2.2. |
| Found: | C, 73.3; | H, 9.3; | N, 2.1. |

Example 30 1-Carbamoyloxy-4-benzoyloxy-2,2,6,6-tetramethylpiperidine

A solution of 6.7 ml of 12 N HCl in 10 ml of water is added dropwise over 10 minutes with cooling below 0°C to a suspension of 20.8 g (75 mmol) of 1-hydroxy-4-benzoyloxy-2,2,6,6-tetramethylpiperidine, 75 ml of methanol, and 25 ml of water in a nitrogen atmosphere. A clear solution resulted. A solution of 6.1 g (75 mmol) of potassium cyanate in 25 ml of water is added dropwise over 30 minutes. The reaction temperature is maintained at 0-5°C during the cyanate addition. The reaction mixture is stirred 30 minutes at ambient temperature, then filtered. The precipitate is washed with water, then dissolved in 200 ml of toluene. Residual water is removed by azeotropic distillation. The toluene solution is then cooled to yield 19.4 g (81 %) of a white crystalline solid, m.p. 148-149°C.

| | | | |
|---|---|---|---|
| Anal. Calcd. for $C_{17}H_{24}N_2O_4$: | C, 63.7; | H, 7.6; | N, 8.7. |
| Found: | C, 63.5; | H, 7.8; | N, 8.8. |

Example 31 Di-(1-carbamoyloxy-2,2,6,6-tetramethylpiperidin-4-yl) Sebacate

The compound is prepared according to the procedure given in Example 30.

| | | | |
|---|---|---|---|
| Anal. Calcd. for $C_{30}H_{54}N_4O_8$: | C, 60.2; | H, 9.1; | N, 9.4. |
| Found: | C, 60.3; | H, 9.0; | N, 9.1. |

Example 32 Di-(1-phenylcarbamoyloxy-2,2,6,6-tetramethylpiperidin-4-yl) Sebacate

Phenyl isocyanate (9.5 g, 80 mmol) is added over 5 minutes to a suspension of 20.0 g (39 mmol) of di-(1-hydroxy-2,2,6,6-tetramethylpiperidin-4-yl) sebacate in 125 ml of dichloromethane. The resulting solution is refluxed for 30 minutes and then evaporated to obtain a solid. The crude solid is briefly refluxed in methanol, filtered off, and recrystallized from 2-propanol:dichloromethane to give 22.2 g (76 % yield) of white solid, m.p. 159-161 °C(dec).

| | | | |
|---|---|---|---|
| Anal. Calcd. for $C_{42}H_{62}N_4O_8$: | C, 67.2; | H, 8.3; | N, 7.5. |
| Found: | C, 66.9; | H, 8.3; | N, 7.3. |

Example 33 4-Benzoyloxy-1-n-butylcarbamoyloxy-2,2,6,6-tetramethylpiperidine

A solution of 4.66 g (40 mmol) of n-butyl isocyanate in 10 ml of toluene is added over 10 minutes to a suspension of 10.6 g (38.2 mmol) of 1-hydroxy-4-benzoyloxy-2,2,6,6-tetramethylpiperidine in 40 ml of toluene. The reaction mixture is heated at 50 °C for 30 minutes. The reaction mixture is then filtered, and the residue from evaporation of the filtrate is crystallized from hexane to give 4.2 g (29 % yield) of a white solid, m.p. 126-127 °C.

| | | | |
|---|---|---|---|
| Anal. Calcd. for $C_{21}H_{32}N_2O_4$: | C, 67.0; | H, 8.6; | N, 7.5. |
| Found: | C, 67.2; | H, 8.7; | N, 7.4. |

Example 34 Di-(1-n-butylcarbamoyloxy-2,2,6,6-tetramethylpiperidin-4-yl) Phthalate

The compound is prepared in 65 % yield according to the procedure given in Example 21, except that the appropriate phthalate and dichloromethane solvent are utilized.
m.p. 180-182 °C.

| | | | |
|---|---|---|---|
| Anal. Calcd. for $C_{36}H_{58}N_4O_8$: | C, 64.1; | H, 8.7; | N, 8.3. |
| Found: | C, 63.9; | H, 8.8; | N, 8.0. |

Example 35 Di-(1-n-butylcarbamoyloxy-2,2,6,6-tetramethylpiperidin-4-yl) Isophthalate

The compound is prepared according to the procedure given in Example 34 except for the use of the isophthalate.
m.p. 176-177 °C.

| | | | |
|---|---|---|---|
| Anal. Calcd. for $C_{36}H_{58}N_4O_8$: | C, 64.1; | H, 8.7; | N, 8.3. |
| Found: | C, 64.1; | H, 8.4; | N, 8.3. |

Example 36 Di-(1-n-butylcarbamoyloxy-2,2,6,6-tetramethylpiperidin-4-yl) 2,2-Diethylmalonate

The compound is prepared according to the procedure given in Example 34 except for the use of the appropriate malonate.
m.p. 166-169 °C.

| | | | |
|---|---|---|---|
| Anal. Calcd. for $C_{35}H_{64}N_4O_8$: | C, 62.8; | H, 9.6; | N, 8.4. |
| Found: | C, 63.2; | H, 9.6; | N, 8.3. |

Example 37 Di-(1-n-butylcarbamoyloxy-2,2,6,6-tetramethylpiperidin-4-yl) n-Butylmalonate

The compound is prepared according to the procedure given in Example 34. m.p. 129-131 °C.

| | | | |
|---|---|---|---|
| Anal. Calcd. for $C_{35}H_{64}N_4O_8$: | C, 62.8; | H, 9.6; | N, 8.4. |
| Found: | C, 63.1; | H, 9.5; | N, 8.3. |

Example 38 Di-(4-benzoyloxy-2,2,6,6-tetramethylpiperidin-1-yl) N,N'-(2,4,4-trimethyl-1,6-hexanediyl)-dicarbamate

The compound is prepared from 4-benzoyloxy-1-hydroxy-2,2,6,6-tetramethylpiperidine and 2,4,4-trimethylhexane-1,6-diisocyanate according to the procedure given in Example 33. m.p. 165-167 °C.

| | | | |
|---|---|---|---|
| Anal. Calcd. for $C_{43}H_{64}N_4O_8$: | C, 67.5; | H, 8.4; | N, 7.3. |
| Found: | C, 67.4; | H, 9.0; | N, 7.4. |

Example 39 n-Butyl (4-Benzoyloxy-2,2,6,6-tetramethylpiperidin-1-yl) Carbonate

A solution of 7.8 g (81 mmol) of n-butylchloroformate in 15 ml of toluene is added dropwise over 20 minutes to a mixture of 15.0 g (54 mmol) of 1-hydroxy-4-benzoyloxy-2,2,6,6-tetramethylpiperidine, 8.2 ml (101 mmol) of triethylamine, and 75 ml of toluene in a nitrogen atmosphere. During the addition, the reaction temperature is maintained below 10 °C with an ice bath. After the addition is complete, the reaction mixture is stirred two hours at room temperature. Triethylamine hydrochloride is filtered, the filtrate washed with 1N HCl (200 ml), saturated sodium bicarbonate (200 ml), and saturated sodium chloride (200 ml), then dried over magnesium sulfate and concentrated. The resulting oil is crystallized from hexane to give 14.1 g (69 % yield) of a white solid, m.p. 83-84 °C.

| | | | |
|---|---|---|---|
| Anal. Calcd. for $C_{21}H_{31}NO_4$: | C, 66.8; | H, 8.3; | N, 3.7. |
| Found: | C, 66.8; | H, 8.4; | N, 3.9. |

Summarizing, this invention is seen to provide a series of new O-acyl, O-carbamoyl and O-carbonate substituted N-hydroxy hindered amine stabilizers. Variations may be made in proportions, procedures and materials without departing from the scope of the invention as defined by the following claims.

## Claims

**Claims for the following Contracting States : BE, DE, FR, GB, IT, NL, SE**

1. A stabilized ambient temperature curable or acid catalyzed thermosetting coating composition containing an effective stabilizing amount of a hindered amine compound containing the group

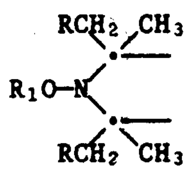

wherein R is hydrogen or methyl and $R_1$ is a group

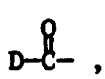

wherein D is $C_1$-$C_{18}$ alkyl, $C_1$-$C_{18}$ alkoxy, phenyl, phenyl substituted by hydroxy, $C_1$-$C_4$ alkyl or $C_1$-$C_4$ alkoxy, or D is amino or amino mono- or disubstituted by $C_1$-$C_{12}$ alkyl or phenyl.

**2.** The composition according to claim 1, which contains a hindered amine compound corresponding to one of the formulae A-N

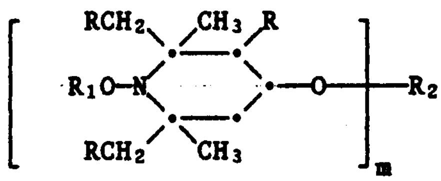

(A)

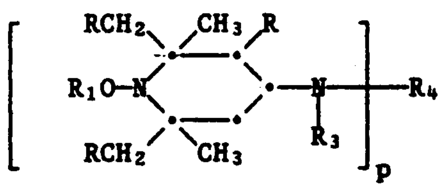

(B)

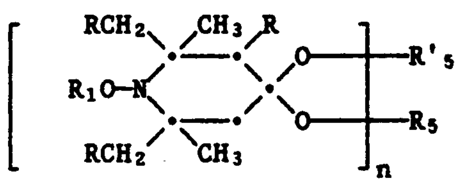

(C)

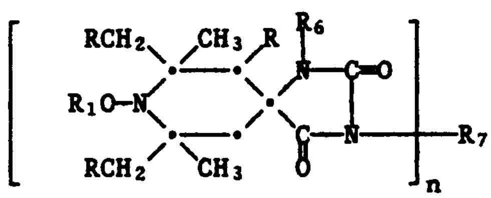

(D)

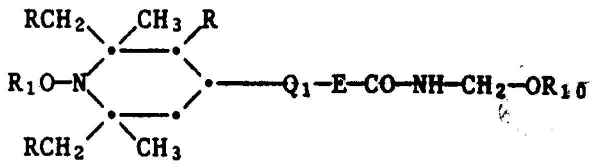

(E)

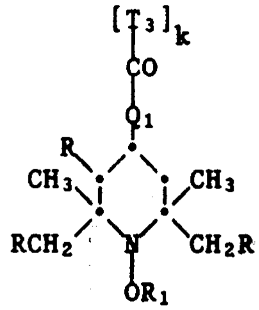

(F)

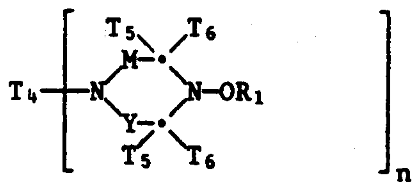

(G)

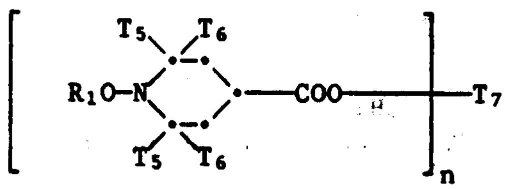

(H)

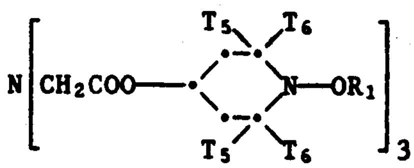

(I)

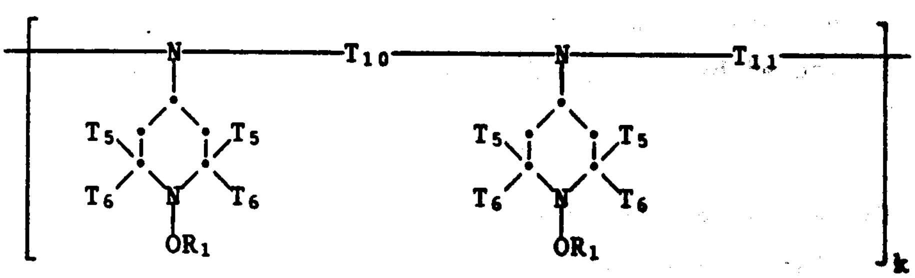

(J)

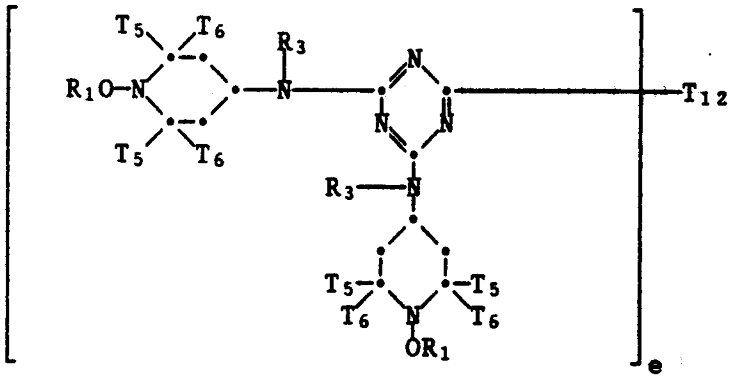

(K)

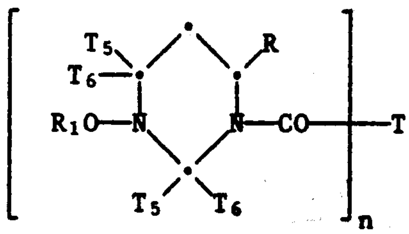

(L)

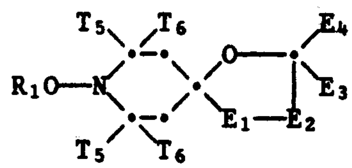

(M)

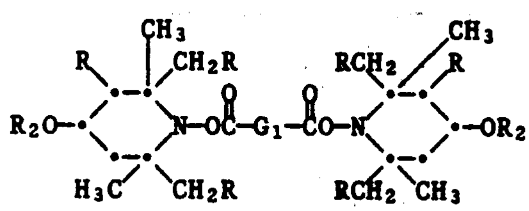

(N)

wherein
R is hydrogen or methyl,
$R_1$ is a group D-CO- , wherein D is $C_1$-$C_{18}$ alkyl, $C_1$-$C_{18}$ alkoxy, phenyl, phenyl substituted by hydroxy, $C_1$-$C_4$ alkyl or $C_1$-$C_4$ alkoxy, or amino or amino mono- or disubstituted by $C_1$-$C_{12}$ alkyl or phenyl;
m is 1-4,
when m is 1,
$R_2$ is hydrogen, $C_1$-$C_{18}$ alkyl optionally interrupted by one or more oxygen atoms, $C_2$-$C_{12}$ alkenyl, $C_6$-$C_{10}$ aryl, $C_7$-$C_{18}$ aralkyl, glycidyl, a monovalent acyl radical of an aliphatic, cycloaliphatic, araliphatic or aromatic carboxylic acid, or of a carbamic acid, or $R_2$ is a group

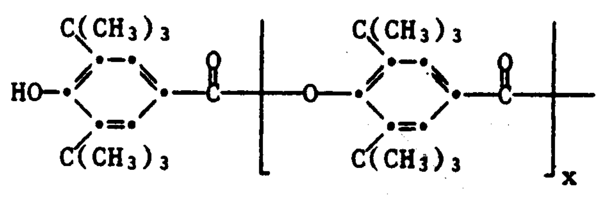

wherein x is 0 or 1, or is a group

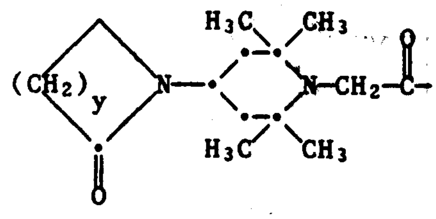

wherein y is 2-4;
when m is 2,
$R_2$ is $C_1$-$C_{12}$ alkylene, $C_4$-$C_{12}$ alkenylene, xylylene, a divalent acyl radical of an aliphatic, cycloaliphatic, araliphatic or aromatic dicarboxylic acid or of a dicarbamic acid, or $R_2$ is a group

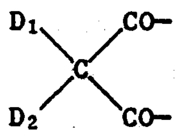

or

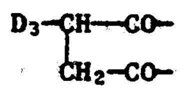

wherein $D_1$ and $D_2$ independently are hydrogen, an alkyl radical containing up to 8 carbon atoms, phenyl, benzyl or 3,5-di-t-butyl-4-hydroxybenzyl, and $D_3$ is an alkyl or alkenyl radical containing up to 18 carbon atoms;
when m is 3, $R_2$ is a trivalent acyl radical of an aliphatic, cycloaliphatic, or aromatic tricarboxylic acid;
when m is 4, $R_2$ is a tetravalent acyl radical of a saturated or unsaturated aliphatic or aromatic tetracarboxylic acid; p is 1, 2 or 3,
$R_3$ is hydrogen, $C_1$-$C_{12}$ alkyl, $C_5$-$C_7$ cycloalkyl, $C_7$-$C_9$ aralkyl, $C_2$-$C_{18}$ alkanoyl, $C_3$-$C_5$ alkenoyl or benzoyl;
when p is 1,
$R_4$ is hydrogen, $C_1$-$C_{18}$ alkyl, $C_5$-$C_7$ cycloalkyl, $C_2$-$C_8$ alkenyl unsubstituted or substituted by a cyano, carbonyl or carbamide group, or it is aryl, aralkyl, glycidyl, a group of the formula $-CH_2-CH(OH)-Z$ or of the formula -CONH-Z wherein Z is hydrogen, methyl or phenyl;
or $R_4$ is a group of the formula I

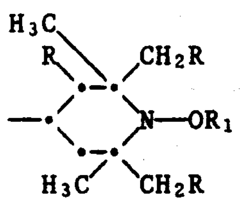

(I) or

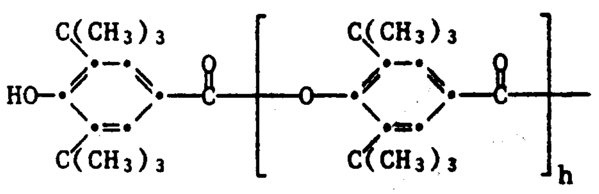

with h as 0 or 1;

or $R_3$ and $R_4$ together are alkylene of 4 to 6 carbon atoms or 1-oxo alkylene or the divalent acyl radical of an aliphatic or aromatic 1,2- or 1,3-dicarboxylic acid;
when p is 2,
$R_4$ is $C_1$-$C_{12}$ alkylene, $C_6$-$C_{12}$ arylene, xylylene, a $-CH_2CH(OH)-CH_2$ group, or a group $-CH_2-CH(OH)-CH_2-O-X-O-CH_2-CH(OH)-CH_2-$ wherein X is $C_2$-$C_{10}$ alkylene, $C_6$-$C_{15}$ arylene or $C_6$-$C_{12}$ cycloalkylene; or, provided that $R_3$ is not alkanoyl, alkenoyl or benzoyl, $R_4$ can also be a divalent acyl radical of an aliphatic, cycloaliphatic or aromatic dicarboxylic or dicarbamic acid, or can be the group -CO-; or $R_4$ is a group of formula II

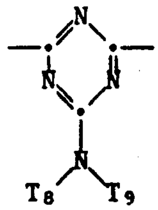 (II)

where $T_8$ and $T_9$ are independently hydrogen, alkyl of 1 to 18 carbon atoms, or $T_8$ and $T_9$ together are alkylene of 4 to 6 carbon atoms or 3-oxapentamethylene, preferably $T_8$ and $T_9$ together are 3-oxapentamethylene;
when p is 3, $R_4$ is 2,4,6-triazinetriyl;
n is 1 or 2 and
when n is 1,
$R_5$ and $R'_5$ are independently $C_1$-$C_{12}$ alkyl, $C_2$-$C_{12}$ alkenyl, $C_7$-$C_{12}$ aralkyl, or $R_5$ is also hydrogen, or $R_5$ and $R'_5$ together are $C_2$-$C_8$ alkylene or hydroxyalkylene or $C_4$-$C_{22}$ acyloxyalkylene;
when n is 2,
$R_5$ and $R'_5$ together are $(-CH_2)_2C(CH_2-)_2$;
$R_6$ is hydrogen, $C_1$-$C_{12}$ alkyl, allyl, benzyl, glycidyl or $C_2$-$C_6$ alkoxyalkyl;
when n is 1,
$R_7$ is hydrogen, $C_1$-$C_{12}$ alkyl, $C_3$-$C_5$ alkenyl, $C_7$-$C_9$ aralkyl, $C_5$-$C_7$ cycloalkyl, $C_2$-$C_4$ hydroxyalkyl, $C_2$-$C_6$ alkoxyalkyl, $C_6$-$C_{10}$ aryl, glycidyl, a group of the formula $-(CH_2)_t$-COO-Q or of the formula $-(CH_2)_t$-O-CO-Q wherein t is 1 or 2, and Q is $C_1$-$C_4$ alkyl or phenyl; or
when n is 2,
$R_7$ is $C_2$-$C_{12}$ alkylene, $C_6$-$C_{12}$ arylene, a group $-CH_2CH(OH)-CH_2-O-X-O-CH_2-CH(OH)-CH_2-$ wherein X is $C_2$-$C_{10}$ alkylene, $C_6$-$C_{15}$ arylene or $C_6$-$C_{12}$ cycloalkylene, or a group $-CH_2CH(OZ')CH_2-(OCH_2-CH-(OZ')CH_2)_2-$ wherein Z' is hydrogen, $C_1$-$C_{18}$ alkyl, allyl, benzyl, $C_2$-$C_{12}$ alkanoyl or benzoyl;
$Q_1$ is $-N(R_8)-$ or -O-;
E is $C_1$-$C_3$ alkylene, the group $-CH_2-CH(R_9)-O-$ wherein $R_9$ is hydrogen, methyl or phenyl, or E is the group $-(CH_2)_3-NH-$ or a direct bond;
$R_{10}$ is hydrogen or $C_1$-$C_{18}$ alkyl;
$R_8$ is hydrogen, $C_1$-$C_{18}$ alkyl, $C_5$-$C_7$ cycloalkyl, $C_7$-$C_{12}$ aralkyl, cyanoethyl, $C_6$-$C_{10}$ aryl, the group $-CH_2-CH(R_9)-OH$ wherein $R_9$ has the meaning defined above; a group of the formula I
or a group of the formula

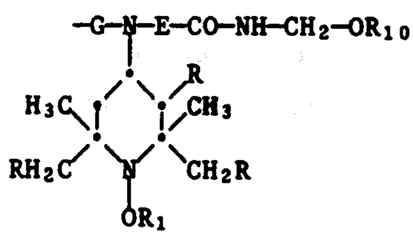

wherein G can be $C_2$-$C_6$ alkylene or $C_6$-$C_{12}$ arylene; or $R_8$ is a group $-E-CO-NH-CH_2-OR_{10}$;
$T_3$ is ethylene or 1,2-propylene, or is the repeating structural unit derived from an alpha-olefin copolymer with an alkyl acrylate or methacrylate;
k is 2 to 100;
$T_4$ has the same meaning as $R_4$ when p is 1 or 2,
$T_5$ is methyl,
$T_6$ is methyl or ethyl,
M and Y are independently methylene or carbonyl;
$T_7$ is the same as $R_7$;
$T_{10}$ and $T_{11}$ are independently alkylene of 2 to 12 carbon atoms, or $T_{11}$ is a group of formula II;
e is 2, 3 or 4 and
$T_{12}$ is a group $-N(R_5)-(CH_2)_d-N(R_5)-$ or

$$-NH(CH_2)_a-N(CH_2)_b-N[(CH_2)_c-N]_fH$$

where a, b and c are independently 2 or 3, d is 2 to 10 and f is 0 or 1;
$T_{13}$ is the same as $R_4$ with the proviso that $R_{13}$ cannot be hydrogen when n is 1;
$E_1$ and $E_2$, being different, each are -CO or $-N(E_5)-$, where $E_5$ is hydrogen, $C_1$-$C_{12}$ alkyl or $C_4$-$C_{22}$ alkoxycarbonylalkyl;
$E_3$ is hydrogen, alkyl of 1 to 30 carbon atoms, phenyl, naphthyl, said phenyl or said naphthyl substituted by chlorine or by alkyl of 1 to 4 carbon atoms, or phenylalkyl of 7 to 12 carbon atoms, or said phenylalkyl substituted by alkyl of 1 to 4 carbon atoms,
$E_4$ is hydrogen, alkyl of 1 to 30 carbon atoms, phenyl, naphthyl or phenylalkyl of 7 to 12 carbon atoms, or
$E_3$ and $E_4$ together are polymethylene of 4 to 17 carbon atoms, or said polymethylene substituted by up to four alkyl groups of 1 to 4 carbon atoms;
$R_2$ of formula (N) is as previously defined when m is 1;
$G_1$ is a direct bond, $C_1$-$C_{12}$ alkylene, phenylene or -NH-G'-NH wherein G' is $C_1$-$C_{12}$ alkylene.

**3.** A composition according to claim 2 which contains a compound of formulae A, B, D, J, K or M wherein R is hydrogen and $T_5$ and $T_6$ are methyl.

**4.** A composition according to claim 2 which contains a compound of formula A wherein R is hydrogen,
$R_1$ is a group D-CO- and D is $C_1$-$C_{12}$ alkyl, $C_1$-$C_4$ alkoxy, phenyl, amino or amino monosubstituted by $C_1$-$C_{12}$ alkyl or phenyl,
m is 1, 2 or 4 and when m is 1,
$R_2$ is an acyl radical of an aliphatic $C_2$-$C_{18}$ carboxylic acid, of a cycloaliphatic $C_6$-$C_{12}$ carboxylic acid or of an aromatic $C_7$-$C_{15}$ carboxylic acid and when m is 2, $R_2$ is a divalent acyl radical of an aliphatic $C_2$-$C_{18}$ dicarboxylic acid or of a cycloaliphatic or aromatic $C_8$-$C_{14}$ dicarboxylic acid, or of an aliphatic, cycloaliphatic or aromatic $C_8$-$C_{14}$ dicarbamic acid, or $R_2$ is a group

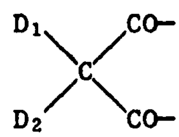

wherein $D_1$ is $C_1$-$C_8$ alkyl or 3,5-di-tert.butyl-4-hydroxybenzyl and $D_2$ is $D_1$ or hydrogen and when m is 4, $R_2$ is a tetravelent acyl radical of a butane- or pentane-tetracarboxylic acid.

**5.** A composition according to claim 2 which contains a compound of formula B wherein R is hydrogen,
$R_1$ is a group D-CO- and D is $C_1$-$C_{12}$ alkyl, $C_1$-$C_4$ alkoxy, phenyl, amino or amino monosubstituted by $C_1$-$C_{12}$ alkyl or phenyl,
p is 1 or 2, $R_3$ is hydrogen, $C_1$-$C_{12}$ alkyl or $C_2$-$C_{12}$ alkanoyl and when p is 1, $R_4$ is $C_1$-$C_{12}$ alkyl, $C_5$-$C_7$ cycloalkyl or a group of formula I,
and when p is 2, $R_4$ is $C_2$-$C_8$ alkylene, phenylene or xylylene, and if $R_3$ is not alkanoyl, $R_4$ may also be a divalent acyl residue of an aliphatic $C_2$-$C_{10}$ dicarboxylic acid or of an aromatic $C_6$ dicarboxylic acid or of an aliphatic or aromatic $C_8$-$C_{15}$ dicarbamic acid.

**6.** A composition of claim 1, which contains di-(1-acetoxy-2,2,6,6-tetramethylpiperidin-4-yl)sebacate.

**7.** The composition of claim 3, which contains alpha,alpha'-(di-1-ethoxy-2,2,6,6-tetramethylpiperidin-4-yloxy)-p-xylene.

**8.** The composition of claim 3, containing 1,4-dibenzyloxy-2,2,6,6-tetramethylpiperidine.

**9.** The composition of claim 3, containing di-(1-benzyloxy-2,2,6,6-tetramethylpiperidin-4-yl) sebacate.

**10.** The composition of claim 3, containing 4-benzyloxy-1-ethoxy-2,2,6,6-tetramethylpiperidine.

**11.** The composition of claim 3, containing 1,4-di-(4-hydroxy-3,5-di-tert-butylbenzoyloxy)-2,2,6,6-tetramethylpiperidine.

**12.** The composition of claim 3, containing alpha,alpha'-(di-1-benzoyloxy-2,2,6,6-tetramethylpiperidin-4-yloxy)-p-xylene.

**13.** A coating composition according to claim 1, which is an ambient temperature curable system based on an alkyd resin, thermoplastic acrylic resin, acrylic alkyd resin, polyurethane resin or polyester resin, or said resins modified with silicones, isocyanates, epoxides, isocyanurates, ketimines or oxazolidines, or the system is based on a cellulose ester or on an epoxide resin.

**14.** A coating composition according to claim 1, which is an acid catalyzed thermosetting system based on a hot crosslinkable acrylic, polyester, polyurethane, polyamide or alkyd resin.

**15.** The composition according to claim 1, which is an enamel for industrial finishes.

**16.** The composition according to claim 1, which is a refinishing enamel for automobiles.

**17.** A compound corresponding to one of the formulae A' to N'

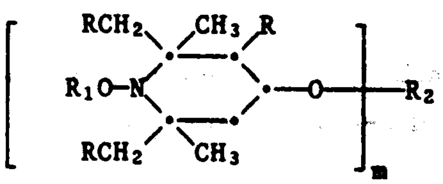

(A')

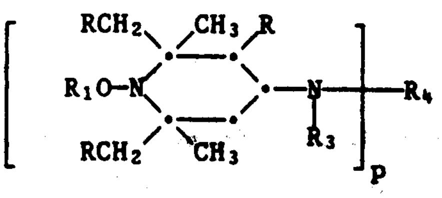

(B')

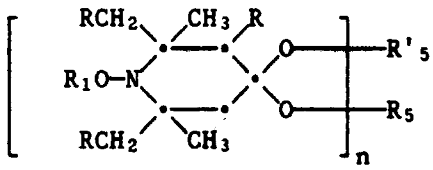

(C')

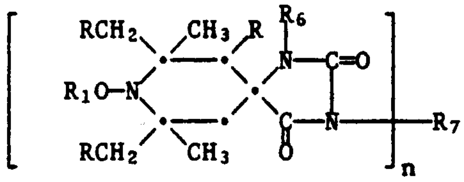

(D')

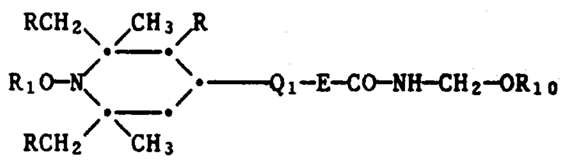

(E')

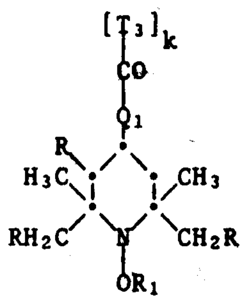

(F')

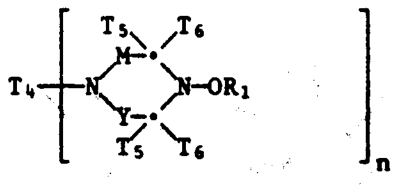

(G')

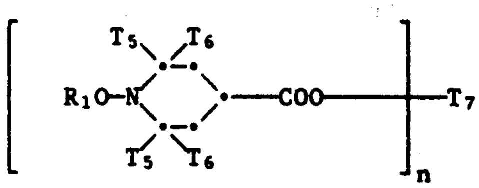

(H')

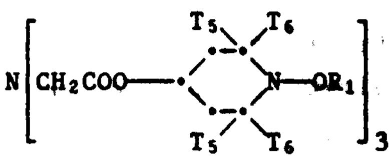

(I')

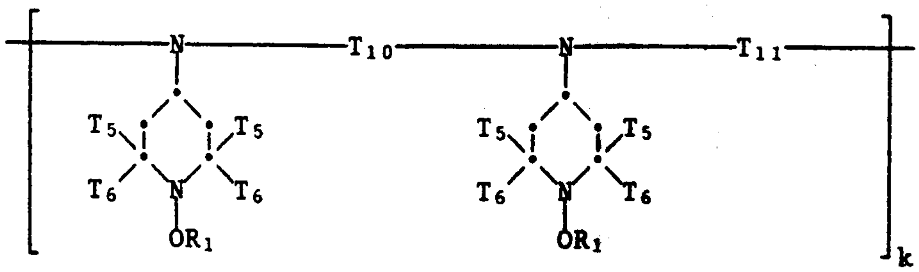

(J')

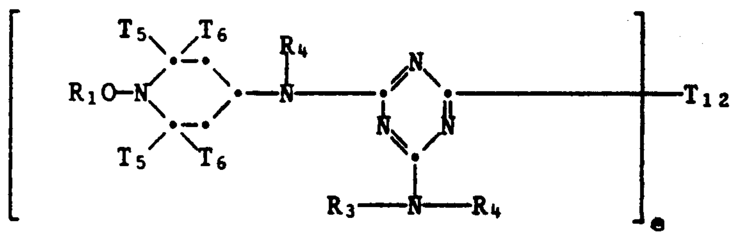

(K')

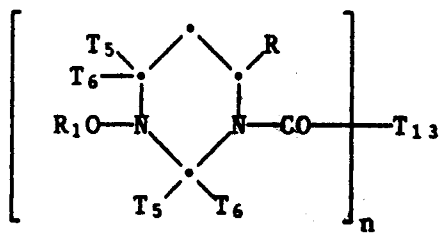

(L')

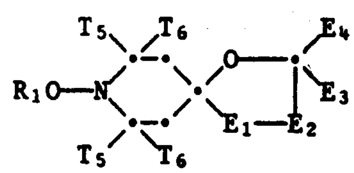

(M')

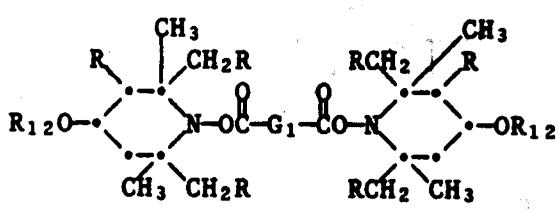

(N')

wherein
R is hydrogen or methyl,
$R_1$ is a group

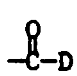

wherein D is $C_1$-$C_{18}$ alkyl, $C_1$-$C_{18}$ alkoxy, phenyl, phenyl substituted by hydroxy, $C_1$-$C_4$ alkyl or $C_1$-$C_4$ alkoxy, or amino or amino mono- or disubstituted by $C_1$-$C_{12}$ alkyl or phenyl;
m is 1-4,
when m is 1
$R_2$ is a group

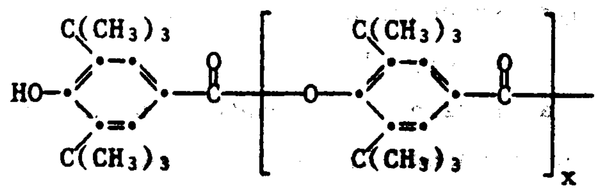

wherein x is 0 or 1, or $R_2$ is a group

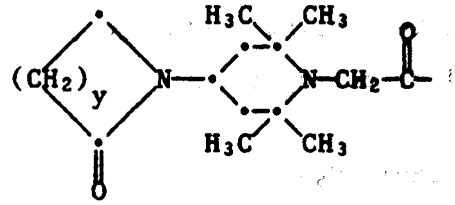

wherein y is 2-4;
when m is 2 and D is alkyl,
$R_2$ is $C_1$-$C_{12}$ alkylene, $C_4$-$C_{12}$ alkenylene, xylylene, a divalent acyl radical of a cycloaliphatic, araliphatic or aromatic dicarboxylic acid having 8-14 C atoms or of an aliphatic, cycloaliphatic or aromatic dicarbamic acid having 8-14 C atoms, or $R_2$ is a group

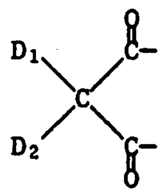

or

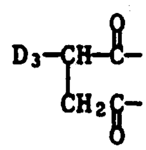

wherein $D_1$ is $C_1$-$C_8$ alkyl, benzyl or 3,5-di-t-butyl-4-hydroxybenzyl, $D_2$ is $D_1$ or hydrogen, $D_3$ is $C_1$-$C_{18}$ alkyl or $C_2$-$C_{18}$ alkenyl;
when m is 2 and D is phenyl or substituted phenyl, amino, substituted amino or alkoxy,
$R_2$ is $C_1$-$C_{12}$ alkylene, $C_4$-$C_{12}$ alkenylene, xylylene, a divalent acyl radical of an aliphatic, cycloaliphatic, araliphatic or aromatic dicarboxylic or dicarbamic acid having up to 14 C atoms; or $R_2$ is a group

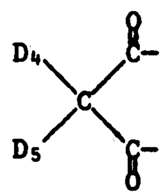

or

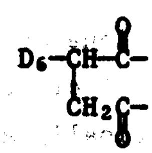

wherein $D_4$ and $D_5$ are independently hydrogen, $C_1$-$C_8$ alkyl, benzyl or 3,5-di-t-butyl-4-hydroxybenzyl and
$D_6$ is $C_1$-$C_{18}$ alkyl or $C_2$-$C_{18}$ alkenyl;
when m is 3, $R_2$ is a trivalent acyl radical of an aliphatic, cycloaliphatic, or aromatic tricarboxylic acid having up to 12 C atoms;
when m is 4, $R_2$ is a tetravalent acyl radical of a saturated or unsaturated aliphatic or aromatic tetracarboxylic acid having up to 18 C atoms;
p is 1, 2 or 3,
$R_3$ is hydrogen, $C_1$-$C_{12}$ alkyl, $C_5$-$C_7$ cycloalkyl, $C_7$-$C_9$ aralkyl, $C_2$-$C_{18}$ alkanoyl, $C_3$-$C_5$ alkenoyl or benzoyl;
when p is 1,

$R_4$ is hydrogen, $C_1$-$C_{18}$ alkyl, $C_5$-$C_7$ cycloalkyl, $C_2$-$C_8$ alkenyl unsubstituted or substituted by a cyano, carbonyl or carbamide group, or it is aryl, aralkyl, glycidyl, a group of the formula $-CH_2-CH(OH)-Z$ or $-CONH-Z$ wherein Z is hydrogen, methyl or phenyl;
or $R_4$ is a group of the formula

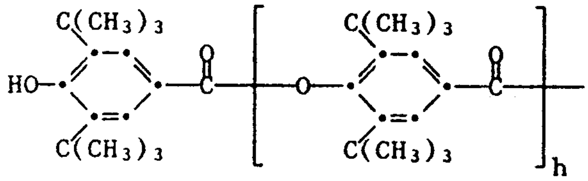 with h as 0 or 1;

or a group of formula I

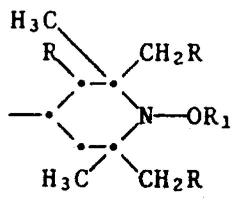

(I);

or $R_3$ and $R_4$ together are alkylene of 4 to 6 carbon atoms or the divalent acyl radical of an aliphatic or aromatic 1,2- or 1,3-dicarboxylic acid;
when p is 2,
$R_4$ is $C_1$-$C_{12}$ alkylene, $C_6$-$C_{12}$ arylene, xylylene, a $-CH_2CH(OH)-CH_2-$ group, or a group $-CH_2-CH(OH)-CH_2-O-X-O-CH_2-CH(OH)-CH_2-$ wherein X is $C_2$-$C_{10}$ alkylene, $C_6$-$C_{15}$ arylene or $C_6$-$C_{12}$ cycloalkylene; or, provided that $R_3$ is not alkanoyl, alkenoyl or benzoyl, $R_4$ can also be a divalent acyl radical of an aliphatic, cycloaliphatic or aromatic dicarboxylic or dicarbamic acid having up to 14 C atoms, or can be the group -CO- or a group of formula II

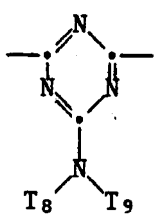

(II)

where $T_8$ and $T_9$ are independently hydrogen, alkyl of 1 to 18 carbon atoms, or $T_8$ and $T_9$ together are alkylene of 4 to 6 carbon atoms or 3-oxapentamethylene;
when p is 3, $R_4$ is 2,4,6-triazinetriyl,
n is 1 or 2, and when n is 1,
$R_5$ and $R'_5$ are independently $C_1$-$C_{12}$ alkyl, $C_2$-$C_{12}$ alkenyl, $C_7$-$C_{12}$ aralkyl, or $R_5$ is also hydrogen, or $R_5$ and $R'_5$ together are $C_2$-$C_8$ alkylene or hydroxyalkylene or $C_4$-$C_{22}$ acyloxyalkylene;
and when n is 2,
$R_5$ and $R'_5$ together are $(-CH_2)_2C(CH_2-)_2$;
$R_6$ is hydrogen, $C_1$-$C_{12}$ alkyl, allyl, benzyl, glycidyl or $C_2$-$C_6$ alkoxyalkyl;
when n is 1,
$R_7$ is hydrogen, $C_1$-$C_{12}$ alkyl, $C_3$-$C_5$ alkenyl, $C_7$-$C_9$ aralkyl, $C_5$-$C_7$ cycloalkyl, $C_2$-$C_4$ hydroxyalkyl, $C_2$-$C_6$ alkoxyalkyl, $C_6$-$C_{10}$ aryl, glycidyl, a group of the formula $-(CH_2)_t-COO-Q$ or of the formula $-(CH_2)_t-O-CO-Q$ wherein t is 1 or 2, and Q is $C_1$-$C_4$ alkyl or phenyl; and
when n is 2,
$R_7$ is $C_2$-$C_{12}$ alkylene, $C_6$-$C_{12}$ arylene, a group $-CH_2CH(OH)-CH_2-O-X-O-CH_2-CH(OH)-CH_2-$ wherein X is $C_2$-$C_{10}$ alkylene, $C_6$-$C_{15}$ arylene or $C_6$-$C_{12}$ cycloalkylene, or a group $-CH_2CH(OZ')CH_2-(OCH_2-CH-(OZ')CH_2)_2-$ wherein Z' is hydrogen, $C_1$-$C_{18}$ alkyl, allyl, benzyl, $C_2$-$C_{12}$ alkanoyl or benzoyl;

$Q_1$ is $-N(R_8)-$ or $-O-$; E is $C_1$-$C_3$ alkylene, the group $-CH_2-CH(R_9)-O-$wherein $R_9$ is hydrogen, methyl or phenyl, or E is the group $-(CH_2)_3-NH-$ or a direct bond;

$R_{10}$ is hydrogen or $C_1$-$C_{18}$ alkyl, $R_8$ is hydrogen, $C_1$-$C_{18}$ alkyl, $C_5$-$C_7$ cycloalkyl, $C_7$-$C_{12}$ aralkyl, cyanoethyl, $C_6$-$C_{10}$ aryl, the group $-CH_2-CH(R_9)-OH$ wherein $R_9$ has the meaning defined above; a group of the formula I or a group of the formula

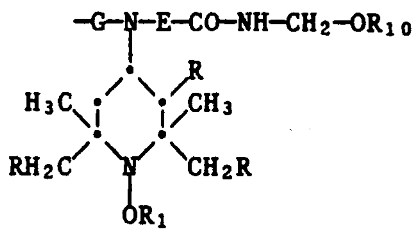

wherein G can be $C_2$-$C_6$ alkylene or $C_6$-$C_{12}$ arylene; or $R_8$ is a group $-E-CO-NH-CH_2-OR_{10}$;

$T_3$ is ethylene or 1,2-propylene, or is the repeating structural unit derived from an alpha-olefin copolymer with an alkyl acrylate or methacrylate;

k is 2 to 100;

$T_4$ has the same meaning as $R_4$ when p is 1 or 2,

$T_5$ is methyl,

$T_6$ is methyl or ethyl, or $T_5$ and $T_6$ together are tetramethylene or pentamethylene,

M and Y are independently methylene or carbonyl;

$T_7$ is the same as $R_7$,

$T_{10}$ and $T_{11}$ are independently alkylene of 2 to 12 carbon atoms, or $T_{11}$ is a group of formula II;

e is 2, 3 or 4,

$T_{12}$ is a group

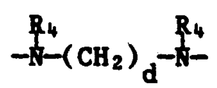

or

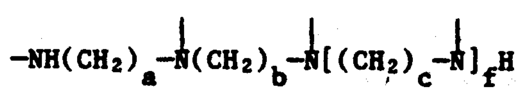

where a, b and c are independently 2 or 3, d is 2-10 and f is 0 or 1;

$T_{13}$ is the same as $R_4$ with the proviso that $T_{13}$ cannot be hydrogen when n is 1;

$E_1$ and $E_2$, being different, each are -CO- or $-N(E_5)-$ where $E_5$ is hydrogen, $C_1$-$C_{12}$ alkyl or alkoxycarbonylalkyl of 4 to 22 carbon atoms;

$E_3$ is hydrogen, alkyl of 1 to 3 carbon atoms, phenyl, naphthyl, said phenyl or said naphthyl substituted by chlorine or by alkyl of 1 to 4 carbon atoms, or phenylalkyl of 7 to 12 carbon atoms, or said phenylalkyl substituted by alkyl of 1 to 4 carbon atoms,

$E_4$ is hydrogen, alkyl of 1 to 30 carbon atoms, phenyl, naphthyl or phenylalkyl of 7 to 12 carbon atoms, or

$E_3$ and $E_4$ together are polymethylene of 4 to 17 carbon atoms, or said polymethylene substituted by up to four alkyl groups of 1 to 4 carbon atoms; and

$R_{12}$ is $C_2$-$C_{18}$ alkanoyl, $C_3$-$C_6$ alkenoyl, benzoyl or benzoyl substituted by $C_1$-$C_4$ alkyl, halogen or hydroxyl, and $G_1$ is a direct bond, $C_1$-$C_{12}$ alkylene, phenylene or -NH-G'-NH wherein G' is $C_1$-$C_{12}$ alkylene.

**18.** A compound of claim 17, wherein D is $C_1$-$C_{18}$ alkyl, phenyl or phenyl substituted by hydroxy, $C_1$-$C_4$ alkyl or $C_1$-$C_4$ alkoxy.

**19.** A compound of claim 17, wherein D is amino or amino mono- or di-substituted by $C_1$-$C_{12}$ alkyl or phenyl.

**20.** A compound of claim 17, wherein D is $C_1$-$C_{18}$ alkoxy.

**21.** A compound of claim 17, wherein R is hydrogen.

**22.** A compound of claim 17, wherein $R_1$ is -CO-D and D is $C_1$-$C_{12}$ alkyl, $C_1$-$C_4$ alkoxy, phenyl, amino, $C_1$-$C_{12}$ alkylamino or phenylamino.

**23.** A compound of claim 17 of formula A', B', D', F', J', K', M' or N', wherein R is hydrogen,
$R_1$ is defined in claim 22,
m is 1, 2 or 4, and
when m is 1, $R_2$ is

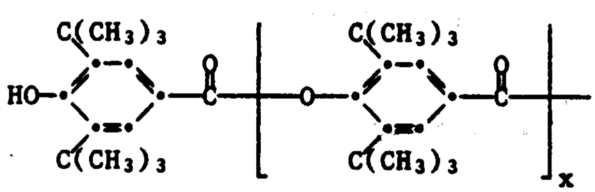

wherein x is 0 or 1,
when m is 2 and D is alkyl,
$R_2$ is a divalent acyl radical of a cycloaliphatic or aromatic dicarboxylic acid having 8-14 C atoms, or of an aliphatic, cycloaliphatic or aromatic dicarbamic acid having 8-14 C atoms; or $R_2$ is a group

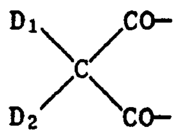

wherein $D_1$ is $C_1$-$C_4$ alkyl or 3,5-di-t-butyl-4-hydroxybenzyl and $D_2$ is $D_1$ or hydrogen, and when m is 2 and D is phenyl, amino, alkylamino, phenylamino or alkoxy, $R_2$ is a divalent acyl radical of an aliphatic, dicarboxylic acid having 2-8 C atoms or of a cycloaliphatic or aromatic dicarboxylic acid having 8-14 C atoms or of an aliphatic, cycloaliphatic or aromatic dicarbamic acid having 8-14 C atoms, or $R_2$ is a group

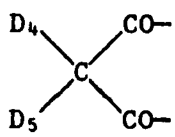

wherein $D_4$ and $D_5$ are independently hydrogen, $C_1$-$C_8$ alkyl, benzyl or 3,5-di-t-butyl-4-hydroxybenzyl, and when m is 4, $R_2$ is a tetravelent acyl radical of 1,2,3,4-butanetetracarboxylic acid, 1,2,3,4-but-2-enetetracarboxylic acid, 1,2,3,5- or 1,2,4,5-pentanetetracarboxylic acid;
p is 1, 2 or 3,
$R_3$ is hydrogen, $C_1$-$C_{12}$ alkyl, cyclohexyl, $C_7$-$C_9$ aralkyl, $C_2$-$C_{18}$ alkanoyl or benzoyl;
when p is 1,
$R_4$ is $C_1$-$C_{18}$ alkyl, cyclohexyl, allyl, benzyl or a group

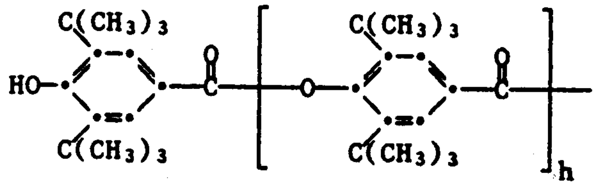

with h as 0 or 1, or is a group

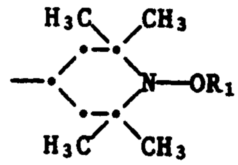

when p is 2,
$R_4$ is $C_1$-$C_{12}$ alkylene, $C_6$-$C_{12}$ arylene, xylylene, a group $-CH_2CH(OH)-CH_2-$, or provided that $R_3$ is not alkanoyl or benzoyl, $R_4$ can also be a divalent acyl radical of an aliphatic, cycloaliphatic or aromatic dicarboxylic acid or dicarbamic acid having 6-12 C atoms,
or $R_4$ is a group of formula II,
where $T_8$ and $T_9$ are independently hydrogen or $C_1$-$C_{12}$ alkyl or $T_8$ and $T_9$ together are $C_4$-$C_6$ alkylene or 3-oxapentamethylene,
when p is 3, $R_4$ is 2,4,6-triazinetriyl;
n is 1 or 2 and
$R_6$ is hydrogen, $C_1$-$C_{12}$ alkyl, allyl or benzyl,
when n is 1, $R_7$ is hydrogen, $C_1$-$C_{12}$ alkyl, allyl, benzyl or cyclohexyl,
and when n is 2, $R_7$ is $C_2$-$C_{12}$ alkylene;
k is 5 to 50, $T_3$ is ethylene or propylene and $Q_1$ is NH or 0;
$T_5$ and $T_6$ each are methyl,
$T_{10}$ and $T_{11}$ are independently $C_2$-$C_{12}$ alkylene or $T_{11}$ is a group of formula II,
e is 4 and
$T_{12}$ is

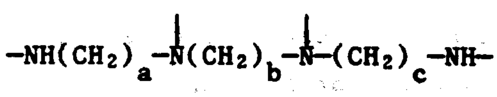

where a, b and c are independently 2 or 3;
$E_1$ is -CO- and $E_2$ is $-N(E_5)-$, $E_3$ and $E_4$ independently are $C_1$-$C_{12}$ alkyl or $E_3$ and $E_4$ together are $C_5$-$C_{12}$ polymethylene, and $E_5$ is hydrogen or $C_1$-$C_{12}$ alkyl;
$G_1$ is a direct bond, $C_2$-$C_8$ alkylene or -NH-G'-NH- wherein G' is $C_2$-$C_8$ alkylene, and $R_{12}$ is $C_2$-$C_{18}$ alkanoyl or benzoyl.

**24.** A compound of claim 17 of formula A', B', F', J' or N', wherein R is hydrogen,
$R_1$ is -CO-D and D is $C_1$-$C_4$ alkyl, $C_1$-$C_4$ alkoxy, phenyl, amino, $C_1$-$C_4$ alkylamino or phenylamino;
m is 1 or 2 and
when m is 1, $R_2$ is a group

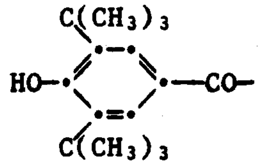

when m is 2 and D is alkyl, $R_2$ is a divalent acyl radical of a phenylenedicarboxylic acid or is a group $-CO-C(D_1)(D_2)-CO-$ wherein $D_1$ is $C_1$-$C_4$ alkyl or 3,5-di-t-butyl-4-hydroxybenzyl and $D_2$ is $D_1$ or hydrogen, and when m is 2 and D is alkoxy, phenyl, amino or alkylamino,
$R_2$ is a divalent acyl radical of an aliphatic or aromatic dicarboxylic acid having 8-10 C atoms or of an aliphatic dicarbamic acid having 8-10 C atoms; or $R_2$ is a group

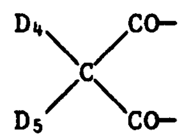

wherein $D_4$ is alkyl or 3,5-di-t-butyl-4-hydroxybenzyl and $D_5$ is $D_4$ or hydrogen;
p is 1 or 2, $R_3$ is $C_1$-$C_4$ alkyl, acetyl or benzoyl,
when p is 1, $R_4$ is $C_1$-$C_{12}$ alkyl or a group

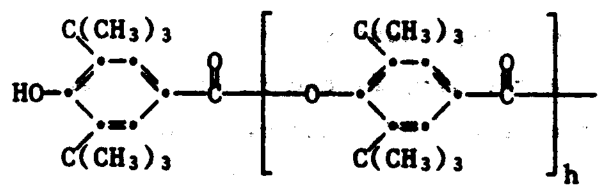

with h as 0 or 1,
and when p is 2, $R_4$ is $C_4$-$C_8$ alkylene;
k is 5 to 50, $T_3$ is ethylene and $Q_1$ is 0;
$T_5$ and $T_6$ are methyl,
$T_{10}$ is hexamethylene and $T_{11}$ is a group of formula II
wherein $T_8$ and $T_9$ are independently hydrogen or $C_1$-$C_{12}$ alkyl or $T_8$ and $T_9$ together are pentamethylene or 3-oxapentamethylene;
$R_{12}$ is acetyl or benzoyl and $G_1$ is a direct bond or $-NH-(CH_2)_6-NH-$.

**25.** Use of a compound of claim 17 as stabilizer for organic materials against actinic degradation.

**26.** Use according to claim 25, as stabilizer for organic polymers.

**27.** Use according to claim 25, as stabilizer for photographic layers.

**28.** Organic material containing an effective stabilizing amount of a compound of claim 17.

**Claims for the following Contracting State : ES**

**1.** A stabilized ambient temperature curable or acid catalyzed thermosetting coating composition containing an effective stabilizing amount of a hindered amine compound containing the group

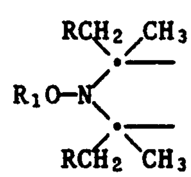

wherein R is hydrogen or methyl and $R_1$ is a group

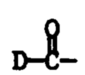 ,

wherein D is $C_1$-$C_{18}$ alkyl, $C_1$-$C_{18}$ alkoxy, phenyl, phenyl substituted by hydroxy, $C_1$-$C_4$ alkyl or $C_1$-$C_4$ alkoxy, or D is amino or amino mono- or disubstituted by $C_1$-$C_{12}$ alkyl or phenyl.

2. The composition according to claim 1, which contains a hindered amine compound corresponding to one of the formulae A-N

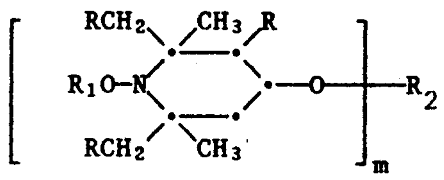

(A)

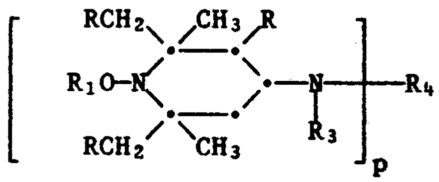

(B)

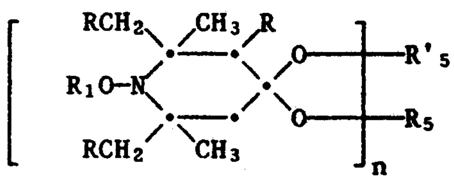

(C)

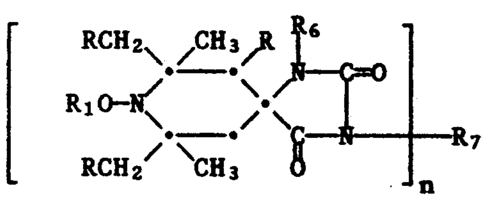

(D)

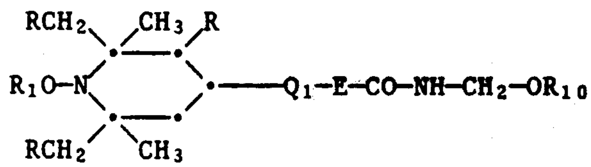

(E)

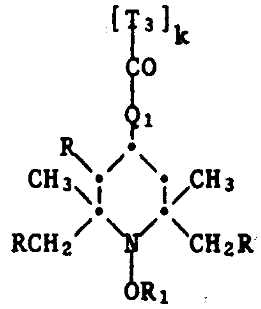

(F)

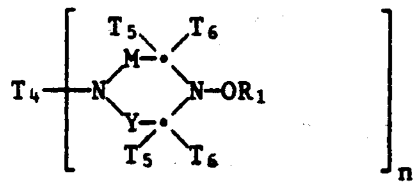

(G)

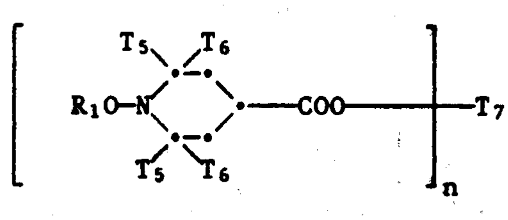

(H)

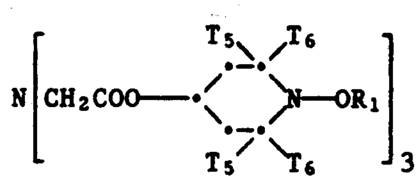

(I)

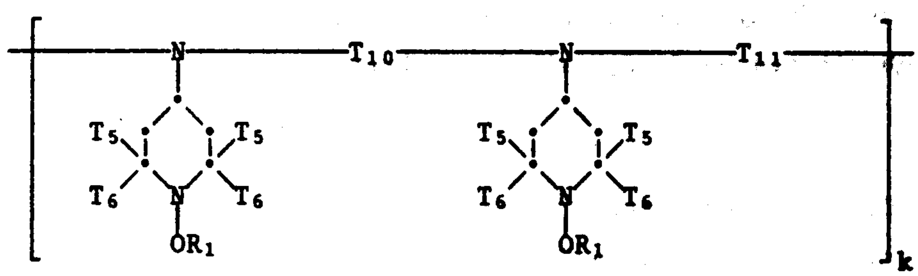

(J)

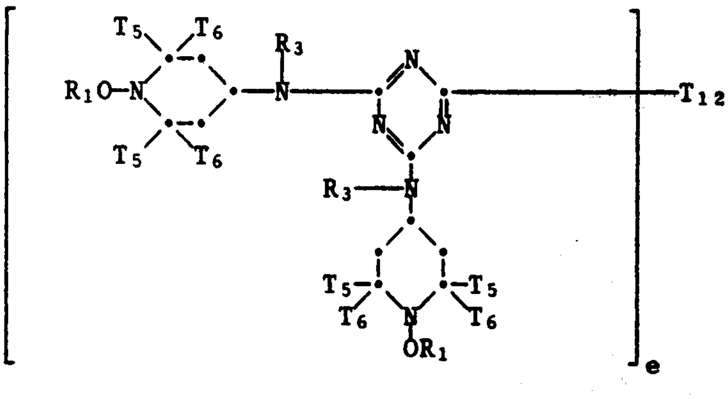

(K)

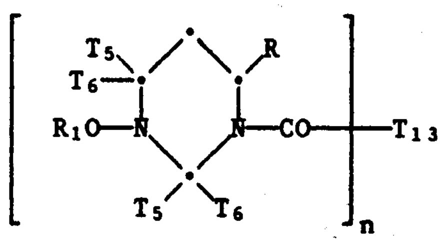

(L)

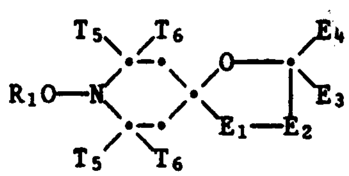

(M)

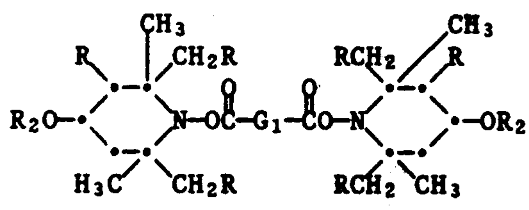

(N)

wherein
R is hydrogen or methyl,
$R_1$ is a group D-CO- , wherein D is $C_1$-$C_{18}$ alkyl, $C_1$-$C_{18}$ alkoxy, phenyl, phenyl substituted by hydroxy, $C_1$-$C_4$ alkoxy, or amino or amino mono- or disubstituted by $C_1$-$C_{12}$ alkyl or phenyl;
m is 1-4,
when m is 1,
$R_2$ is hydrogen, $C_1$-$C_{18}$ alkyl optionally interrupted by one or more oxygen atoms, $C_2$-$C_{12}$ alkenyl, $C_6$-$C_{10}$ aryl, $C_7$-$C_{18}$ aralkyl, glycidyl, a monovalent acyl radical of an aliphatic, cycloaliphatic, araliphatic or aromatic carboxylic acid, or of a carbamic acid, or $R_2$ is a group

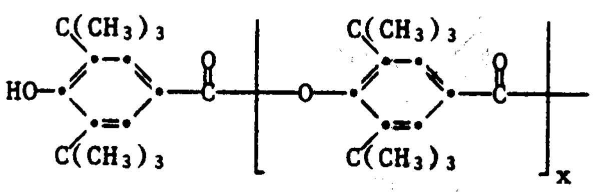

wherein x is 0 or 1, or is a group

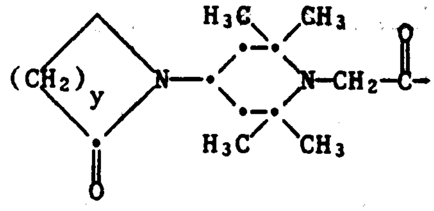

wherein y is 2-4;
when m is 2,
$R_2$ is $C_1$-$C_{12}$ alkylene, $C_4$-$C_{12}$ alkenylene, xylylene, a divalent acyl radical of an aliphatic, cycloaliphatic, araliphatic or aromatic dicarboxylic acid or of a dicarbamic acid, or $R_2$ is a group

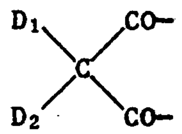 or 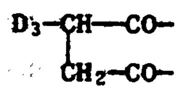

wherein $D_1$ and $D_2$ independently are hydrogen, an alkyl radical containing up to 8 carbon atoms, phenyl, benzyl or 3,5-di-t-butyl-4-hydroxybenzyl, and $D_3$ is an alkyl or alkenyl radical containing up to 18 carbon atoms; when m is 3, $R_2$ is a trivalent acyl radical of an aliphatic, cycloaliphatic, or aromatic tricarboxylic acid; when m is 4, $R_2$ is a tetravelent acyl radical of a saturated or unsaturated aliphatic or aromatic tetracarboxylic acid: p is 1, 2 or 3,
$R_3$ is hydrogen, $C_1$-$C_{12}$ alkyl, $C_5$-$C_7$ cycloalkyl, $C_7$-$C_9$ aralkyl, $C_2$-$C_{18}$ alkanoyl, $C_3$-$C_5$ alkenoyl or benzoyl;
when p is 1,
$R_4$ is hydrogen, $C_1$-$C_{18}$ alkyl, $C_5$-$C_7$ cycloalkyl, $C_2$-$C_8$ alkenyl unsubstituted or substituted by a cyano, carbonyl or carbamide group, or it is aryl, aralkyl, glycidyl, a group of the formula $-CH_2-CH(OH)-Z$ or of the formula -CONH-Z wherein Z is hydrogen, methyl or phenyl;
or $R_4$ is a group of the formula I

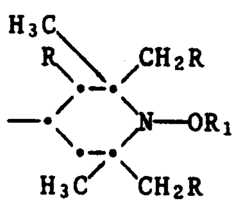 (I) or

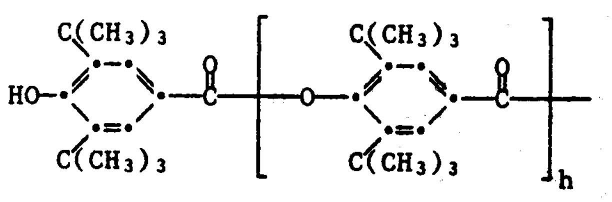 with h as 0 or 1;

or $R_3$ and $R_4$ together are alkylene of 4 to 6 carbon atoms or 1-oxo alkylene or the divalent acyl radical of an aliphatic or aromatic 1,2- 1,3-dicarboxylic acid;
when p is 2,
$R_4$ is $C_1$-$C_{12}$ alkylene, $C_6$-$C_{12}$ arylene, xylylene, a $-CH_2CH(OH)-CH_2$ group, or a group $-CH_2-CH(OH)-CH_2-O-X-O-CH_2-CH(OH)-CH_2-$ wherein X is $C_2$-$C_{10}$ alkylene, $C_6$-$C_{15}$ arylene or $C_6$-$C_{12}$ cycloalkylene; or, provided that $R_3$ is not alkanoyl, alkenoyl or benzoyl, $R_4$ can also be a divalent acyl radical of an aliphatic, cycloaliphatic or aromatic dicarboxylic or dicarbamic acid, or can be the group -CO-; or $R_4$ is a group of formula II

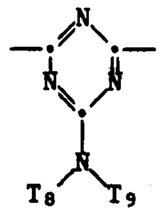

(II)

where $T_8$ and $T_9$ are independently hydrogen, alkyl of 1 to 18 carbon atoms, or $T_8$ and $T_9$ together are alkylene of 4 to 6 carbon atoms or 3-oxapentamethylene, preferably $T_8$ and $T_9$ together are 3-oxapentamethylene;

when p is 3, $R_4$ is 2,4,6-triazinetriyl;

n is 1 or 2 and

when n is 1,

$R_5$ and $R'_5$ are independently $C_1$-$C_{12}$ alkyl, $C_2$-$C_{12}$ alkenyl, $C_7$-$C_{12}$ aralkyl, or $R_5$ is also hydrogen, or $R_5$ and $R'_5$ together are $C_2$-$C_8$ alkylene or hydroxyalkylene or $C_4$-$C_{22}$ acyloxyalkylene;

when n is 2,

$R_5$ and $R'_5$ together are $(-CH_2)_2C(CH_2-)_2$;

$R_6$ is hydrogen, $C_1$-$C_{12}$ alkyl, allyl, benzyl, glycidyl or $C_2$-$C_6$ alkoxyalkyl;

when n is 1,

$R_7$ is hydrogen, $C_1$-$C_{12}$ alkyl, $C_3$-$C_5$ alkenyl, $C_7$-$C_9$ aralkyl, $C_5$-$C_7$ cycloalkyl, $C_2$-$C_4$ hydroxyalkyl, $C_2$-$C_6$ alkoxyalkyl, $C_6$-$C_{10}$ aryl, glycidyl, a group of the formula $-(CH_2)_t$-COO-Q or of the formula $-(CH_2)_t$-O-CO-Q wherein t is 1 or 2, and Q is $C_1$-$C_4$ alkyl or phenyl; or

when n is 2,

$R_7$ is $C_2$-$C_{12}$ alkylene, $C_6$-$C_{12}$ arylene, a group $-CH_2CH(OH)-CH_2-O-X-O-CH_2-CH(OH)-CH_2-$ wherein X is $C_2$-$C_{10}$ alkylene, $C_6$-$C_{15}$ arylene or $C_6$-$C_{12}$ cycloalkylene, or a group $-CH_2CH(OZ')CH_2-(OCH_2-CH-(OZ')CH_2)_2-$ wherein Z' is hydrogen, $C_1$-$C_{18}$ alkyl, allyl, benzyl, $C_2$-$C_{12}$ alkanoyl or benzoyl;

$Q_1$ is $-N(R_8)-$ or -O-;

E is $C_1$-$C_3$ alkylene, the group $-CH_2-CH(R_9)-O-$ wherein $R_9$ is hydrogen, methyl or phenyl, or E is the group $-(CH_2)_3-NH$ or a direct bond;

$R_{10}$ is hydrogen or $C_1$-$C_{18}$ alkyl;

$R_8$ is hydrogen, $C_1$-$C_{18}$ alkyl, $C_5$-$C_7$ cycloalkyl, $C_7$-$C_{12}$ aralkyl, cyanoethyl, $C_6$-$C_{10}$ aryl, the group $-CH_2-CH(R_9)-OH$ wherein $R_9$ has the meaning defined above; a group of the formula I or a group of the formula

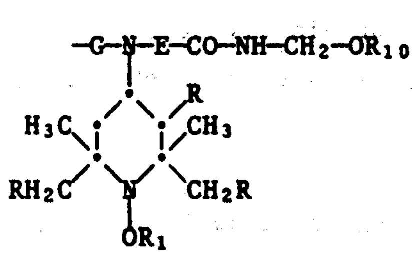

wherein G can be $C_2$-$C_6$ alkylene or $C_6$-$C_{12}$ arylene; or $R_8$ is a group $-E-CO-NH-CH_2-OR_{10}$;

$T_3$ is ethylene or 1,2-propylene, or is the repeating structural unit derived from an alpha-olefin copolymer with an alkyl acrylate or methacrylate;

k is 2 to 100;

$T_4$ has the same meaning as $R_4$ when p is 1 or 2,

$T_5$ is methyl,

$T_6$ is methyl or ethyl,

M and Y are independently methylene or carbonyl;

$T_7$ is the same as $R_7$;

$T_{10}$ and $T_{11}$ are independently alkylene of 2 to 12 carbon atoms, or $T_{11}$ is a group of formula II;

e is 2, 3 or 4 and

$T_{12}$ is a group $-N(R_5)-(CH_2)_d-N(R_5)-$ or

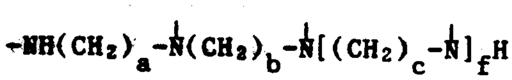

where a, b and c are independently 2 or 3, d is 2 to 10 and f is 0 or 1;
$T_{13}$ is the same as $R_4$ with the proviso that $R_{13}$ cannot be hydrogen when n is 1;
$E_1$ and $E_2$, being different, each are -CO- or -N($E_5$)-, where $E_5$ is hydrogen, $C_1$-$C_{12}$ alkyl or $C_4$-$C_{22}$ alkoxycarbonylalkyl;
$E_3$ is hydrogen, alkyl of 1 to 30 carbon atoms, phenyl, naphthyl, said phenyl or said naphthyl substituted by chlorine or by alkyl of 1 to 4 carbon atoms, or phenylalkyl of 7 to 12 carbon atoms, or said phenylalkyl substituted by alkyl of 1 to 4 carbon atoms,
$E_4$ is hydrogen, alkyl of 1 to 30 carbon atoms, phenyl, naphthyl or phenylalkyl of 7 to 12 carbon atoms, or
$E_3$ and $E_4$ together are polymethylene of 4 to 17 carbon atoms, or said polymethylene substituted by up to four alkyl groups of 1 to 4 carbon atoms;
$R_2$ of formula (N) is as previously defined when m is 1;
$G_1$ is a direct bond, $C_1$-$C_{12}$ alkylene, phenylene or -NH-G'-NH wherein G' is $C_1$-$C_{12}$ alkylene.

3. A composition according to claim 2 which contains a compound of formulae A, B, D, J, K or M wherein R is hydrogen and $T_5$ and $T_6$ are methyl.

4. A composition according to claim 2 which contains a compound of formula A wherein R is hydrogen, $R_1$ is a group D-CO- and D is $C_1$-$C_{12}$ alkyl, $C_1$-$C_4$ alkoxy, phenyl, amino or amino monosubstituted by $C_1$-$C_{12}$ alkyl or phenyl,
m is 1, 2 or 4 and when m is 1,
$R_2$ is an acyl radical of an aliphatic $C_2$-$C_{18}$ carboxylic acid, of a cycloaliphatic $C_6$-$C_{12}$ carboxylic acid or of an aromatic $C_7$-$C_{15}$ carboxylic acid and when m is 2, $R_2$ is a divalent acyl radical of an aliphatic $C_2$-$C_{18}$ dicarboxylic acid or of a cycloaliphatic or aromatic $C_8$-$C_{14}$ dicarboxylic acid, or of an aliphatic, cycloaliphatic or aromatic $C_8$-$C_{14}$ dicarbamic acid, or $R_2$ is a group

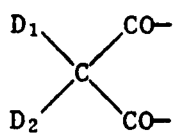

wherein $D_1$ is $C_1$-$C_8$ alkyl or 3,5-di-tert.butyl-4-hydroxybenzyl and $D_2$ is $D_1$ or hydrogen and when m is 4, $R_2$ is a tetravalent acyl radical of a butane- or pentane-tetracarboxylic acid.

5. A composition according to claim 2 which contains a compound of formula B wherein R is hydrogen, $R_1$ is a group D-CO- and D is $C_1$-$C_{12}$ alkyl, $C_1$-$C_4$ alkoxy, phenyl, amino or amino monosubstituted by $C_1$-$C_{12}$ alkyl or phenyl,
p is 1 or 2, $R_3$ is hydrogen, $C_1$-$C_{12}$ alkyl or $C_2$-$C_{12}$ alkanoyl and when p is 1, $R_4$ is $C_1$-$C_{12}$ alkyl, $C_5$-$C_7$ cycloalkyl or a group of formula I,
and when p is 2, $R_4$ is $C_2$-$C_8$ alkylene, phenylene or xylylene, and if $R_3$ is not alkanoyl, $R_4$ may also be a divalent acyl residue of an aliphatic $C_2$-$C_{10}$ dicarboxylic acid or of an aromatic $C_6$ dicarboxylic acid or of an aliphatic or aromatic $C_8$-$C_{15}$ dicarbamic acid.

6. A composition of claim 1, which contains di-(1-acetoxy-2,2,6,6-tetramethylpiperidin-4-yl)sebacate.

7. The composition of claim 3, which contains alpha,alpha'-(di-1-ethoxy-2,2,6,6-tetramethylpiperidin-4-yloxy)-p-xylene.

8. The composition of claim 3, containing 1,4-dibenzyloxy-2,2,6,6-tetramethylpiperidine.

9. The composition of claim 3, containing di-(1-benzyloxy-2,2,6,6-tetramethylpiperidin-4-yl) sebacate.

10. The composition of claim 3, containing 4-benzyloxy-1-ethoxy-2,2,6,6-tetramethylpiperidine.

**11.** The composition of claim 3, containing 1,4-di-(4-hydroxy-3,5-di-tert-butylbenzoyloxy)-2,2,6,6-tetramethylpiperidine.

**12.** The composition of claim 3, containing alpha,alpha'-(di-1-benzoyloxy-2,2,6,6-tetramethylpiperidin-4-yloxy)-p-xylene.

**13.** A coating composition according to claim 1, which is an ambient temperature curable system based on an alkyd resin, thermoplastic acrylic resin, acrylic alkyd resin, polyurethane resin or polyester resin, or said resins modified with silicones, isocyanates, epoxides, isocyanurates, ketimines or oxazolidines, or the system is based on a cellulose ester or on an epoxide resin.

**14.** A coating composition according to claim 1, which is an acid catalyzed thermosetting system based on a hot crosslinkable acrylic, polyester, polyurethane, polyamide or alkyd resin.

**15.** The composition according to claim 1, which is an enamel for industrial finishes.

**16.** The composition according to claim 1, which is a refinishing enamel for automobiles.

**Patentansprüche**
**Patentansprüche für folgende Vertragsstaaten : BE, DE, FR, GB, IT, NL, SE**

**1.** Stabilisierte, bei Umgebungstemperatur härtende oder säurekatalysiert wärmehärtende Beschichtungsmasse, enthaltend eine wirksam stabilisierende Menge eines gehinderten Amins mit der Gruppe

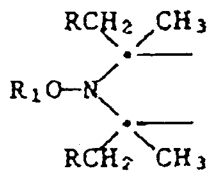

worin R Wasserstoff oder Methyl bedeutet und $R_1$ eine Gruppe D-(C=O)- darstellt, worin D $C_1$-$C_{18}$-Alkyl, $C_1$-$C_{18}$-Alkoxy, Phenyl, Phenyl, substituiert mit Hydroxy, $C_1$-$C_4$-Alkyl oder $C_1$-$C_4$-Alkoxy, bedeutet oder D Amino oder Amino, mono- oder disubstituiert mit $C_1$-$C_{12}$-Alkyl oder Phenyl darstellt.

**2.** Masse nach Anspruch 1, enthaltend eine gehinderte Aminverbindung gemäß einer der Formeln A-N

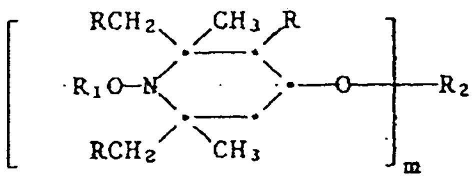

(A)

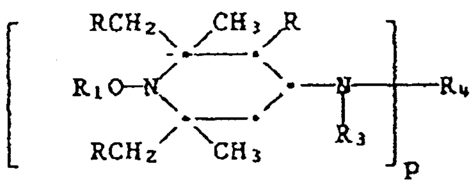

(B)

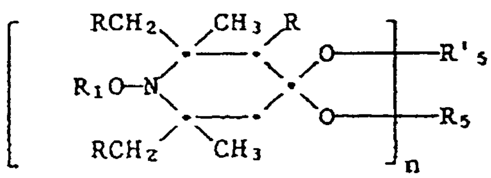

(C)

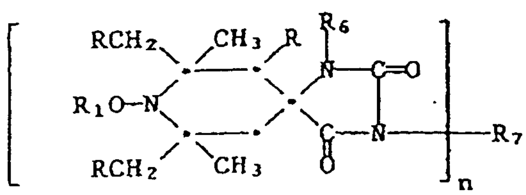

(D)

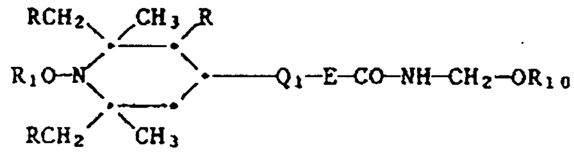

(E)

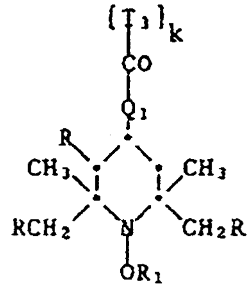

(F)

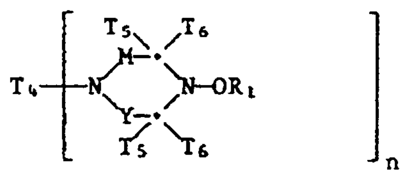

(G)

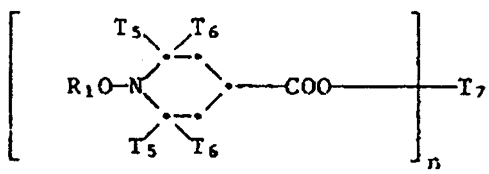

(H)

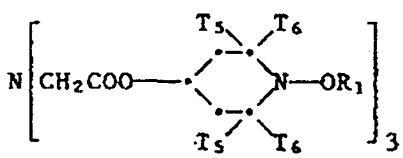

(I)

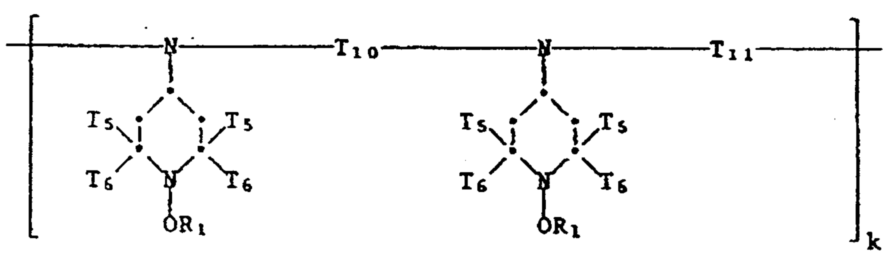

(J)

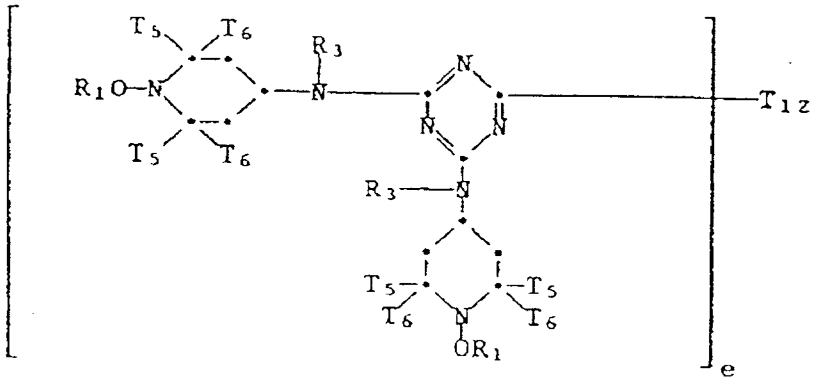

(K)

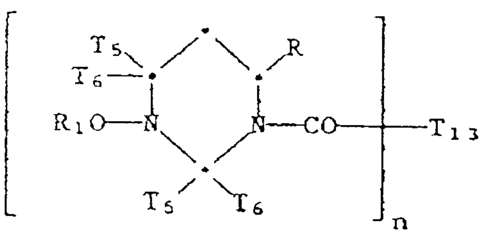

(L)

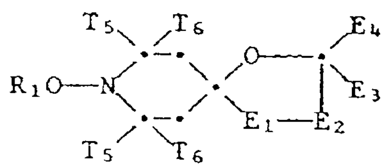

(M)

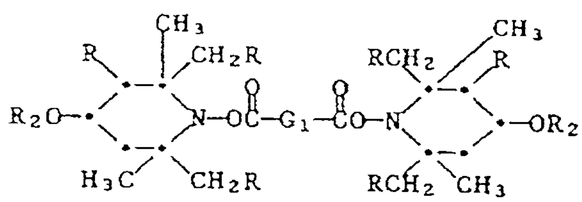

(N)

worin R Wasserstoff oder Methyl bedeutet,
$R_1$ eine Gruppe D-CO- darstellt, worin D $C_1$-$C_{18}$-Alkyl, $C_1$-$C_{18}$-Alkoxy, Phenyl, Phenyl substituiert mit Hydroxy, $C_1$-$C_4$-Alkyl oder $C_1$-$C_4$-Alkoxy, oder Amino oder Amino, mono- oder disubstituiert mit $C_1$-$C_{12}$-Alkyl, oder Phenyl bedeutet;
m 1-4 darstellt,
wenn m 1 bedeutet,
$R_2$ Wasserstoff, $C_1$-$C_{18}$-Alkyl, gegebenenfalls unterbrochen mit einem oder mehreren Sauerstoffatomen, $C_2$-$C_{12}$-Alkenyl, $C_6$-$C_{10}$-Aryl, $C_7$-$C_{18}$-Aralkyl, Glycidyl, einen einwertigen Acylrest einer aliphatischen, cycloaliphatischen, araliphatischen oder aromatischen Carbonsäure, oder einer Carbaminsäure darstellt, oder $R_2$ eine Gruppe

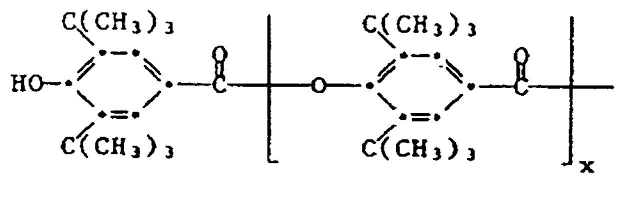

bedeutet, worin x 0 oder 1 darstellt, oder eine Gruppe

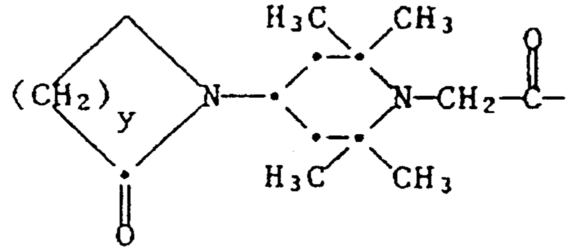

bedeutet,

worin y 2-4 darstellt; wenn m 2 bedeutet,

$R_2$ $C_1$-$C_{12}$-Alkylen, $C_4$-$C_{12}$-Alkenylen, Xylylen, einen zweiwertigen Acylrest einer aliphatischen, cycloaliphatischen, araliphatischen oder aromatischen Dicarbonsäure, oder einer Dicarbaminsäure darstellt, oder $R_2$ eine Gruppe

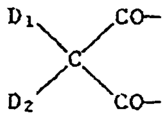

oder

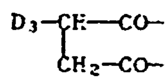

bedeutet, worin $D_1$ und $D_2$ unabhängig voneinander Wasserstoff, einen Alkylrest mit bis zu 8 Kohlenstoffatomen, Phenyl, Benzyl oder 3,5-Di-t-butyl-4-hydroxybenzyl bedeuten, und $D_3$ einen Alkyl- oder Alkenylrest mit bis zu 18 Kohlenstoffatomen darstellt; wenn m 3 bedeutet, $R_2$ einen dreiwertigen Acylrest einer aliphatischen, cycloaliphatischen oder aromatischen Tricarbonsäure darstellt; wenn m 4 bedeutet, $R_2$ einen vierwertigen Acylrest einer gesättigten oder ungesättigten, aliphatischen oder aromatischen Tetracarbonsäure darstellt; p 1, 2 oder 3 bedeutet,

$R_3$ Wasserstoff, $C_1$-$C_{12}$-Alkyl, $C_5$-$C_7$-Cycloalkyl, $C_7$-$C_9$-Aralkyl, $C_2$-$C_{18}$-Alkanoyl, $C_3$-$C_5$-Alkenoyl oder Benzoyl darstellt; wenn p 1 bedeutet,

$R_4$ Wasserstoff, $C_1$-$C_{18}$-Alkyl, $C_5$-$C_7$-Cycloalkyl, $C_2$-$C_8$-Alkenyl, unsubstituiert oder substituiert mit einer Cyano-, Carbonyl- oder Carbamidgruppe darstellt, oder Aryl, Aralkyl, Glycidyl, eine Gruppe der Formel $-CH_2-CH(OH)-Z$ oder der Formel -CONH-Z bedeutet, worin Z Wasserstoff, Methyl oder Phenyl darstellt; oder $R_4$ eine Gruppe der Formel I

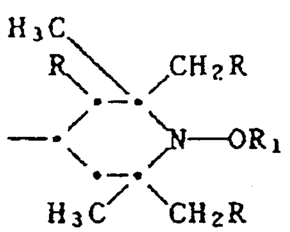

(I) oder

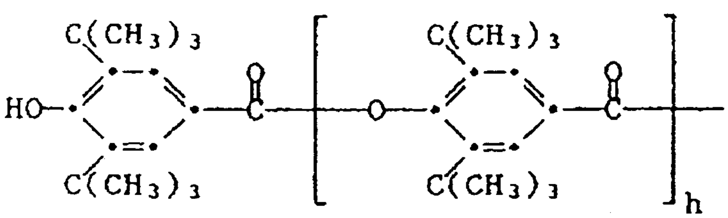

bedeutet, mit h als 0 oder 1;

oder $R_3$ und $R_4$ zusammen Alkylen mit 4 bis 6 Kohlenstoffatomen oder 1-Oxoalkylen oder den zweiwertigen Acylrest einer aliphatischen oder aromatischen 1,2- oder 1,3-Dicarbonsäure bedeuten;

wenn p 2 darstellt,

$R_4$ $C_1$-$C_{12}$-Alkylen, $C_6$-$C_{12}$-Arylen, Xylylen, eine -$CH_2CH(OH)$-$CH_2$-Gruppe, oder eine Gruppe -$CH_2$-CH-(OH)-$CH_2$-O-X-O-$CH_2$-CH(OH)-$CH_2$- bedeutet, worin X $C_2$-$C_{10}$-Alkylen, $C_6$-$C_{15}$-Arylen oder $C_6$-$C_{12}$-Cycloalkylen darstellt; oder, mit der Maßgabe, daß $R_3$ nicht Alkanoyl, Alkenoyl oder Benzoyl bedeutet, $R_4$ auch einen zweiwertigen Acylrest einer aliphatischen, cycloaliphatischen oder aromatischen Dicarbon- oder Dicarbaminsäure bedeuten kann, oder die Gruppe -CO- bedeuten kann; oder $R_4$ eine Gruppe der Formel II

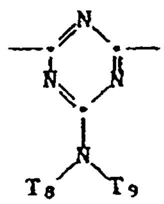

(II)

darstellt, worin $T_8$ und $T_9$ unabhängig voneinander Wasserstoff oder Alkyl mit 1 bis 18 Kohlenstoffatomen bedeuten, oder $T_8$ und $T_9$ zusammen Alkylen mit 4 bis 6 Kohlenstoffatomen oder 3-Oxapentamethylen bedeuten, vorzugsweise $T_8$ und $T_9$ zusammen 3-Oxapentamethylen bedeuten;
wenn p 3 bedeutet, $R_4$ 2,4,6-Triazintriyl darstellt;
n 1 oder 2 bedeutet und
wenn n 1 bedeutet,
$R_5$ und $R'_5$ unabhängig voneinander $C_1$-$C_{12}$-Alkyl, $C_2$-$C_{12}$-Alkenyl, $C_7$-$C_{12}$-Aralkyl bedeuten, oder $R_5$ auch Wasserstoff darstellt, oder $R_5$ und $R'_5$ zusammen $C_2$-$C_8$-Alkylen oder Hydroxyalkylen oder $C_4$-$C_{22}$-Acyloxyalkylen bedeuten;
wenn n 2 bedeutet,
$R_5$ und $R'_5$ zusammen $(-CH_2)_2C(CH_2-)_2$ bedeuten;
$R_6$ Wasserstoff, $C_1$-$C_{12}$-Alkyl, Allyl, Benzyl, Glycidyl oder $C_2$-$C_6$-Alkoxyalkyl darstellt;
wenn n 1 bedeutet,
$R_7$ Wasserstoff, $C_1$-$C_{12}$-Alkyl, $C_3$-$C_5$-Alkenyl, $C_7$-$C_9$-Aralkyl, $C_5$-$C_7$-Cycloalkyl, $C_2$-$C_4$-Hydroxyalkyl, $C_2$-$C_6$-Alkoxyalkyl, $C_6$-$C_{10}$-Aryl, Glycidyl, eine Gruppe der Formel $-(CH_2)_t$-COO-Q oder der Formel $-(CH_2)_t$-O-CO-Q darstellt worin t 1 oder 2 bedeutet, und Q $C_1$-$C_4$-Alkyl oder Phenyl darstellt; oder wenn n 2 bedeutet,
$R_7$ $C_2$-$C_{12}$-Alkylen, $C_6$-$C_{12}$-Arylen, eine Gruppe -$CH_2CH(OH)$-$CH_2$-O-X-O-$CH_2$-CH(OH)-$CH_2$- darstellt, worin X $C_2$-$C_{10}$-Alkylen, $C_6$-$C_{15}$-Arylen oder $C_6$-$C_{12}$-Cycloalkylen, oder eine Gruppe -$CH_2CH(OZ')CH_2$-$(OCH_2$-$CH(OZ')CH_2)_2$- bedeutet, worin Z' Wasserstoff, $C_1$-$C_{18}$-Alkyl, Allyl, Benzyl, $C_2$-$C_{12}$-Alkanoyl oder Benzoyl darstellt; $Q_1$ -N($R_8$)- oder -O- bedeutet;
E $C_1$-$C_3$-Alkylen oder die Gruppe -$CH_2$-CH($R_9$)-O- darstellt, worin $R_9$ Wasserstoff, Methyl oder Phenyl bedeutet, oder E die Gruppe $-(CH_2)_3$-NH- oder eine direkte Bindung darstellt;
$R_{10}$ Wasserstoff oder $C_1$-$C_{18}$-Alkyl bedeutet;
$R_8$ Wasserstoff, $C_1$-$C_{18}$-Alkyl, $C_5$-$C_7$-Cycloalkyl, $C_7$-$C_{12}$-Aralkyl, Cyanoethyl, $C_6$-$C_{10}$-Aryl, die Gruppe -$CH_2$-CH($R_9$)-OH, worin $R_9$ wie vorstehend definiert ist; eine Gruppe der Formel I oder eine Gruppe der Formel

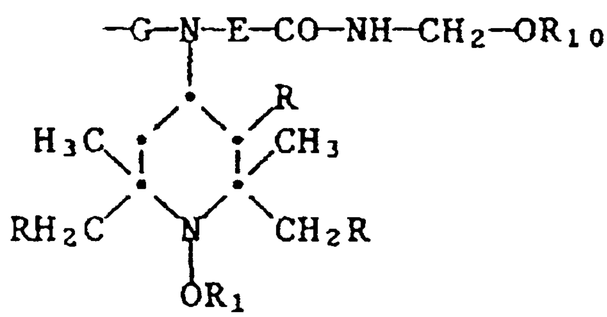

darstellt, worin G $C_2$-$C_6$-Alkylen oder $C_6$-$C_{12}$-Arylen bedeuten kann; oder $R_8$ eine Gruppe -E-CO-NH-$CH_2$-$OR_{10}$ bedeutet;
$T_3$ Ethylen oder 1,2-Propylen darstellt, oder die sich wiederholende Struktureinheit, abgeleitet von einem $\alpha$-Olefincopolymer mit einem Alkylacrylat oder -methacrylat bedeutet;

k 2 bis 100 darstellt;
$T_4$ die gleiche Bedeutung wie $R_4$ hat, wenn p 1 oder 2 bedeutet,
$T_5$ Methyl darstellt,
$T_6$ Methyl oder Ethyl bedeutet,
M und Y unabhängig voneinander Methylen oder Carbonyl bedeuten;
$T_7$ das gleiche wie $R_7$ darstellt;
$T_{10}$ und $T_{11}$ unabhängig voneinander Alkylen mit 2 bis 12 Kohlenstoffatomen bedeuten, oder $T_{11}$ eine Gruppe der Formel II bedeutet;
e 2, 3 oder 4 darstellt und
$T_{12}$ eine Gruppe $-N(R_5)-(CH_2)_d-N(R_5)-$ oder

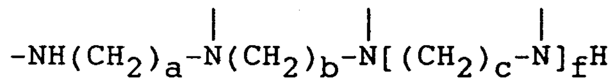

bedeutet, worin a, b und c unabhängig voneinander 2 oder 3 bedeuten, d 2 bis 10 darstellt und f 0 oder 1 bedeutet; $T_{13}$ das gleiche wie $R_4$ darstellt, mit der Maßgabe, daß $R_{13}$ nicht Wasserstoff sein kann, wenn n 1 ist;
$E_1$ und $E_2$ jeweils verschieden -CO- oder $-N(E_5)-$ bedeuten, wobei $E_5$ Wasserstoff, $C_1$-$C_{12}$-Alkyl oder $C_4$-$C_{22}$-Alkoxycarbonylalkyl bedeutet;
$E_3$ Wasserstoff, Alkyl mit 1 bis 30 Kohlenstoffatomen, Phenyl, Naphthyl, die Phenyl- oder die Naphthylgruppe, substituiert mit Chlor oder mit Alkyl mit 1 bis 4 Kohlenstoffatomen, oder Phenylalkyl mit 7 bis 12 Kohlenstoffatomen, oder die Phenylalkylgruppe, substitutiert mit Alkyl mit 1 bis 4 Kohlenstoffatomen, darstellt,
$E_4$ Wasserstoff, Alkyl mit 1 bis 30 Kohlenstoffatomen, Phenyl, Naphthyl oder Phenylalkyl mit 7 bis 12 Kohlenstoffatomen bedeutet, oder
$E_3$ und $E_4$ zusammen Polymethylen mit 4 bis 17 Kohlenstoffatomen, oder die Polymethylengruppe, substituiert mit bis zu vier Alkylgruppen mit 1 bis 4 Kohlenstoffatomen, bedeuten;
$R_2$ von Formel (N) wie vorstehend definiert ist, wenn m 1 darstellt;
$G_1$ eine direkte Bindung, $C_1$-$C_{12}$-Alkylen, Phenylen oder -NH-G'-NH bedeutet, worin G' $C_1$-$C_{12}$-Alkylen ist.

**3.** Masse nach Anspruch 2, eine Verbindung der Formeln A, B, D, J, K oder M enthaltend, worin R Wasserstoff darstellt und $T_5$ und $T_6$ Methyl bedeuten.

**4.** Masse nach Anspruch 2, eine Verbindung der Formel A enthaltend, worin R Wasserstoff bedeutet,
$R_1$ eine Gruppe D-CO- darstellt und D $C_1$-$C_{12}$-Alkyl, $C_1$-$C_4$-Alkoxy, Phenyl, Amino oder Amino, monosubstituiert mit $C_1$-$C_{12}$-Alkyl oder Phenyl bedeutet,
m 1, 2 oder 4 darstellt und wenn m 1 bedeutet,
$R_2$ einen Acylrest einer aliphatischen $C_2$-$C_{18}$-Carbonsäure, einer cycloaliphatischen $C_6$-$C_{12}$-Carbonsäure oder einer aromatischen $C_7$-$C_{15}$-Carbonsäure darstellt und wenn m 2 bedeutet, $R_2$ einen zweiwertigen Acylrest einer aliphatischen $C_2$-$C_{18}$-Dicarbonsäure oder einer cycloaliphatischen oder aromatischen $C_8$-$C_{14}$-Dicarbonsäure, oder einer aliphatischen, cycloaliphatischen oder aromatischen $C_8$-$C_{14}$-Dicarbaminsäure darstellt, oder $R_2$ eine Gruppe

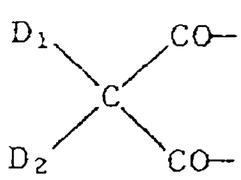

bedeutet, worin $D_1$ $C_1$-$C_8$-Alkyl oder 3,5-Di-tert.butyl-4-hydroxybenzyl darstellt und $D_2$ $D_1$ oder Wasserstoff bedeutet und wenn m 4 darstellt, $R_2$ einen vierwertigen Acylrest einer Butan- oder Pentantetracarbonsäure bedeutet.

5. Masse nach Anspruch 2, eine Verbindung der Formel B enthaltend, worin R Wasserstoff darstellt, $R_1$ eine Gruppe D-CO- bedeutet und D $C_1$-$C_{12}$-Alkyl, $C_1$-$C_4$-Alkoxy, Phenyl, Amino oder Amino, monosubstituiert mit $C_1$-$C_{12}$-Alkyl oder Phenyl, darstellt,
p 1 oder 2 bedeutet, $R_3$ Wasserstoff, $C_1$-$C_{12}$-Alkyl oder $C_2$-$C_{12}$-Alkanoyl darstellt und wenn p 1 bedeutet, $R_4$ $C_1$-$C_{12}$-Alkyl, $C_5$-$C_7$-Cycloalkyl oder eine Gruppe der Formel I darstellt und wenn p 2 bedeutet, $R_4$ $C_2$-$C_8$-Alkylen, Phenylen oder Xylylen darstellt, und wenn $R_3$ nicht Alkanoyl bedeutet, $R_4$ auch einen zweiwertigen Acylrest einer aliphatischen $C_2$-$C_{10}$-Dicarbonsäure oder einer aromatischen $C_6$-Dicarbonsäure oder einer aliphatischen oder aromatischen $C_8$-$C_{15}$ Dicarbaminsäure bedeuten kann.

6. Masse nach Anspruch 1, enthaltend Di(1-acetoxy-2,2,6,6-tetramethylpiperidin-4-yl)sebacat.

7. Masse nach Anspruch 3, enthaltend $\alpha,\alpha'$-(Di-1-ethoxy-2,2,6,6-tetramethylpiperidin-4-yloxy)-p-xylol.

8. Masse nach Anspruch 3, enthaltend 1,4-Dibenzyloxy-2,2,6,6-tetramethylpiperidin.

9. Masse nach Anspruch 3, enthaltend Di-(1-benzyloxy2,2,6,6-tetramethylpiperidin-4-yl)sebacat.

10. Masse nach Anspruch 3, enthaltend 4-Benzyloxy-1-ethoxy-2,2,6,6-tetramethylpiperidin.

11. Masse nach Anspruch 3, enthaltend 1,4-Di-(4-hydroxy-3,5-di-tertbutylbenzoyloxy)-2,2,6,6-tetramethylpiperidin.

12. Masse nach Anspruch 3, enthaltend $\alpha,\alpha'$-(Di-1-benzoyloxy-2,2,6,6-tetramethylpiperidin-4-yloxy)-p-xylol.

13. Überzugsmasse nach Anspruch 1, nämlich ein bei Raumtemperatur härtbares System auf der Grundlage eines Alkydharzes, thermoplastischen acrylischen Harzes, acrylischen Alkydharzes, Polyurethanharzes oder Polyesterharzes, oder den Harzen, modifiziert mit Siliconen, Isocyanaten, Epoxiden, Isocyanuraten, Ketiminen oder Oxazolidinen oder wobei das System auf der Grundlage eines Celluloseesters oder eines Epoxidharzes beruht.

14. Überzugsmasse nach Anspruch 1, nämlich ein säurekatalysiertes wärmehärtbares System auf der Grundlage eines vernetzbaren Acryl-, Polyester-, Polyurethan-, Polyamid- oder Alkydharzes.

15. Masse nach Anspruch 1, nämlich ein Email für industrielle Veredelungsüberzüge.

16. Masse nach Anspruch 1, nämlich ein Ausbesserungsemail für Kraftfahrzeuge.

17. Verbindung gemäß einer der Formeln A' bis N'

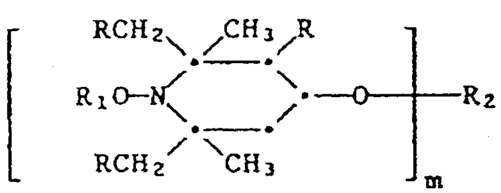

(A')

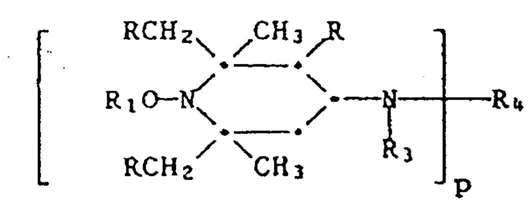

(B')

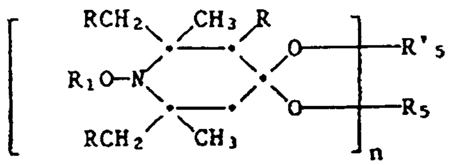

(C')

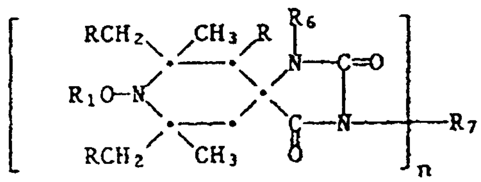

(D')

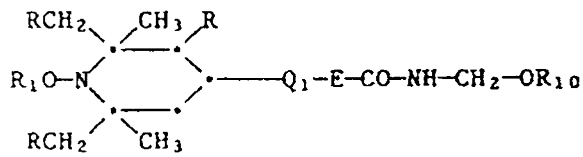

(E')

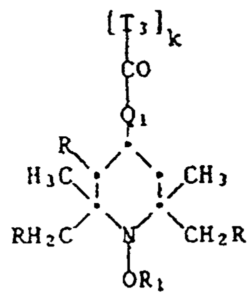

(F')

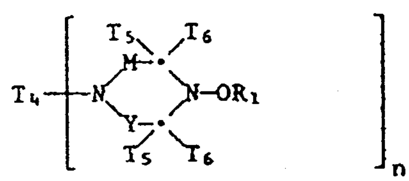

(G')

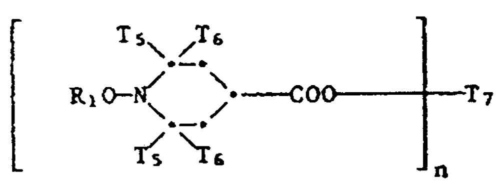

(H')

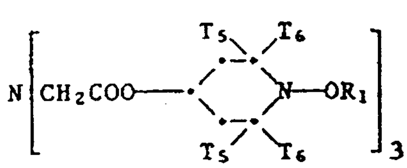

(I')

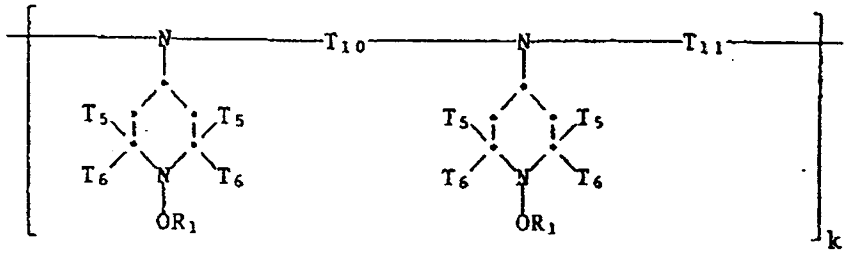

(J')

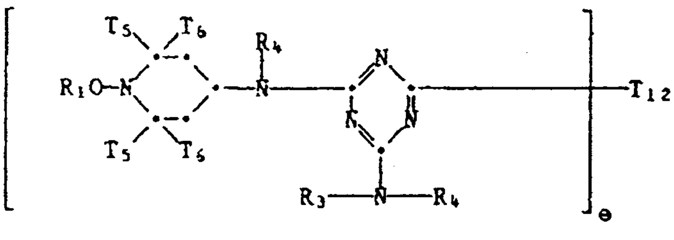

(K')

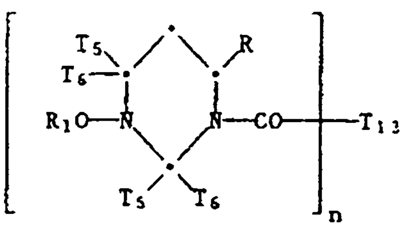

(L')

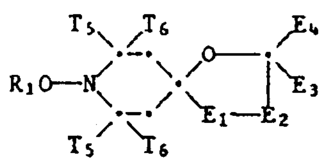

(M')

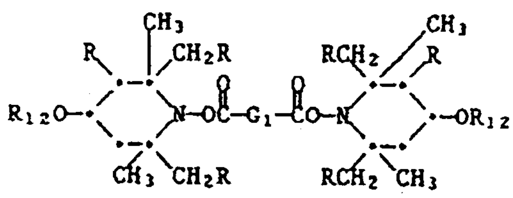

(N')

worin
R Wasserstoff oder Methyl darstellt,
$R_1$ eine Gruppe

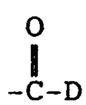

bedeutet, worin D $C_1$-$C_{18}$-Alkyl, $C_1$-$C_{18}$-Alkoxy, Phenyl, Phenyl, substituiert mit Hydroxy, $C_1$-$C_4$-Alkyl oder $C_1$-$C_4$-Alkoxy, oder Amino oder Amino, mono- oder disubstituiert mit $C_1$-$C_{12}$-Alkyl oder Phenyl, darstellt;
m 1-4 bedeutet,
wenn m 1 darstellt
$R_2$ eine Gruppe

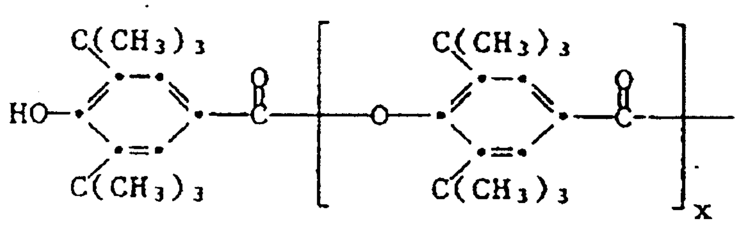

bedeutet, worin x 0 oder 1 darstellt, oder $R_2$ eine Gruppe

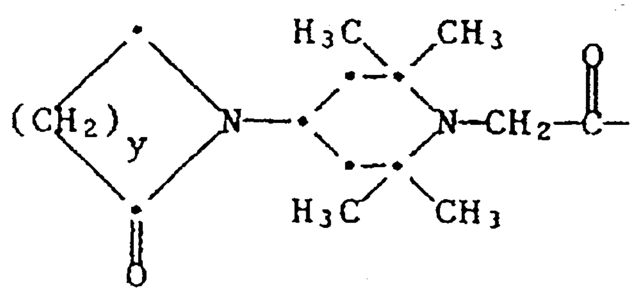

bedeutet, worin y 2-4 darstellt;
wenn m 2 bedeutet und D Alkyl darstellt,
$R_2$ $C_1$-$C_{12}$-Alkylen, $C_4$-$C_{12}$-Alkenylen, Xylylen, einen zweiwertigen Acylrest einer cycloaliphatischen, araliphatischen oder aromatischen Dicarbonsäure mit 8-14 C-Atomen oder einer aliphatischen, cycloaliphatischen oder aromatischen Dicarbaminsäure mit 8-14 C-Atomen bedeutet; oder $R_2$ eine Gruppe

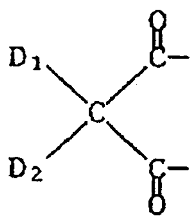

oder

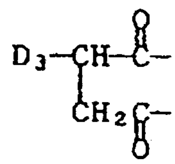

darstellt, worin $D_1$ $C_1$-$C_8$-Alkyl, Benzyl oder 3,5-Di-t-butyl-4-hydroxybenzyl bedeutet, $D_2$ $D_1$ oder Wasserstoff darstellt, $D_3$ $C_1$-$C_{18}$-Alkyl oder $C_2$-$C_{18}$-Alkenyl bedeutet;
wenn m 2 darstellt und D Phenyl oder substituiertes Phenyl, Amino, substituiertes Amino oder Alkoxy bedeutet,
$R_2$ $C_1$-$C_{12}$ Alkylen, $C_4$-$C_{12}$-Alkenylen, Xylylen, einen zweiwertigen Acylrest einer aliphatischen, cycloaliphatischen, araliphatischen oder aromatischen Dicarbon- oder Dicarbaminsäure mit bis zu 14 C-Atomen darstellt; oder $R_2$ eine Gruppe

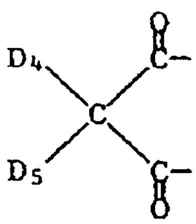

oder

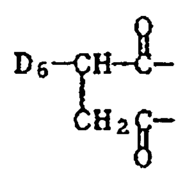

bedeutet, worin $D_4$ und $D_5$ unabhängig voneinander Wasserstoff, $C_1$-$C_8$-Alkyl, Benzyl oder 3,5-Di-t-butyl-4-hydroxybenzyl bedeuten und
$D_6$ $C_1$-$C_{18}$-Alkyl oder $C_2$-$C_{18}$-Alkenyl darstellt;
wenn m 3 bedeutet, $R_2$ einen dreiwertigen Acylrest einer aliphatischen, cycloaliphatischen, oder

aromatischen Tricarbonsäure mit bis zu 12 C-Atomen darstellt; wenn m 4 bedeutet, $R_2$ einen vierwertigen Acylrest einer gesättigten oder ungesättigten, aliphatischen oder aromatischen Tetracarbonsäure mit bis zu 18 C-Atomen darstellt; p 1, 2 oder 3 bedeutet,

$R_3$ Wasserstoff, $C_1$-$C_{12}$-Alkyl, $C_5$-$C_7$-Cycloalkyl, $C_7$-$C_9$-Aralkyl, $C_2$-$C_{18}$-Alkanoyl, $C_3$-$C_5$-Alkenoyl oder Benzoyl darstellt; wenn p 1 bedeutet,

$R_4$ Wasserstoff, $C_1$-$C_{18}$-Alkyl, $C_5$-$C_7$-Cycloalkyl, $C_2$-$C_8$-Alkenyl, unsubstituiert oder substituiert mit einer Cyano-, Carbonyl- oder Carbamidgruppe darstellt, oder Aryl, Aralkyl, Glycidyl, eine Gruppe der Formel $-CH_2$-CH(OH)-Zoder -CONH-Z bedeutet, wobei Z Wasserstoff, Methyl oder Phenyl darstellt;

oder $R_4$ eine Gruppe der Formel

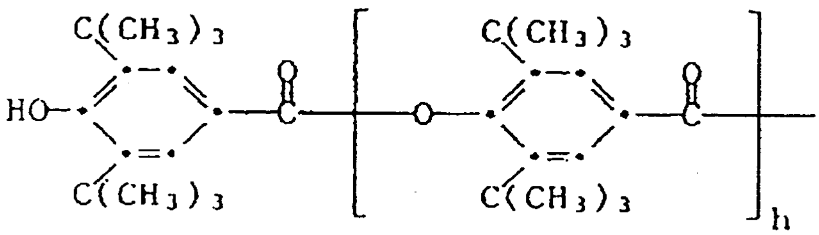

bedeutet, mit h als 0 oder 1; oder eine Gruppe der Formel I

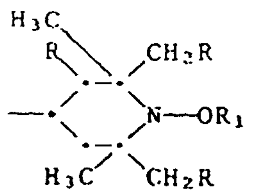

(I);

oder $R_3$ und $R_4$ zusammen Alkylen mit 4 bis 6 Kohlenstoffatomen oder den zweiwertigen Acylrest einer aliphatischen oder aromatischen 1,2- oder 1,3-Dicarbonsäure bedeuten; wenn p 2 darstellt,

$R_4$ $C_1$-$C_{12}$-Alkylen, $C_6$-$C_{12}$-Arylen, Xylylen, eine $-CH_2CH(OH)-CH_2$-Gruppe, oder eine Gruppe $-CH_2-CH(OH)-CH_2-O-X-O-CH_2-CH(OH)-CH_2-$ bedeutet, worin X $C_2$-$C_{10}$-Alkylen, $C_6$-$C_{15}$-Arylen oder $C_6$-$C_{12}$-Cycloalkylen darstellt; oder, mit der Maßgabe, daß wenn $R_3$ nicht Alkanoyl, Alkenoyl oder Benzoyl bedeutet, $R_4$ auch einen zweiwertigen Acylrest einer aliphatischen, cycloaliphatischen oder aromatischen Dicarbon- oder Dicarbaminsäure mit bis zu 14 C-Atomen bedeuten kann, oder die Gruppe -CO- oder

eine Gruppe der Formel II bedeuten kann,

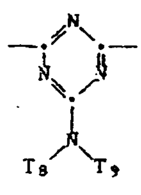

(II)

wobei $T_8$ und $T_9$ unabhängig voneinander Wasserstoff, Alkyl mit 1 bis 18 Kohlenstoffatomen bedeuten, oder $T_8$ und $T_9$ zusammen Alkylen mit 4 bis 6 Kohlenstoffatomen oder 3-Oxapentamethylen bedeuten; wenn p 3 darstellt, $R_4$ 2,4,6-Triazintriyl bedeutet,

n 1 oder 2 darstellt, und wenn n 1 bedeutet,

$R_5$ und $R'_5$ unabhängig voneinander $C_1$-$C_{12}$-Alkyl, $C_2$-$C_{12}$-Alkenyl oder $C_7$-$C_{12}$-Aralkyl bedeuten, oder $R_5$ auch Wasserstoff darstellt, oder $R_5$ und $R'_5$ zusammen $C_2$-$C_8$-Alkylen oder Hydroxyalkylen oder $C_4$-$C_{22}$-Acyloxyalkylen bedeuten;

und wenn n 2 bedeutet,

$R_5$ und $R'_5$ zusammen $(-CH_2)_2C(CH_2-)_2$ bedeuten;

$R_6$ Wasserstoff, $C_1$-$C_{12}$-Alkyl, Allyl, Benzyl, Glycidyl oder $C_2$-$C_6$-Alkoxyalkyl bedeutet;
wenn n 1 darstellt,
$R_7$ Wasserstoff, $C_1$-$C_{12}$-Alkyl, $C_3$-$C_5$-Alkenyl, $C_7$-$C_9$-Aralkyl, $C_5$-$C_7$-Cycloalkyl, $C_2$-$C_4$-Hydroxyalkyl, $C_2$-$C_6$-Alkoxyalkyl, $C_6$-$C_{10}$-Aryl, Glycidyl, eine Gruppe der Formel $-(CH_2)_t$-COO-Q oder der Formel $-(CH_2)_t$-O-CO-Q bedeutet, worin t 1 oder 2 darstellt, und Q $C_1$-$C_4$-Alkyl oder Phenyl bedeutet; und wenn n 2 darstellt,
$R_7$ $C_2$-$C_{12}$-Alkylen, $C_6$-$C_{12}$-Arylen, eine Gruppe $-CH_2CH(OH)-CH_2-O-X-O-CH_2-CH(OH)-CH_2-$, worin X $C_2$-$C_{10}$-Alkylen, $C_6$-$C_{15}$-Arylen oder $C_6$-$C_{12}$-Cycloalkylen bedeutet, oder eine Gruppe $-CH_2CH(OZ')-CH_2-(OCH_2-CH(OZ')CH_2)_2-$ darstellt, worin Z' Wasserstoff, $C_1$-$C_{18}$-Alkyl, Allyl, Benzyl, $C_2$-$C_{12}$-Alkanoyl oder Benzoyl bedeutet; $Q_1$ $-N(R_8)-$ oder -O- darstellt; E $C_1$-$C_3$-Alkylen oder die Gruppe $-CH_2-CH(R_9)-O-$ bedeutet, worin $R_9$ Wasserstoff, Methyl oder Phenyl darstellt, oder E die Gruppe $-(CH_2)_3-NH-$ oder eine direkte Bindung bedeutet;
$R_{10}$ Wasserstoff oder $C_1$-$C_{18}$-Alkyl darstellt, $R_8$ Wasserstoff, $C_1$-$C_{18}$-Alkyl, $C_5$-$C_7$-Cycloalkyl, $C_7$-$C_{12}$-Aralkyl, Cyanoethyl, $C_6$-$C_{10}$-Aryl oder die Gruppe $-CH_2-CH(R_9)-OH$, worin $R_9$ wie vorstehend definiert ist; eine Gruppe der Formel I oder eine Gruppe der Formel

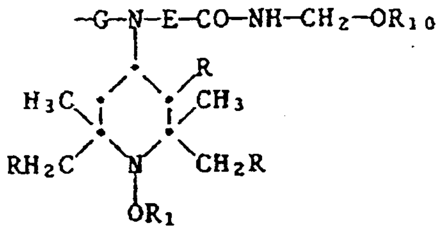

bedeutet, worin G $C_2$-$C_6$-Alkylen oder $C_6$-$C_{12}$-Arylen sein kann; oder $R_8$ eine Gruppe $-E-C0-NH-CH_2-OR_{10}$ darstellt
$T_3$ Ethylen oder 1,2-Propylen bedeutet, oder die sich wiederholende Struktureinheit, abgeleitet von einem $\alpha$-Olefincopolymer mit einem Alkylacrylat oder Methacrylat darstellt;
k 2 bis 100 bedeutet;
$T_4$ die gleiche Bedeutung wie $R_4$ hat, wenn p 1 oder 2 darstellt,
$T_5$ Methyl bedeutet,
$T_6$ Methyl oder Ethyl darstellt, oder $T_5$ und $T_6$ zusammen Tetramethylen oder Pentamethylen bedeuten,
M und Y unabhängig voneinander Methylen oder Carbonyl bedeuten;
$T_7$ das gleiche wie $R_7$ darstellt,
$T_{10}$ und $T_{11}$ unabhängig voneinander Alkylen mit 2 bis 12 Kohlenstoffatomen bedeuten, oder $T_{11}$ eine Gruppe der Formel II bedeutet; e 2, 3 oder 4 darstellt,
$T_{12}$ eine Gruppe

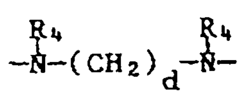

oder

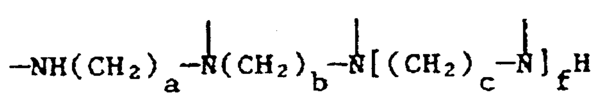

bedeutet, worin a, b und c unabhängig voneinander 2 oder 3 bedeuten, d 2-10 darstellt und f 0 oder 1 bedeutet;
$T_{13}$ das gleiche wie $R_4$ darstellt, mit der Maßgabe, daß $T_{13}$ nicht Wasserstoff sein kann, wenn n 1 bedeutet;
$E_1$ und $E_2$ jeweils verschieden -CO- oder $-N(E_5)-$ bedeuten, worin $E_5$ Wasserstoff, $C_1$-$C_{12}$-Alkyl oder Alkoxycarbonylalkyl mit 4 bis 22 Kohlenstoffatomen darstellt;
$E_3$ Wasserstoff, Alkyl mit 1 bis 30 Kohlenstoffatomen, Phenyl, Naphthyl, die Phenyl- oder die Naphthylgruppe, substituiert mit Chlor oder mit Alkyl mit 1 bis 4 Kohlenstoffatomen, oder Phenylalkyl mit 7 bis 12 Kohlenstoffatomen, oder die Phenylalkylgruppe, substituiert mit Alkyl mit 1 bis 4

Kohlenstoffatomen bedeutet,
$E_4$ Wasserstoff, Alkyl mit 1 bis 30 Kohlenstoffatomen, Phenyl, Naphthyl oder Phenylalkyl mit 7 bis 12 Kohlenstoffatomen darstellt, oder $E_3$ und $E_4$ zusammen Polymethylen mit 4 bis 17 Kohlenstoffatomen, oder die Polymethylengruppe, substituiert mit bis zu vier Alkylgruppen mit 1 bis 4 Kohlenstoffatomen bedeuten; und
$R_{12}$ $C_2$-$C_{18}$-Alkanoyl, $C_3$-$C_6$-Alkenoyl, Benzoyl oder Benzoyl, substituiert mit $C_1$-$C_4$-Alkyl, Halogen oder Hydroxyl bedeutet, und $G_1$ eine direkte Bindung, $C_1$-$C_{12}$-Alkylen, Phenylen oder -NH-G'-NH darstellt, worin G' $C_1$-$C_{12}$-Alkylen bedeutet.

**18.** Verbindung nach Anspruch 17, worin D $C_1$-$C_{18}$-Alkyl, Phenyl oder Phenyl, substituiert mit Hydroxy, $C_1$-$C_4$-Alkyl oder $C_1$-$C_4$-Alkoxy darstellt.

**19.** Verbindung nach Anspruch 17, worin D Amino oder Amino, mono- oder disubstitutiert mit $C_1$-$C_{12}$-Alkyl oder Phenyl, bedeutet.

**20.** Verbindung nach Anspruch 17, worin D $C_1$-$C_{18}$-Alkoxy darstellt.

**21.** Verbindung nach Anspruch 17, worin R Wasserstoff bedeutet.

**22.** Verbindung nach Anspruch 17, worin $R_1$ -CO-D darstellt und D $C_1$-$C_{12}$-Alkyl, $C_1$-$C_4$-Alkoxy, Phenyl, Amino, $C_1$-$C_{12}$-Alkylamino oder Phenylamino bedeutet.

**23.** Verbindung nach Anspruch 17 der Formel A', B', D', F', J', K', M' oder N', worin R Wasserstoff darstellt, $R_1$ wie in Anspruch 22 definiert ist, m 1, 2 oder 4 bedeutet, und wenn m 1 darstellt, $R_2$

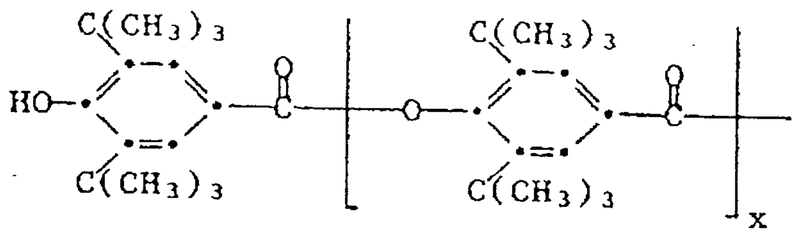

bedeutet, worin x 0 oder 1 darstellt,
wenn m 2 bedeutet und D Alkyl darstellt,
$R_2$ einen zweiwertigen Acylrest einer cycloaliphatischen oder aromatischen Dicarbonsäure mit 8-14 Kohlenstoffatomen, oder einer aliphatischen, cycloaliphatischen oder aromatischen Dicarbaminsäure mit 8-14 Kohlenstoffatomen bedeutet; oder $R_2$ eine Gruppe

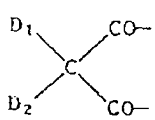

darstellt, worin $D_1$ $C_1$-$C_4$-Alkyl oder 3,5-Di-t-butyl-4-hydroxybenzyl bedeutet und $D_2$ $D_1$ oder Wasserstoff darstellt, und wenn m 2 bedeutet und D Phenyl, Amino, Alkylamino, Phenylamino oder Alkoxy darstellt, $R_2$ einen zweiwertigen Acylrest einer aliphatischen Dicarbonsäure mit 2-8 Kohlenstoffatomen oder eine cycloaliphatische oder aromatische Dicarbonsäure mit 8-14 Kohlenstoffatomen oder eine aliphatische, cycloaliphatische oder aromatische Dicarbaminsäure mit 8-14 Kohlenstoffatomen bedeutet, oder $R_2$ eine Gruppe

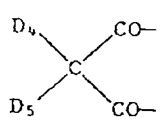

darstellt, worin $D_4$ und $D_5$ unabhängig voneinander Wasserstoff, $C_1$-$C_8$-Alkyl, Benzyl oder 3,5-Di-t-butyl-4-hydroxybenzyl bedeuten, und wenn m 4 bedeutet, $R_2$ einen vierwertigen Acylrest einer 1,2,3,4-Butantetracarbonsäure, 1,2,3,4-But-2-entetracarbonsäure, 1,2,3,5- oder 1,2,4,5-Pentantetracarbonsäure darstellt;
p 1, 2 oder 3 bedeutet,
$R_3$ Wasserstoff, $C_1$-$C_{12}$-Alkyl, Cyclohexyl, $C_7$-$C_9$-Aralkyl, $C_2$- $C_{18}$-Alkanoyl oder Benzoyl darstellt;
wenn p 1 bedeutet,
$R_4$ $C_1$-$C_{18}$-Alkyl, Cyclohexyl, Allyl, Benzyl oder eine Gruppe

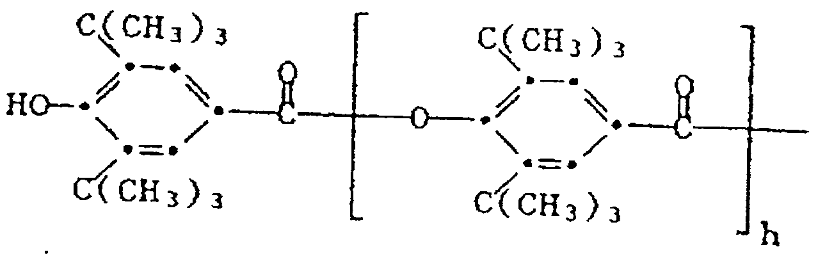

darstellt, mit h als 0 oder 1, oder eine Gruppe

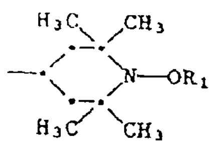

bedeutet, wenn p 2 darstellt,
$R_4$ $C_1$-$C_{12}$-Alkylen, $C_6$-$C_{12}$-Arylen, Xylylen oder eine Gruppe $-CH_2CH(OH)-CH_2-$ bedeutet, oder mit der Maßgabe, daß $R_3$ nicht Alkanoyl oder Benzoyl darstellt, $R_4$ auch einen zweiwertigen Acylrest einer aliphatischen, cycloaliphatischen oder aromatischen Dicarbonsäure oder Dicarbaminsäure mit 6-12 Kohlenstoffatomen bedeuten kann,
oder $R_4$ eine Gruppe der Formel II darstellt,
worin $T_8$ und $T_9$ unabhängig voneinander Wasserstoff oder $C_1$-$C_{12}$-Alkyl bedeuten oder $T_8$ und $T_9$ zusammen $C_4$-$C_6$-Alkylen oder 3-Oxapentamethylen bedeuten, wenn p 3 bedeutet, $R_4$ 2,4,6-Triazintriyl darstellt;
n 1 oder 2 bedeutet und
$R_6$ Wasserstoff, $C_1$-$C_{12}$-Alkyl, Allyl oder Benzyl darstellt, wenn n 1 bedeutet, $R_7$ Wasserstoff, $C_1$-$C_{12}$-Alkyl, Allyl, Benzyl oder Cyclohexyl darstellt,
und wenn n 2 bedeutet, $R_7$ $C_2$-$C_{12}$-Alkylen darstellt;
k 5 bis 50 bedeutet, $T_3$ Ethylen oder Propylen darstellt und $Q_1$ NH oder O bedeutet;
$T_5$ und $T_6$ jeweils Methyl bedeuten,
$T_{10}$ und $T_{11}$ unabhängig voneinander $C_2$-$C_{12}$-Alkylen bedeuten oder $T_{11}$ eine Gruppe der Formel II darstellt,
e 4 bedeutet und $T_{12}$

$$-NH(CH_2)_a-\overset{|}{N}(CH_2)_b-\overset{|}{N}-(CH_2)_c-NH-$$

darstellt, worin a, b und c unabhängig voneinander 2 oder 3 bedeuten;
$E_1$ -CO- bedeutet und $E_2$ $-N(E_5)-$, $E_3$ und $E_4$ unabhängig voneinander $C_1$-$C_{12}$-Alkyl bedeuten oder $E_3$ und $E_4$ zusammen $C_5$-$C_{12}$-Polymethylen bedeuten, und $E_5$ Wasserstoff oder $C_1$-$C_{12}$-Alkyl darstellt;
$G_1$ eine direkte Bindung, $C_2$-$C_8$-Alkylen oder -NH-G'-NH- bedeutet, worin G' $C_2$-$C_8$-Alkylen darstellt, und $R_{12}$ $C_2$-$C_{18}$-Alkanoyl oder Benzoyl bedeutet.

**24.** Verbindung nach Anspruch 17 der Formel A', B', F', J' oder N', worin R Wasserstoff darstellt, $R_1$ -CO-D bedeutet und D $C_1$-$C_4$-Alkyl, $C_1$-$C_4$-Alkoxy, Phenyl, Amino, $C_1$-$C_4$-Alkylamino oder Phenylamino darstellt;
m 1 oder 2 darstellt und
wenn m 1 bedeutet, $R_2$ eine Gruppe

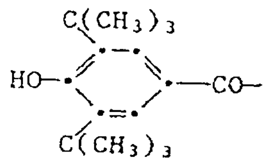

darstellt, wenn m 2 bedeutet und D Alkyl darstellt, $R_2$ einen zweiwertigen Acylrest einer Phenylendicarbonsäure bedeutet oder eine Gruppe -CO-C($D_1$)($D_2$)-CO- darstellt, worin $D_1$ $C_1$-$C_4$-Alkyl oder 3,5-Di-t-butyl-4-hydroxybenzyl bedeutet und $D_2$ $D_1$ oder Wasserstoff darstellt, und wenn m 2 bedeutet und D Alkoxy, Phenyl, Amino oder Alkylamino darstellt,
$R_2$ einen zweiwertigen Acylrest einer aliphatischen oder aromatischen Dicarbonsäure mit 8-10 Kohlenstoffatomen oder einer aliphatischen Dicarbaminsäure mit 8-10 Kohlenstoffatomen bedeutet; oder $R_2$ eine Gruppe

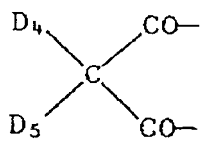

darstellt, worin $D_4$ Alkyl oder 3,5-Di-t-butyl-4-hydroxybenzyl bedeutet und $D_5$ $D_4$ oder Wasserstoff darstellt;
p 1 oder 2 bedeutet, $R_3$ $C_1$-$C_4$-Alkyl, Acetyl oder Benzoyl darstellt,
wenn p 1 bedeutet, $R_4$ $C_1$-$C_{12}$-Alkyl oder eine Gruppe darstellt

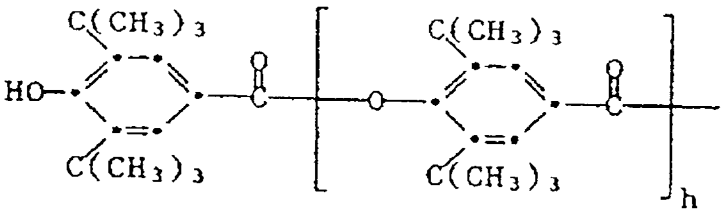

mit h als 0 oder 1,
und wenn p 2 bedeutet, $R_4$ $C_4$-$C_8$-Alkylen darstellt; k 5 bis 50 bedeutet, $T_3$ Ethylen darstellt und $Q_1$ O bedeutet;
$T_5$ und $T_6$ Methyl bedeuten,
$T_{10}$ Hexamethylen darstellt und $T_{11}$ eine Gruppe der Formel II bedeutet, worin $T_8$ und $T_9$ unabhängig voneinander Wasserstoff oder $C_1$-$C_{12}$-Alkyl bedeuten oder $T_8$ und $T_9$ zusammen Pentamethylen oder 3-Oxapentamethylen bedeuten;
$R_{12}$ Acetyl oder Benzoyl darstellt und $G_1$ eine direkte Bindung oder -NH-$(CH_2)_6$-NH- bedeutet.

**25.** Verwendung einer Verbindung nach Anspruch 17 als Stabilisator für organische Materialien gegen Abbau durch harte Strahlung.

**26.** Verwendung nach Anspruch 25, als Stabilisator für organische Polymere.

**27.** Verwendung nach Anspruch 25, als Stabilisator für photographische Schichten.

**28.** Organisches Material, enthaltend eine wirksam stabilisierende Menge einer Verbindung nach Anspruch 17.

**Patentansprüche für folgenden Vertragsstaat : ES**

**1.** Stabilisierte, bei Umgebungstemperatur härtende oder säurekatalysiert wärmehärtende Beschichtungsmasse, enthaltend eine wirksam stabilisierende Menge eines gehinderten Amins mit der Gruppe

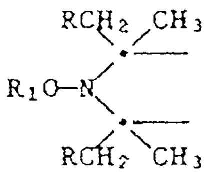

worin R Wasserstoff oder Methyl bedeutet und $R_1$ eine Gruppe D-(C=O)- darstellt, worin D $C_1$-$C_{18}$-Alkyl, $C_1$-$C_{18}$-Alkoxy, Phenyl, Phenyl, substituiert mit Hydroxy, $C_1$-$C_4$-Alkyl oder $C_1$-$C_4$-Alkoxy, bedeutet oder D Amino oder Amino, mono- oder disubstituiert mit $C_1$-$C_{12}$-Alkyl oder Phenyl darstellt.

**2.** Masse nach Anspruch 1, enthaltend eine gehinderte Aminverbindung gemäß einer der Formeln A-N

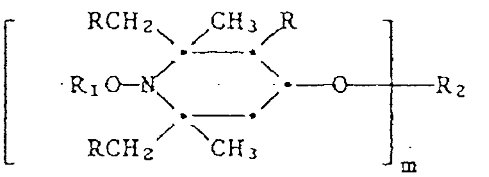

(A)

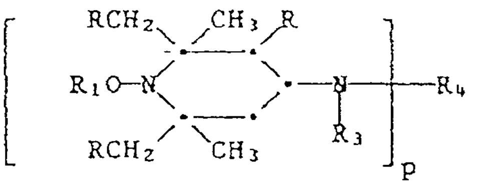

(B)

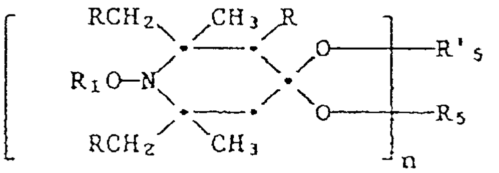

(C)

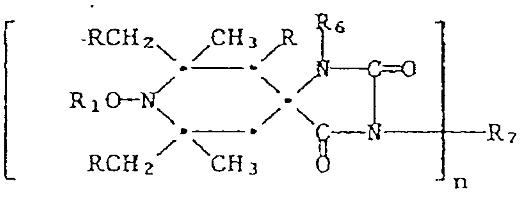

(D)

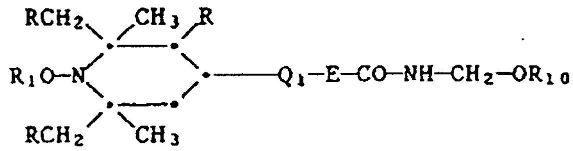

(E)

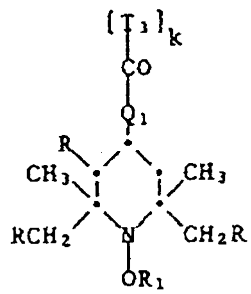

(F)

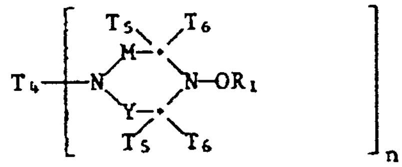

(G)

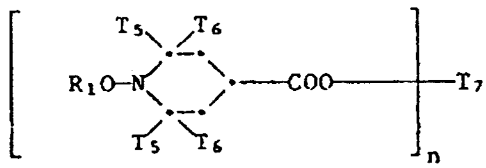

(H)

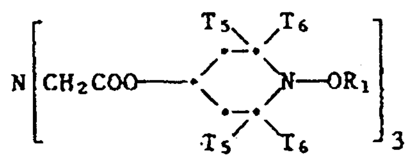

(I)

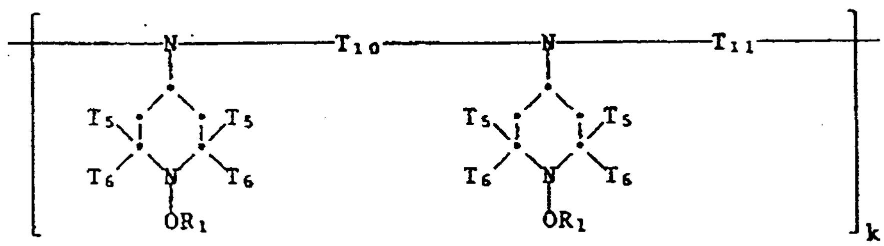

(J)

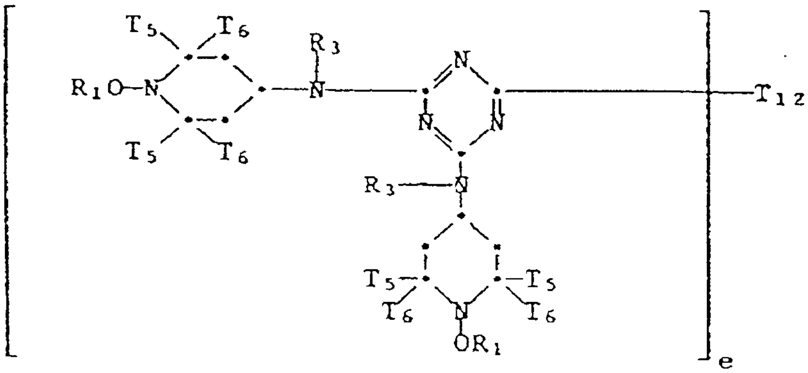

(K)

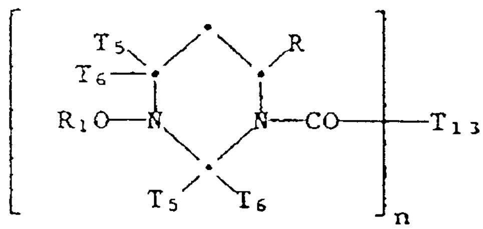

(L)

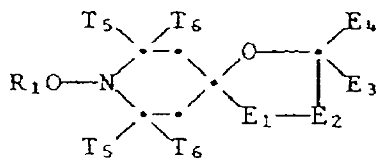

(M)

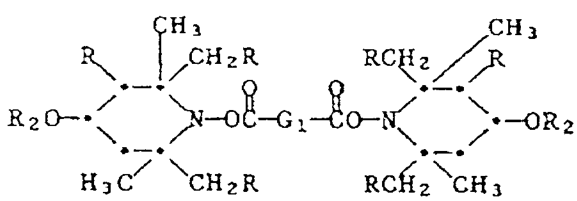

(N)

worin R Wasserstoff oder Methyl bedeutet,
$R_1$ eine Gruppe D-CO- darstellt, worin D $C_1$-$C_{18}$-Alkyl, $C_1$-$C_{18}$-Alkoxy, Phenyl, Phenyl substituiert mit Hydroxy, $C_1$-$C_4$-Alkyl oder $C_1$-$C_4$-Alkoxy, oder Amino oder Amino, mono- oder disubstituiert mit $C_1$-$C_{12}$-Alkyl, oder Phenyl bedeutet;
m 1-4 darstellt,
wenn m 1 bedeutet,
$R_2$ Wasserstoff, $C_1$-$C_{18}$-Alkyl, gegebenenfalls unterbrochen mit einem oder mehreren Sauerstoffatomen, $C_2$-$C_{12}$-Alkenyl, $C_6$-$C_{10}$-Aryl, $C_7$-$C_{18}$-Aralkyl, Glycidyl, einen einwertigen Acylrest einer aliphatischen, cycloaliphatischen, araliphatischen oder aromatischen Carbonsäure, oder einer Carbaminsäure darstellt,
oder $R_2$ eine Gruppe

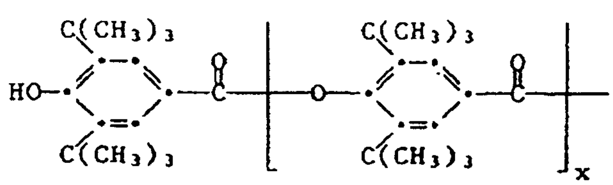

bedeutet, worin x 0 oder 1 darstellt, oder eine Gruppe

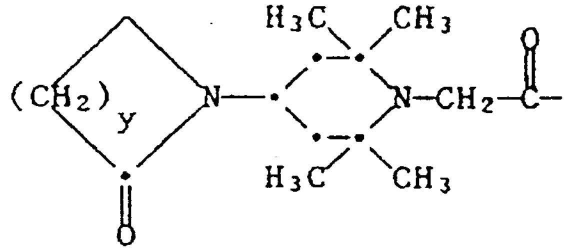

bedeutet,

worin y 2-4 darstellt; wenn m 2 bedeutet,

$R_2$ $C_1$-$C_{12}$-Alkylen, $C_4$-$C_{12}$-Alkenylen, Xylylen, einen zweiwertigen Acylrest einer aliphatischen, cycloaliphatischen, araliphatischen oder aromatischen Dicarbonsäure, oder einer Dicarbaminsäure darstellt, oder $R_2$ eine Gruppe

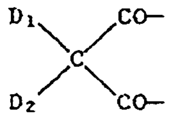

oder

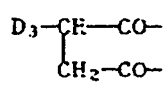

bedeutet, worin $D_1$ und $D_2$ unabhängig voneinander Wasserstoff, einen Alkylrest mit bis zu 8 Kohlenstoffatomen, Phenyl, Benzyl oder 3,5-Di-t-butyl-4-hydroxybenzyl bedeuten, und $D_3$ einen Alkyl- oder Alkenylrest mit bis zu 18 Kohlenstoffatomen darstellt; wenn m 3 bedeutet, $R_2$ einen dreiwertigen Acylrest einer aliphatischen, cycloaliphatischen oder aromatischen Tricarbonsäure darstellt; wenn m 4 bedeutet, $R_2$ einen vierwertigen Acylrest einer gesättigten oder ungesättigten, aliphatischen oder aromatischen Tetracarbonsäure darstellt; p 1, 2 oder 3 bedeutet,

$R_3$ Wasserstoff, $C_1$-$C_{12}$-Alkyl, $C_5$-$C_7$-Cycloalkyl, $C_7$-$C_9$-Aralkyl, $C_2$-$C_{18}$-Alkanoyl, $C_3$-$C_5$-Alkenoyl oder Benzoyl darstellt; wenn p 1 bedeutet,

$R_4$ Wasserstoff, $C_1$-$C_{18}$-Alkyl, $C_5$-$C_7$-Cycloalkyl, $C_2$-$C_8$-Alkenyl, unsubstituiert oder substituiert mit einer Cyano-, Carbonyl- oder Carbamidgruppe darstellt, oder Aryl, Aralkyl, Glycidyl, eine Gruppe der Formel $-CH_2$-CH(OH)-Z oder der Formel -CONH-Z bedeutet, worin Z Wasserstoff, Methyl oder Phenyl darstellt; oder $R_4$ eine Gruppe der Formel I

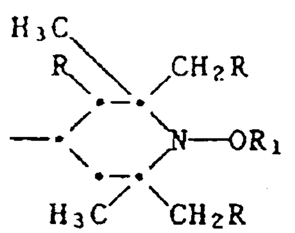

(I) oder

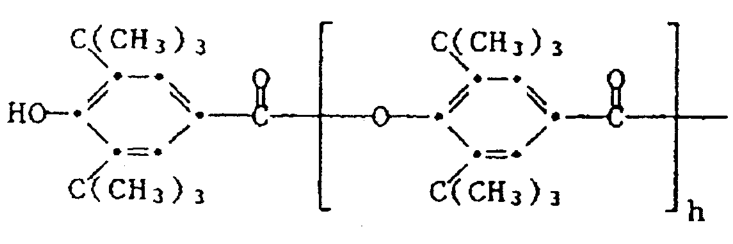

bedeutet, mit h als 0 oder 1;

oder $R_3$ und $R_4$ zusammen Alkylen mit 4 bis 6 Kohlenstoffatomen oder 1-Oxoalkylen oder den zweiwertigen Acylrest einer aliphatischen oder aromatischen 1,2- oder 1,3-Dicarbonsäure bedeuten;

wenn p 2 darstellt,

$R_4$ $C_1$-$C_{12}$-Alkylen, $C_6$-$C_{12}$-Arylen, Xylylen, eine $-CH_2CH(OH)$-$CH_2$-Gruppe, oder eine Gruppe $-CH_2$-CH-

(OH)-$CH_2$-O-X-O-$CH_2$-CH(OH)-$CH_2$- bedeutet, worin X $C_2$-$C_{10}$-Alkylen, $C_6$-$C_{15}$-Arylen oder $C_6$-$C_{12}$-Cycloalkylen darstellt; oder, mit der Maßgabe, daß $R_3$ nicht Alkanoyl, Alkenoyl oder Benzoyl bedeutet, $R_4$ auch einen zweiwertigen Acylrest einer aliphatischen, cycloaliphatischen oder aromatischen Dicarbon- oder Dicarbaminsäure bedeuten kann, oder die Gruppe -CO- bedeuten kann; oder $R_4$ eine Gruppe der Formel II

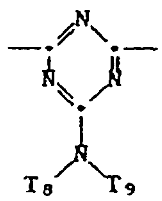

(II)

darstellt, worin $T_8$ und $T_9$ unabhängig voneinander Wasserstoff oder Alkyl mit 1 bis 18 Kohlenstoffatomen bedeuten, oder $T_8$ und $T_9$ zusammen Alkylen mit 4 bis 6 Kohlenstoffatomen oder 3-Oxapentamethylen bedeuten, vorzugsweise $T_8$ und $T_9$ zusammen 3-Oxapentamethylen bedeuten;
wenn p 3 bedeutet, $R_4$ 2,4,6-Triazintriyl darstellt;
n 1 oder 2 bedeutet und
wenn n 1 bedeutet,
$R_5$ und $R'_5$ unabhängig voneinander $C_1$-$C_{12}$-Alkyl, $C_2$-$C_{12}$-Alkenyl, $C_7$-$C_{12}$-Aralkyl bedeuten, oder $R_5$ auch Wasserstoff darstellt, oder $R_5$ und $R'_5$ zusammen $C_2$-$C_8$-Alkylen oder Hydroxyalkylen oder $C_4$-$C_{22}$-Acyloxyalkylen bedeuten;
wenn n 2 bedeutet,
$R_5$ und $R'_5$ zusammen $(-CH_2)_2C(CH_2-)_2$ bedeuten;
$R_6$ Wasserstoff, $C_1$-$C_{12}$-Alkyl, Allyl, Benzyl, Glycidyl oder $C_2$-$C_6$-Alkoxyalkyl darstellt;
wenn n 1 bedeutet,
$R_7$ Wasserstoff, $C_1$-$C_{12}$-Alkyl, $C_3$-$C_5$-Alkenyl, $C_7$-$C_9$-Aralkyl, $C_5$-$C_7$-Cycloalkyl, $C_2$-$C_4$-Hydroxyalkyl, $C_2$-$C_6$-Alkoxyalkyl, $C_6$-$C_{10}$-Aryl, Glycidyl, eine Gruppe der Formel $-(CH_2)_t$-COO-Q oder der Formel $-(CH_2)_t$-O-CO-Q darstellt, worin t 1 oder 2 bedeutet, und Q $C_1$-$C_4$-Alkyl oder Phenyl darstellt; oder wenn n 2 bedeutet,
$R_7$ $C_2$-$C_{12}$-Alkylen, $C_6$-$C_{12}$-Arylen, eine Gruppe -$CH_2$CH(OH)-$CH_2$-O-X-O-$CH_2$-CH(OH)-$CH_2$- darstellt, worin X $C_2$-$C_{10}$-Alkylen, $C_6$-$C_{15}$-Arylen oder $C_6$-$C_{12}$-Cycloalkylen, oder eine Gruppe -$CH_2$CH(OZ')$CH_2$-(O$CH_2$-CH(OZ')$CH_2$)$_2$- bedeutet, worin Z' Wasserstoff, $C_1$-$C_{18}$-Alkyl, Allyl, Benzyl, $C_2$-$C_{12}$-Alkanoyl oder Benzoyl darstellt; $Q_1$ -N($R_8$)- oder -O- bedeutet;
E $C_1$-$C_3$-Alkylen oder die Gruppe -$CH_2$-CH($R_9$)-O- darstellt, worin $R_9$ Wasserstoff, Methyl oder Phenyl bedeutet, oder E die Gruppe $-(CH_2)_3$-NH- oder eine direkte Bindung darstellt;
$R_{10}$ Wasserstoff oder $C_1$-$C_{18}$-Alkyl bedeutet;
$R_8$ Wasserstoff, $C_1$-$C_{18}$-Alkyl, $C_5$-$C_7$-Cycloalkyl, $C_7$-$C_{12}$-Aralkyl, Cyanoethyl, $C_6$-$C_{10}$-Aryl, die Gruppe -$CH_2$-CH($R_9$)-OH, worin $R_9$ wie vorstehend definiert ist; eine Gruppe der Formel I oder eine Gruppe der Formel

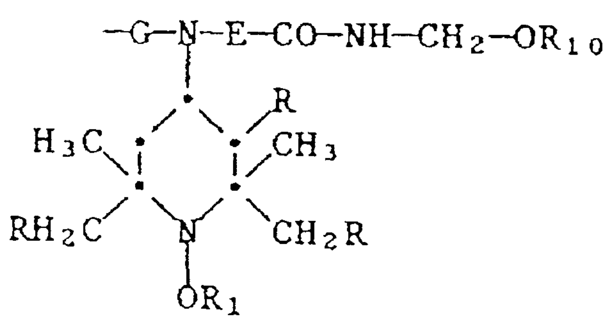

darstellt, worin G $C_2$-$C_6$-Alkylen oder $C_6$-$C_{12}$-Arylen bedeuten kann; oder $R_8$ eine Gruppe -E-CO-NH-$CH_2$-$OR_{10}$ bedeutet;
$T_3$ Ethylen oder 1,2-Propylen darstellt, oder die sich wiederholende Struktureinheit, abgeleitet von einem $\alpha$-Olefincopolymer mit einem Alkylacrylat oder -methacrylat ist;
k 2 bis 100 darstellt;

$T_4$ die gleiche Bedeutung wie $R_4$ hat, wenn p 1 oder 2 bedeutet,
$T_5$ Methyl darstellt,
$T_6$ Methyl oder Ethyl bedeutet,
M und Y unabhängig voneinander Methylen oder Carbonyl bedeuten;
$T_7$ das gleiche wie $R_7$ darstellt;
$T_{10}$ und $T_{11}$ unabhängig voneinander Alkylen mit 2 bis 12 Kohlenstoffatomen bedeuten, oder $T_{11}$ eine Gruppe der Formel II bedeutet;
e 2, 3 oder 4 darstellt und
$T_{12}$ eine Gruppe $-N(R_5)-(CH_2)_d-N(R_5)-$ oder

$$-NH(CH_2)_a-\overset{|}{N}(CH_2)_b-\overset{|}{N}[(CH_2)_c-\overset{|}{N}]_fH$$

bedeutet, worin a, b und c unabhängig voneinander 2 oder 3 bedeuten, d 2 bis 10 darstellt und f 0 oder 1 bedeutet; $T_{13}$ das gleiche wie $R_4$ darstellt, mit der Maßgabe, daß $R_{13}$ nicht Wasserstoff sein kann, wenn n 1 ist;
$E_1$ und $E_2$ jeweils verschieden -CO- oder $-N(E_5)-$ bedeuten, wobei $E_5$ Wasserstoff, $C_1$-$C_{12}$-Alkyl oder $C_4$-$C_{22}$-Alkoxycarbonylalkyl bedeutet;
$E_3$ Wasserstoff, Alkyl mit 1 bis 30 Kohlenstoffatomen, Phenyl, Naphthyl, die Phenyl- oder die Naphthylgruppe, substituiert mit Chlor oder mit Alkyl mit 1 bis 4 Kohlenstoffatomen, oder Phenylalkyl mit 7 bis 12 Kohlenstoffatomen, oder die Phenylalkylgruppe, substitutiert mit Alkyl mit 1 bis 4 Kohlenstoffatomen, darstellt,
$E_4$ Wasserstoff, Alkyl mit 1 bis 30 Kohlenstoffatomen, Phenyl, Naphthyl oder Phenylalkyl mit 7 bis 12 Kohlenstoffatomen bedeutet, oder
$E_3$ und $E_4$ zusammen Polymethylen mit 4 bis 17 Kohlenstoffatomen, oder die Polymethylengruppe, substituiert mit bis zu vier Alkylgruppen mit 1 bis 4 Kohlenstoffatomen, bedeuten;
$R_2$ von Formel (N) wie vorstehend definiert ist, wenn m 1 darstellt;
$G_1$ eine direkte Bindung, $C_1$-$C_{12}$-Alkylen, Phenylen oder -NH-G'-NH bedeutet, worin G' $C_1$-$C_{12}$-Alkylen ist.

**3.** Masse nach Anspruch 2, eine Verbindung der Formeln A, B, D, J, K oder M enthaltend, worin R Wasserstoff darstellt und $T_5$ und $T_6$ Methyl bedeuten.

**4.** Masse nach Anspruch 2, eine Verbindung der Formel A enthaltend, worin R Wasserstoff bedeutet,
$R_1$ eine Gruppe D-CO- darstellt und D $C_1$-$C_{12}$-Alkyl, $C_1$-$C_4$-Alkoxy, Phenyl, Amino oder Amino, monosubstituiert mit $C_1$-$C_{12}$-Alkyl oder Phenyl bedeutet,
m 1, 2 oder 4 darstellt und wenn m 1 bedeutet,
$R_2$ einen Acylrest einer aliphatischen $C_2$-$C_{18}$-Carbonsäure, einer cycloaliphatischen $C_6$-$C_{12}$-Carbonsäure oder einer aromatischen $C_7$-$C_{15}$-Carbonsäure darstellt und wenn m 2 bedeutet, $R_2$ einen zweiwertigen Acylrest einer aliphatischen $C_2$-$C_{18}$-Dicarbonsäure oder einer cycloaliphatischen oder aromatischen $C_8$-$C_{14}$-Dicarbonsäure, oder einer aliphatischen, cycloaliphatischen oder aromatischen $C_8$-$C_{14}$-Dicarbaminsäure darstellt, oder $R_2$ eine Gruppe

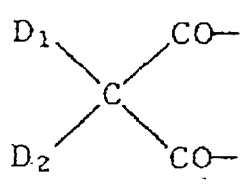

bedeutet, worin $D_1$ $C_1$-$C_8$-Alkyl oder 3,5-Di-tert.butyl-4-hydroxybenzyl darstellt und $D_2$ $D_1$ oder Wasserstoff bedeutet und wenn m 4 darstellt, $R_2$ einen vierwertigen Acylrest einer Butan- oder Pentantetracarbonsäure bedeutet.

**5.** Masse nach Anspruch 2, eine Verbindung der Formel B enthaltend, worin R Wasserstoff darstellt,
$R_1$ eine Gruppe D-CO- bedeutet und D $C_1$-$C_{12}$-Alkyl, $C_1$-$C_4$-Alkoxy, Phenyl, Amino oder Amino, monosubstituiert mit $C_1$-$C_{12}$-Alkyl oder Phenyl, darstellt,

p 1 oder 2 bedeutet, $R_3$ Wasserstoff, $C_1$-$C_{12}$-Alkyl oder $C_2$-$C_{12}$-Alkanoyl darstellt und wenn p 1 bedeutet, $R_4$ $C_1$-$C_{12}$-Alkyl, $C_5$-$C_7$-Cycloalkyl oder eine Gruppe der Formel I darstellt und wenn p 2 bedeutet, $R_4$ $C_2$-$C_8$-Alkylen, Phenylen oder Xylylen darstellt, und wenn $R_3$ nicht Alkanoyl bedeutet, $R_4$ auch einen zweiwertigen Acylrest einer aliphatischen $C_2$-$C_{10}$-Dicarbonsäure oder einer aromatischen $C_6$-Dicarbonsäure oder einer aliphatischen oder aromatischen $C_8$-$C_{15}$ Dicarbaminsäure bedeuten kann.

6. Masse nach Anspruch 1, enthaltend Di(1-acetoxy-2,2,6,6-tetramethylpiperidin-4-yl)sebacat.

7. Masse nach Anspruch 3, enthaltend $\alpha,\alpha'$-(Di-1-ethoxy-2,2,6,6-tetramethylpiperidin-4-yloxy)-p-xylol.

8. Masse nach Anspruch 3, enthaltend 1,4-Dibenzyloxy-2,2,6,6-tetramethylpiperidin.

9. Masse nach Anspruch 3, enthaltend Di-(1-benzyloxy-2,2,6,6-tetramethylpiperidin-4-yl)sebacat.

10. Masse nach Anspruch 3, enthaltend 4-Benzyloxy-1-ethoxy-2,2,6,6-tetramethylpiperidin.

11. Masse nach Anspruch 3, enthaltend 1,4-Di-(4-hydroxy-3,5-di-tertbutylbenzoyloxy)-2,2,6,6-tetramethylpiperidin.

12. Masse nach Anspruch 3, enthaltend $\alpha,\alpha'$-(Di-1-benzoyloxy-2,2,6,6-tetramethylpiperidin-4-yloxy)-p-xylol.

13. Beschichtungsmasse nach Anspruch 1, nämlich ein bei Raumtemperatur härtbares System auf der Grundlage eines Alkydharzes, thermoplastischen acrylischen Harzes, acrylischen Alkydharzes, Polyurethanharzes oder Polyesterharzes, oder der Harze, modifiziert mit Siliconen, Isocyanaten, Epoxiden, Isocyanuraten, Ketiminen oder Oxazolidinen oder wobei das System auf der Grundlage eines Celluloseesters oder eines Epoxidharzes beruht.

14. Beschichtungsmasse nach Anspruch 1, nämlich ein säurekatalysiertes wärmehärtbares System auf der Grundlage eines vernetzbaren Acryl-, Polyester-, Polyurethan-, Polyamid- oder Alkydharzes.

15. Masse nach Anspruch 1, nämlich ein Email für industrielle Veredelungsüberzüge.

16. Masse nach Anspruch 1, nämlich ein Ausbesserungsemail für Kraftfahrzeuge.

**Revendications**
**Revendications pour les Etats contractants suivants : BE, DE, FR, GB, IT, NL, SE**

1. Une composition stabilisée de revêtement thermodurcissable catalysée par un acide ou durcissable à température ambiante contenant une quantité efficace pour la stabilisation d'un composé aminé à empêchement stérique contenant le groupement

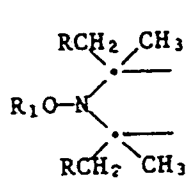

dans lequel R est un atome d'hydrogène ou un groupement méthyle et $R_1$ est un groupement

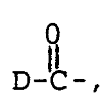

dans lequel D est un groupement alkyle en $C_1$-$C_{18}$, alkoxy en $C_1$-$C_{18}$, phényle, phényle substitué par un groupement hydroxy, alkyle en $C_1$-$C_4$ ou alkoxy en $C_1$-$C_4$, ou D est un groupement amino ou amino

mono- ou di-substitué par un groupement alkyle en $C_1$-$C_{12}$ ou phényle.

**2.** La composition selon la Revendication 1, qui contient un composé aminé à empêchement stérique correspondant à l'une des formules A-N

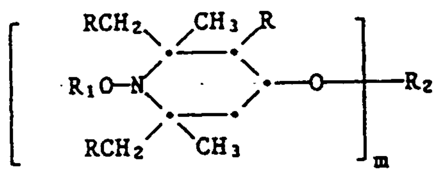

(A)

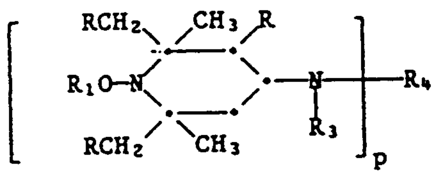

(B)

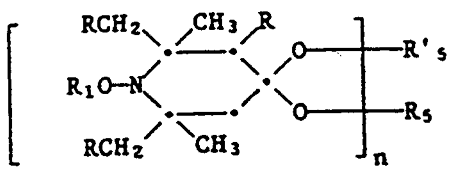

(C)

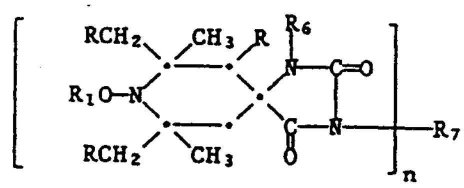

(D)

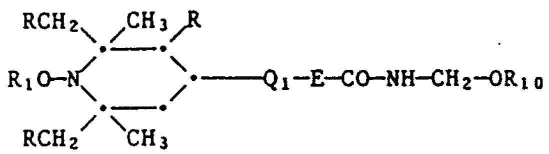

(E)

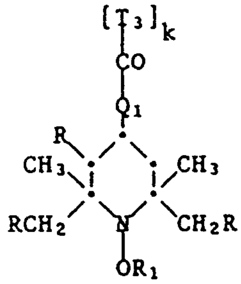

(F)

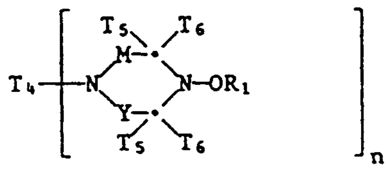

(G)

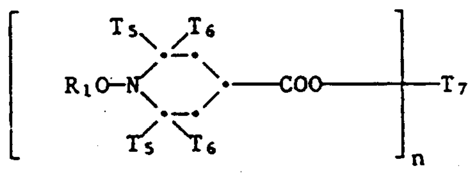

(H)

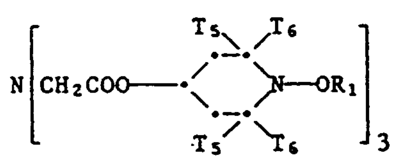

(I)

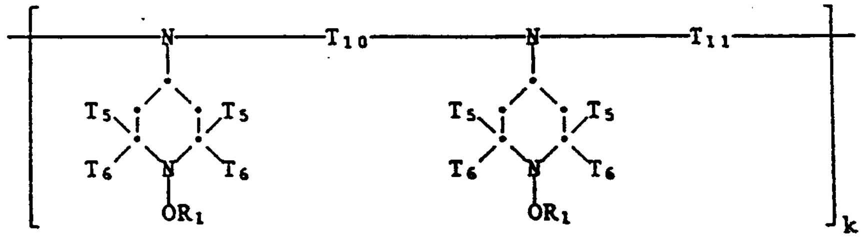

(J)

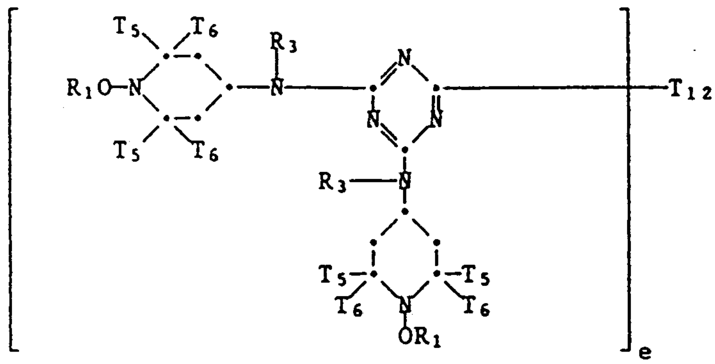

(K)

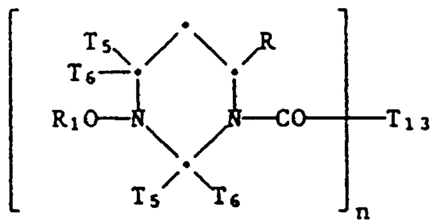

(L)

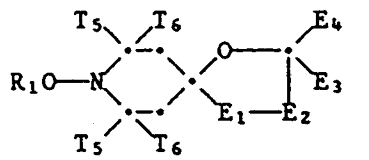

(M)

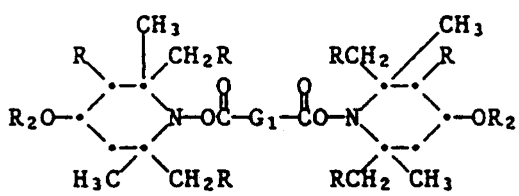

(N)

dans laquelle

R est un atome d'hydrogène ou un groupement méthyle,

$R_1$ est un groupement D-CO- dans lequel D est un groupement alkyle en $C_1$-$C_{18}$, alkoxy en $C_1$-$C_{18}$, phényle, phényle substitué par un groupement hydroxy, alkyle en $C_1$-$C_4$ ou alkoxy en $C_1$-$C_4$, ou un groupement amino ou amino mono- ou di-substitué par un groupement alkyle en $C_1$-$C_{12}$, ou phényle ;

m vaut 1-4,

lorsque m vaut 1,

$R_2$ est un atome d'hydrogène, un groupement alkyle en $C_1$-$C_{18}$, éventuellement interrompu par un ou plusieurs atomes d'oxygène, alkényle en $C_2$-$C_{12}$, aryle en $C_6$-$C_{10}$, aralkyle en $C_7$-$C_{18}$, glycidyle, un radical acyle monovalent d'un acide carboxylique aliphatique, cycloaliphatique, araliphatique ou aromatique, ou d'un acide carbamique, ou $R_2$ est un groupement

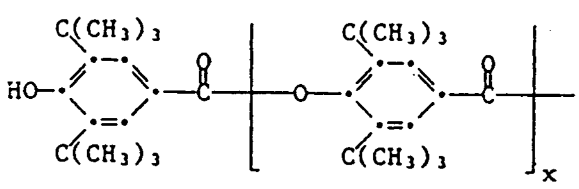

dans lequel x vaut 0 ou 1, ou est un groupement

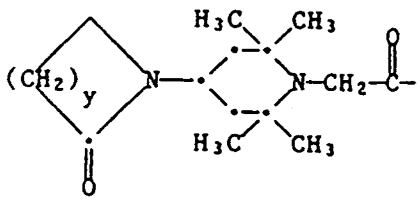

dans lequel y vaut 2-4 ;
lorsque m vaut 2,
$R_2$ est un groupement alkylène en $C_1$-$C_{12}$, alkénylène en $C_4$-$C_{12}$, xylylène, un radical acyle divalent d'un acide dicarboxylique aliphatique, cycloaliphatique, araliphatique ou aromatique ou d'un acide dicarbamique, ou $R_2$ est un groupement

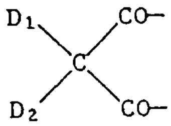

ou

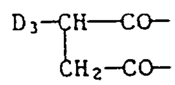

dans lequel $D_1$ et $D_2$ représentent indépendamment un atome d'hydrogène, un radical alkyle comprenant jusqu'à 8 atomes de carbone, un groupement phényle, benzyle ou 3,5-di-t-butyl-4-hydroxybenzyle et $D_3$ est un radical alkyle ou alkényle contenant jusqu'à 18 atomes de carbone ; lorsque m vaut 3, $R_2$ est un radical acyle trivalent d'un acide tricarboxylique aliphatique, cycloaliphatique ou aromatique ; lorsque m vaut 4, $R_2$ est un radical acyle tétravalent d'un acide tétracarboxylique aliphatique ou aromatique saturé ou insaturé ; p vaut 1, 2 ou 3,
$R_3$ représente un atome d'hydrogène, un groupement alkyle en $C_1$-$C_{12}$, cycloalkyle en $C_5$-$C_7$, aralkyle en $C_7$-$C_9$, alkanoyle en $C_2$-$C_{18}$, alkénoyle en $C_3$-$C_5$, ou benzoyle ;
lorsque p vaut 1,
$R_4$ représente un atome d'hydrogène, un groupement alkyle en $C_1$-$C_{18}$, cycloalkyle en $C_5$-$C_7$, alkényle en $C_2$-$C_8$ non substitué ou substitué par un groupement cyano, carbonyle ou carbamide, ou représente un groupement aryle, aralkyle, glycidyle, un groupement de formule $-CH_2-CH(OH)-Z$ ou de formule -CONH-Z dans laquelle Z représente un atome d'hydrogène, un groupement méthyle ou phényle ;
ou $R_4$ est un groupement de formule I

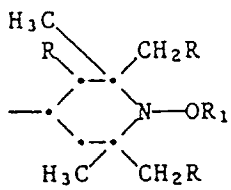

(I) ou

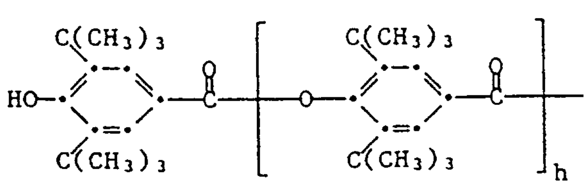

avec h valant 0 ou 1;

ou $R_3$ et $R_4$ pris ensemble sont un groupement alkylène possédant de 4 à 6 atomes de carbone, ou 1-oxo-alkylène ou le radical acyle divalent d'un acide 1,2- ou 1,3-dicarboxylique aliphatique ou aromatique ;
lorsque p vaut 2,
$R_4$ est un groupement alkylène en $C_1$-$C_{12}$, arylène en $C_6$-$C_{12}$, xylylène, un groupement $-CH_2CH(OH)-CH_2$, ou un groupement $-CH_2-CH(OH)-CH_2-O-X-O-CH_2-CH(OH)-CH_2-$ dans lequel X représente un

groupement alkylène en $C_2$-$C_{10}$, arylène en $C_6$-$C_{15}$ ou cycloalkylène en $C_6$-$C_{12}$ ou, à condition que $R_3$ ne soit pas un groupement alkanoyle, alkénoyle ou benzoyle, $R_4$ peut aussi être un radical acyle divalent d'un acide dicarbamique ou dicarboxylique aliphatique, cycloaliphatique ou aromatique, ou peut être le groupe -CO- ; ou $R_4$ représente un groupement de formule II

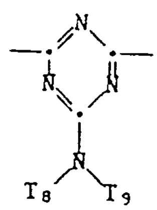

(II)

dans laquelle $T_8$ et $T_9$ représentent indépendamment un atome d'hydrogène, un groupement alkyle de 1 à 18 atomes de carbone, ou $T_8$ et $T_9$ représentent ensemble un groupement alkylène de 4 à 6 atomes de carbone ou 3-oxapentaméthylène, $T_8$ et $T_9$ représentant de préférence conjointement un groupement 3-oxapentaméthylène ;

lorsque p vaut 3, $R_4$ représente un groupement 2,4,6-triazinetriyle ;

n vaut 1 ou 2 et

lorsque n vaut 1,

$R_5$ et $R'_5$ représentent indépendamment un groupement alkyle en $C_1$-$C_{12}$, alkényle en $C_2$-$C_{12}$, aralkyle en $C_7$-$C_{12}$, ou $R_5$ représente aussi un atome d'hydrogène ou $R_5$ et $R'_5$ représentent ensemble un groupement alkylène en $C_2$-$C_8$, ou hydroxyalkylène ou (acyle en $C_4$-$C_{22}$)oxyalkylène ;

lorsque n vaut 2, $R_5$ et $R'_5$ représentent ensemble $(-CH_2)_2C(CH_2-)_2$ ;

$R_6$ représente un atome d'hydrogène, un groupement alkyle en $C_1$-$C_{12}$, allyle, benzyle, glycidyle ou alkoxyalkyle en $C_2$-$C_6$ ;

lorsque n vaut 1,

$R_7$ représente un atome d'hydrogène, un groupement alkyle en $C_1$-$C_{12}$, alkényle en $C_3$-$C_5$, aralkyle en $C_7$-$C_9$, cycloalkyle en $C_5$-$C_7$, hydroxyalkyle en $C_2$-$C_4$, alkoxyalkyle en $C_2$-$C_6$, aryle en $C_6$-$C_{10}$, glycidyle, un groupement de formule $-(CH_2)_t$-COO-Q ou de formule $-(CH_2)_t$-O-CO-Q dans laquelle t vaut 1 ou 2, et Q représente un groupement alkyle en $C_1$-$C_4$, ou phényle ; ou

lorsque n vaut 2,

$R_7$ représente un groupement alkylène en $C_2$-$C_{12}$, arylène en $C_6$-$C_{12}$, un groupement $-CH_2CH(OH)-CH_2$-O-X-O-$CH_2$-CH(OH)-$CH_2$- dans lequel X est un groupement alkylène en $C_2$-$C_{10}$, arylène en $C_6$-$C_{15}$ ou cycloalkylène en $C_6$-$C_{12}$, ou un groupement $-CH_2CH(OZ')CH_2-(OCH_2-CH(OZ')CH_2)_2$- dans lequel Z' est un atome d'hydrogène, un groupement (alkyle en $C_1$-$C_{18}$), allyle, benzyle, (alkanoyle en $C_2$-$C_{12}$), ou benzoyle ;

$Q_1$ représente -N($R_8$)- ou -O- ;

E est un groupement alkylène en $C_1$-$C_3$, le groupement $CH_2$-CH($R_9$)-O- dans lequel $R_9$ est un atome d'hydrogène, un groupement méthyle ou phényle, ou E est le groupement $-(CH_2)_3$-NH- ou une liaison directe ;

$R_{10}$ est un atome d'hydrogène ou un groupement alkyle en $C_1$-$C_{18}$,

$R_8$ est un atome d'hydrogène, un groupement alkyle en $C_1$-$C_{18}$, cycloalkyle en $C_5$-$C_7$, aralkyle en $C_7$-$C_{12}$, cyanoéthyle, aryle en $C_6$-$C_{10}$, le groupement $-CH_2$-CH($R_9$)-OH dans lequel $R_9$ a la signification définie ci-dessus ; un groupement de formule I ou un groupement de formule

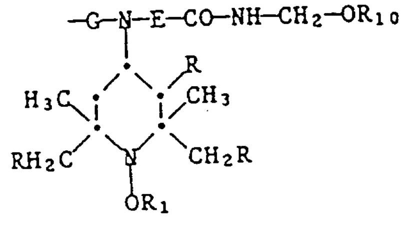

dans laquelle G peut être un groupement alkylène en $C_2$-$C_6$ ou arylène en $C_6$-$C_{12}$ ; ou $R_8$ est un groupe -E-CO-NH-$CH_2$-$OR_{10}$ ;

$T_3$ est un groupement éthylène ou 1,2-propylène, ou est l'unité structurale répétitive dérivée d'un copolymère d'alpha-oléfine avec un acrylate ou un méthacrylate d'alkyle ;
k vaut de 2 à 100 ;
$T_4$ a la même signification que $R_4$ lorsque p vaut 1 ou 2,
$T_5$ représente un groupement méthyle,
$T_6$ est un groupement méthyle ou éthyle,
M et Y représentent indépendamment un groupement méthylène ou carbonyle ;
$T_7$ est identique à $R_7$ ;
$T_{10}$ et $T_{11}$ représentent indépendamment un groupement alkylène de 2 à 12 atomes de carbone, ou $T_{11}$ représente un groupe de formule II ;
e vaut 2, 3 ou 4 et
$T_{12}$ est un groupe $-N(R_5)-(CH_2)_d-N(R_5)-$ ou $-NH(CH_2)_a-N(CH_2)_b-N[(CH_2)_c-N]_fH$ dans lequel a, b et c valent indépendamment 2 ou 3, d vaut de 2 à 10 et f vaut 0 ou 1 ;
$T_{13}$ est le même que $R_4$ à condition que $R_{13}$ ne puisse pas être un hydrogène lorsque n vaut 1 ;
$E_1$ et $E_2$, étant différents, représentent chacun -CO- ou $-N(E_5)-$, dans lequel $E_5$ est un atome d'hydrogène, un groupement alkyle en $C_1$-$C_{12}$ ou alkoxycarbonylalkyle en $C_4$-$C_{22}$, $E_3$ est un groupement hydrogène, un groupement alkyle de 1 à 30 atomes de carbone, phényle, naphtyle, ledit groupement phényle ou ledit groupement naphtyle étant substitués par un atome de chlore ou par une groupement alkyle comprenant de 1 à 4 atomes de carbone, ou un groupement phénylalkyle comprenant de 7 à 12 atomes de carbone, ou ledit groupement phénylalkyle substitué par un groupement alkyle comprenant de 1 à 4 atomes de carbone,
$E_4$ représente un atome d'hydrogène, un groupement alkyle comprenant de 1 à 30 atomes de carbone, un groupement phényle, naphtyle, ou phénylalkyle comprenant de 7 à 12 atomes de carbone ou,
$E_3$ et $E_4$ représentent ensemble un groupement polyméthylène de 4 à 17 atomes de carbone, ou ledit polyméthylène substitué par jusqu'à 4 groupements alkyle comprenant de 1 à 4 atomes de carbone ;
$R_2$ de formule (N) est comme défini précédemment lorsque m vaut 1 ;
$G_1$ est une liaison directe, un groupement alkylène en $C_1$-$C_{12}$, phénylène ou -NH-G'-NH dans lequel G' est un groupement alkylène en $C_1$-$C_{12}$.

**3.** Composition selon la revendication 2 qui contient un composé de formule A, B, D, J, K ou M, dans laquelle R est un atome d'hydrogène, et $T_5$ et $T_6$ représentent un groupement méthyle.

**4.** Composition selon la revendication 2 qui contient un composé de formule A dans laquelle R est un atome d'hydrogène,
$R_1$ est un groupe D-CO- et D représente un groupement alkyle en $C_1$-$C_{12}$, alkoxy en $C_1$-$C_4$, phényle, amino ou amino monosubstitué par un groupement alkyle en $C_1$-$C_{12}$, ou phényle ;
m vaut 1, 2 ou 4 et lorsque m vaut 1,
$R_2$ est un radical acyle d'un acide carboxylique aliphatique en $C_2$-$C_{18}$, d'un acide carboxylique cycloaliphatique en $C_6$-$C_{12}$ ou d'un acide carboxylique aromatique en $C_7$-$C_{15}$ et lorsque m vaut 2, $R_2$ est un radical acyle divalent d'un acide dicarboxylique aliphatique en $C_2$-$C_{18}$ ou d'un acide dicarboxylique cycloaliphatique ou aromatique en $C_8$-$C_{14}$, ou d'un acide dicarbamique aliphatique, cycloaliphatique ou aromatique en $C_8$-$C_{14}$, ou $R_2$ est un groupement

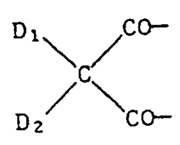

dans lequel $D_1$ représente un groupement alkyle en $C_1$-$C_8$ ou 3,5-di-tert-butyl-4-hydroxybenzyle et $D_2$ est identique à $D_1$ ou représente un atome d'hydrogène et lorsque m vaut 4, $R_2$ est un radical acyle tétravalent d'un acide butane- ou pentane-tétracarboxylique.

**5.** Composition selon la revendication 2 qui contient un composé de formule B dans laquelle R est un atome d'hydrogène,
$R_1$ est un groupe D-CO- et D représente un groupement alkyle en $C_1$-$C_{12}$, alkoxy en $C_1$-$C_4$, phényle, amino ou amino monosubstitué par un groupement alkyle en $C_1$-$C_{12}$, ou phényle ,
p vaut 1 ou 2, $R_3$ représente un atome d'hydrogène, un groupement alkyle en $C_1$-$C_{12}$, alkanoyle en $C_2$-

$C_{12}$ et lorsque p vaut 1, $R_4$ est un groupement alkyle en $C_1$-$C_{12}$, cycloalkyle en $C_5$-$C_7$ ou un groupe de formule I, et lorsque p vaut 2, $R_4$ est un groupement alkylène en $C_2$-$C_8$, phénylène ou xylylène, et si $R_3$ n'est pas un groupement alkanoyle, $R_4$ peut aussi être un résidu acyle divalent d'un acide dicarboxylique aliphatique en $C_2$-$C_{10}$ ou d'un acide dicarboxylique aromatique en $C_6$ ou d'un acide dicarbamique aliphatique ou aromatique en $C_8$-$C_{15}$.

**6.** Composition selon la revendication 1, qui contient du di-(1-acétoxy-2,2,6,6-tétraméthylpipéridin-4-yl)-sébaçate.

**7.** Composition selon la revendication 3, qui contient de l'α,α-(di-1-éthoxy-2,2,6,6-tétraméthylpipéridin-4-yloxy)-p-xylène.

**8.** Composition selon la revendication 3, qui contient de la 1,4-dibenzyloxy-2,2,6,6-tétraméthylpipéridine.

**9.** Composition selon la revendication 3, qui contient du di-(1-benzyloxy-2,2,6,6-tétraméthylpipéridin-4-yl)-sébaçate.

**10.** Composition selon la revendication 3, qui contient de la 4-benzyloxy-1-éthoxy-2,2,6,6-tétraméthylpipéridine.

**11.** Composition selon la revendication 3, qui contient de la 1,4-di-(4-hydroxy-3,5-di-tert-butylbenzoyloxy)-2,2,6,6-tétraméthylpipéridine.

**12.** Composition selon la revendication 3, qui contient de l'α,α-(di-1-benzoyloxy-2,2,6,6-tétraméthylpipéridin-4-yloxy)-p-xylène.

**13.** Composition de revêtement selon la revendication 1, qui est un système durcissable à température ambiante à base de résine alkyde, de résine acrylique themoplastique, de résine alkyde acrylique, de résine de polyuréthane ou de résine polyester, ou desdites résines modifiées avec des silicones, isocyanates, époxydes, isocyanurates, cétimines ou oxazolidines, ou bien encore le système est à base d'ester cellulosique ou de résine époxyde.

**14.** Composition de revêtement selon la revendication 1, qui est un système thermodurcissable catalysé par un acide à base de résine réticulable à chaud de type acrylique, polyester, polyuréthane, polyamide ou alkyde.

**15.** Composition selon la revendication 1, qui est un vernis industriel de finition.

**16.** Composition selon la revendication 1, qui est un vernis de retouche pour les automobiles.

**17.** Composé correspondant à l'une des formules A'-N'

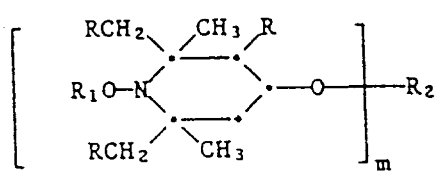

(A')

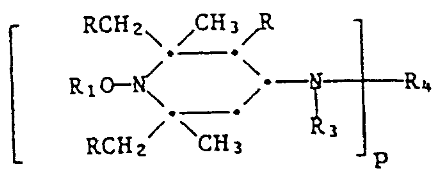

(B')

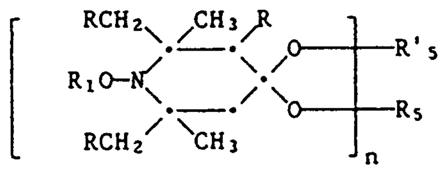

(C')

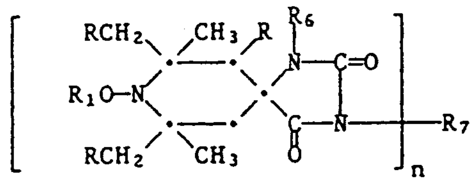

(D')

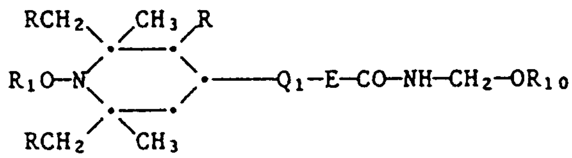

(E')

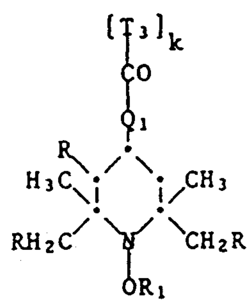

(F')

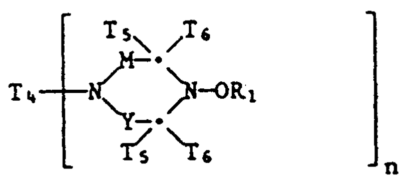

(G')

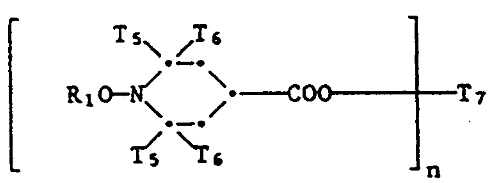

(H')

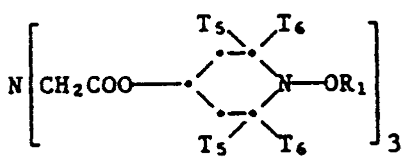

(I')

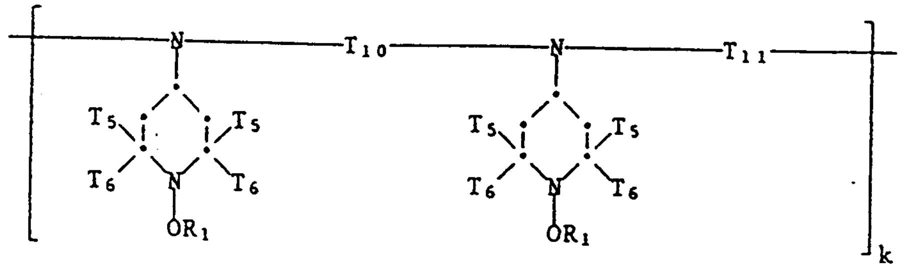

(J')

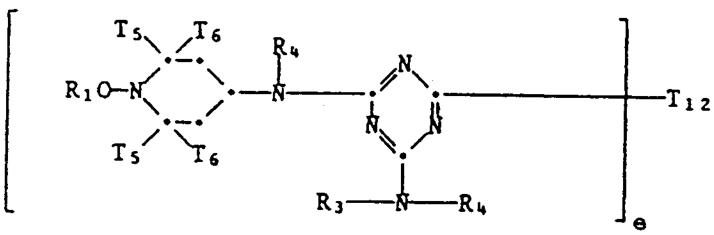

(K')

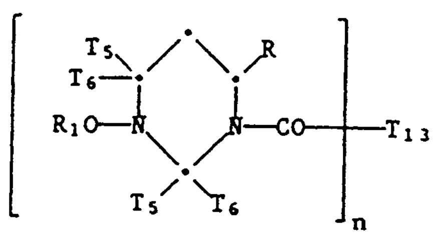

(L')

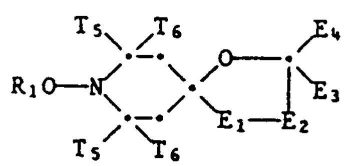

(M')

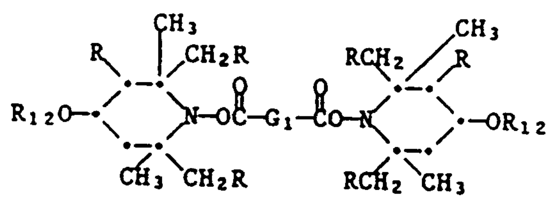

(N')

dans laquelle

R est un atome d'hydrogène ou un groupement méthyle, de préférence un atome d'hydrogène ;

$R_1$ est un groupement D-CO-, dans lequel D est un groupement alkyle en $C_1$-$C_{18}$, alkoxy en $C_1$-$C_{18}$, phényle, phényle substitué par un groupement hydroxy, alkyle en $C_1$-$C_4$ ou alkoxy en $C_1$-$C_4$, ou un groupement amino ou amino mono- ou di-substitué par un groupement alkyle en $C_1$-$C_{12}$, ou phényle ;

m vaut 1-4,

lorsque m vaut 1,

$R_2$ est un groupement

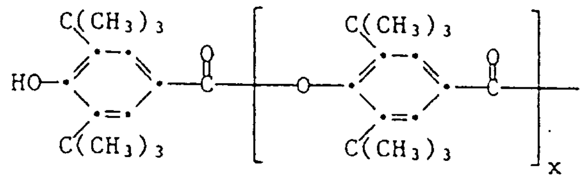

dans lequel x vaut 0 ou 1, ou $R_2$ est un groupement

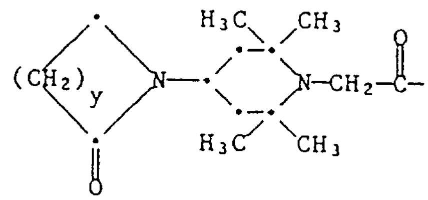

dans lequel y vaut 2-4 ;
lorsque m vaut 2 et que D est un groupement alkyle,
$R_2$ est un groupement alkylène en $C_1$-$C_{12}$, alkénylène en $C_4$-$C_{12}$, xylylène, un radical acyle divalent d'un acide dicarboxylique cycloaliphatique, araliphatique ou aromatique ayant 8 à 14 atomes de carbone, ou d'un acide dicarbamique aliphatique, cycloaliphatique ou aromatique ayant de 8 à 14 atomes de carbone ; ou $R_2$ est un groupement

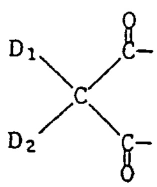

ou

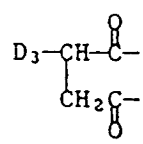

dans lequel $D_1$ représente un groupement alkyle comprenant jusqu'à 8 atomes de carbone, un groupement benzyle ou 3,5-di-t-butyl-4-hydroxybenzyle, $D_2$ est identique à $D_1$ ou représente un atome d'hydrogène, $D_3$ représente un groupement alkyle en $C_1$-$C_{18}$ ou alkényle en $C_2$-$C_{18}$ ;
lorsque m vaut 2 et que D est un groupement phényle ou phényle substitué, amino, amino substitué ou alkoxy,
$R_2$ est un groupement alkylène en $C_1$-$C_{12}$, alkénylène en $C_4$-$C_{12}$, xylylène, un radical acyle divalent d'un acide dicarboxylique ou dicarbamique aliphatique, cycloaliphatique, araliphatique ou aromatique comportant jusqu'à 14 atomes de carbone ; ou $R_2$ est un groupement

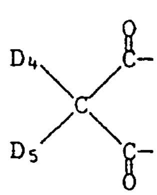

ou

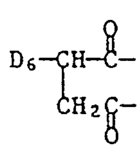

dans lequel $D_4$ et $D_5$ représentent indépendamment un atome d'hydrogène, un radical alkyle comprenant jusqu'à 8 atomes de carbone, un groupement benzyle ou 3,5-di-t-butyl-4-hydroxybenzyle et
$D_6$ représente un groupement alkyle en $C_1$-$C_{18}$ ou alkényle en $C_2$-$C_{18}$ ;
lorsque m vaut 3, $R_2$ est un radical acyle trivalent d'un acide tricarboxylique aliphatique, cycloaliphatique ou aromatique comportant jusqu'à 12 atomes de carbone ;
lorsque m vaut 4, $R_2$ est un radical acyle tétravalent d'un acide tétracarboxylique aliphatique ou aromatique saturé ou insaturé comportant jusqu'à 18 atomes de carbone ;

p vaut 1, 2 ou 3,
$R_3$ représente un atome d'hydrogène, un groupement alkyle en $C_1$-$C_{12}$, cycloalkyle en $C_5$-$C_7$, aralkyle en $C_7$-$C_9$, alkanoyle en $C_2$-$C_{18}$, alkénoyle en $C_3$-$C_5$, ou benzoyle ;
lorsque p vaut 1,
$R_4$ représente un atome d'hydrogène, un groupement alkyle en $C_1$-$C_{18}$, cycloalkyle en $C_5$-$C_7$, alkényle en $C_2$-$C_8$ non substitué ou substitué par un groupement cyano, carbonyle ou carbamide, ou représente un groupement aryle, aralkyle, glycidyle, un groupement de formule $-CH_2-CH(OH)-Z$ ou de formule -CONH-Z dans laquelle Z représente un atome d'hydrogène, un groupement méthyle ou phényle ; ou $R_4$ est un groupement de formule

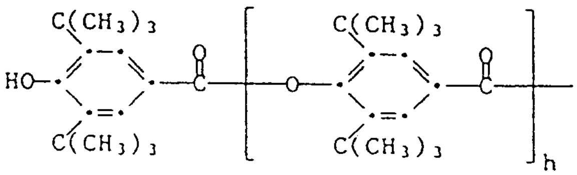 avec h valant 0 ou 1;

ou un groupement de formule I

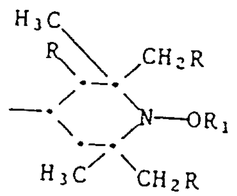

(I) ;

ou $R_3$ et $R_4$ pris ensemble forment un groupement alkylène comprenant de 4 à 6 atomes de carbone, ou le radical acyle divalent d'un acide 1,2- ou 1,3-dicarboxylique aliphatique ou aromatique ;
lorsque p vaut 2,
$R_4$ est un groupement alkylène en $C_1$-$C_{12}$, arylène en $C_6$-$C_{12}$, xylylène, un groupement $-CH_2CH(OH)-CH_2$ ou un groupement $-CH_2-CH(OH)-CH_2-O-X-O-CH_2-CH(OH)-CH_2-$ dans lequel X représente un groupement alkylène en $C_2$-$C_{10}$, arylène en $C_6$-$C_{15}$ ou cycloalkylène en $C_6$-$C_{12}$ ; ou, à condition que $R_3$ ne soit pas un groupement alkanoyle, alkénoyle ou benzoyle, $R_4$ peut aussi être un radical acyle divalent d'un acide dicarbamique ou dicarboxylique aliphatique, cycloaliphatique ou aromatique comprenant jusqu'à 14 atomes de carbone, ou peut être le groupe -CO- ; ou $R_4$ représente un groupement de formule II

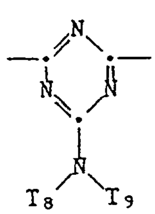

(II)

dans laquelle $T_8$ et $T_9$ représentent indépendamment un atome d'hydrogène, un groupement alkyle de 1 à 18 atomes de carbone, ou $T_8$ et $T_9$ forment ensemble un groupement alkylène de 4 à 6 atomes de carbone ou 3-oxapentaméthylène ;
lorsque p vaut 3, $R_4$ représente un groupement 2,4,6-triazinetriyle ;
n vaut 1 ou 2 et lorsque n vaut 1,
$R_5$ et $R'_5$ représentent indépendamment un groupement alkyle en $C_1$-$C_{12}$, alkényle en $C_2$-$C_{12}$, aralkyle en $C_7$-$C_{12}$, ou $R_5$ représente aussi un atome d'hydrogène, ou $R_5$ et $R'_5$ forment ensemble un groupement alkylène en $C_2$-$C_8$, ou hydroxyalkylène ou acyloxyalkylène en $C_4$-$C_{22}$ ;
et lorsque n vaut 2,
$R_5$ et $R'_5$ représentent ensemble $(-CH_2)_2C(CH_2-)_2$ ;

$R_6$ représente un atome d'hydrogène, un groupement alkyle en $C_1$-$C_{12}$, allyle, benzyle, glycidyle ou alkoxyalkyle en $C_2$-$C_6$ ;

lorsque n vaut 1,

$R_7$ représente un atome d'hydrogène, un groupement alkyle en $C_1$-$C_{12}$, alkényle en $C_3$-$C_5$, aralkyle en $C_7$-$C_9$, cycloalkyle en $C_5$-$C_7$, hydroxyalkyle en $C_2$-$C_4$, alkoxyalkyle en $C_2$-$C_6$, aryle en $C_6$-$C_{10}$, glycidyle, un groupement de formule $-(CH_2)_t$-COO-Q ou de formule $-(CH_2)_t$-O-CO-Q dans laquelle t vaut 1 ou 2, et Q représente un groupement alkyle en $C_1$-$C_4$ ou phényle ; et

lorsque n vaut 2,

$R_7$ représente un groupement alkylène en $C_2$-$C_{12}$, arylène en $C_6$-$C_{12}$, un groupement $-CH_2CH(OH)-CH_2-O-X-O-CH_2-CH(OH)-CH_2-$ dans lequel X est un groupement alkylène en $C_2$-$C_{10}$, arylène en $C_6$-$C_{15}$ ou cycloalkylène en $C_6$-$C_{12}$, ou un groupement $-CH_2CH(OZ')CH_2-(OCH_2-CH(OZ')CH_2)_2-$ dans lequel Z' est un atome d'hydrogène, un groupement alkyle en $C_1$-$C_{18}$, allyle, benzyle, alkanoyle en $C_2$-$C_{12}$, ou benzoyle ;

$Q_1$ représente $-N(R_8)-$ ou -O- ; E est un groupement alkylène en $C_1$-$C_3$, le groupement $CH_2-CH(R_9)-O-$ dans lequel $R_9$ est un atome d'hydrogène, un groupement méthyle ou phényle, ou E est le groupement $-(CH_2)_3-NH-$ ou une liaison directe ;

$R_{10}$ est un atome d'hydrogène ou un groupement alkyle en $C_1$-$C_{18}$, $R_8$ est un atome d'hydrogène, un groupement alkyle en $C_1$-$C_{18}$, cycloalkyle en $C_5$-$C_7$, aralkyle en $C_7$-$C_{12}$, cyanoéthyle, aryle en $C_6$-$C_{10}$, le groupement $-CH_2-CH(R_9)-OH$ dans lequel $R_9$ a la signification définie ci-dessus ; un groupement de formule I ou un groupement de formule

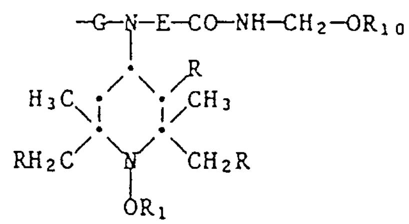

dans laquelle G est un groupement alkylène en $C_2$-$C_6$ ou arylène en $C_6$-$C_{12}$ ; ou $R_8$ est un groupe $-E-CO-NH-CH_2-OR_{10}$ ;

$T_3$ est un groupement éthylène ou 1,2-propylène, ou est l'unité structurale répétitive dérivée d'un copolymère d'alpha-oléfine avec un acrylate ou un méthacrylate d'alkyle ;

k vaut de 2 à 100 ;

$T_4$ a la même signification que $R_4$ lorsque p vaut 1 ou 2,

$T_5$ représente un groupement méthyle,

$T_6$ est un groupement méthyle ou éthyle, ou $T_5$ et $T_6$ forment ensemble un groupement tétraméthylène ou pentaméthylène,

M et Y représentent indépendamment un groupement méthylène ou carbonyle ;

$T_7$ est identique à $R_7$ ;

$T_{10}$ et $T_{11}$ représentent indépendamment un groupement alkylène de 2 à 12 atomes de carbone, ou $T_{11}$ représente un groupe de formule II ;

e vaut 2, 3 ou 4,

$T_{12}$ est un groupe

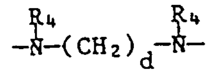

ou

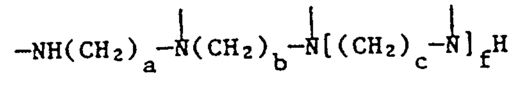

dans lequel a, b et c valent indépendamment 2 ou 3, d vaut de 2 à 10 et f vaut 0 ou 1 ;

$T_{13}$ est le même que $R_4$ à la différence que $R_{13}$ ne peut pas être un atome d'hydrogène lorsque n vaut 1 ;

$E_1$ et $E_2$, étant différents, représentent chacun un groupe -CO- ou -N($E_5$)-, dans lequel $E_5$ est un atome d'hydrogène, un groupement alkyle en $C_1$-$C_{12}$ ou alkoxycarbonylalkyle comportant de 4 à 22 atomes de carbone, de préférence $E_1$ représente un groupe -CO- et $E_2$ représente un groupe -N($E_5$)- ;
$E_3$ est un atome d'hydrogène, un groupement alkyle comprenant de 1 à 30 atomes de carbone, un groupement phényle, naphtyle, ledit groupement phényle ou ledit groupement naphtyle étant substitué par un atome de chlore ou par un groupement alkyle comprenant de 1 à 4 atomes de carbone, ou un groupement phénylalkyle comprenant de 7 à 12 atomes de carbone, ou ledit groupement phénylalkyle substitué par un groupement alkyle comprenant de 1 à 4 atomes de carbone,
$E_4$ représente un atome d'hydrogène, un groupement alkyle comprenant de 1 à 30 atomes de carbone, un groupement phényle, naphtyle, ou phénylalkyle comprenant de 7 à 12 atomes de carbone ou,
$E_3$ et $E_4$ forment ensemble un groupement polyméthylène comprenant de 4 à 17 atomes de carbone, ou ledit polyméthylène substitué par jusqu'à quatre groupements alkyle comprenant de 1 à 4 atomes de carbone, de préférence un groupe méthyle, et ;
$R_{12}$ est un groupement alkanoyle en $C_2$-$C_{18}$, alkénoyle en $C_3$-$C_6$, benzoyle ou benzoyle substitué par un groupement alkyle en $C_1$-$C_4$, halogène ou hydroxyle, et $G_1$ est une liaison directe, un groupement alkylène en $C_1$-$C_{12}$, phénylène ou -NH-G'-NH dans lequel G' est un groupement alkylène en $C_1$-$C_{12}$.

**18.** Composé selon la revendication 17, dans lequel D est un groupement alkyle en $C_1$-$C_{18}$, phényle ou phényle substitué par un groupement hydroxy, alkyle en $C_1$-$C_4$ ou alkoxy en $C_1$-$C_4$.

**19.** Composé selon la revendication 17, dans lequel D est un groupement amino ou amino mono ou di-substitué par un groupement alkyle en $C_1$-$C_{12}$ ou phényle.

**20.** Composé selon la revendication 17, dans lequel D est un groupement alkoxy en $C_1$-$C_{18}$.

**21.** Composé selon la revendication 17, dans lequel R est un atome d'hydrogène.

**22.** Composé selon la revendication 17, dans lequel $R_1$ est un groupement -CO-D- et D est un groupement alkyle en $C_1$-$C_{12}$, alkoxy en $C_1$-$C_4$, phényle, amino, alkylamino en $C_1$-$C_{12}$ ou phénylamino.

**23.** Composé selon la revendication 17 de formule A', B', D', F', J', K', M' ou N', dans laquelle R est un atome d'hydrogène,
$R_1$ est comme défini à la revendication 22,
m vaut 1, 2 ou 4,
lorsque m vaut 1, $R_2$ est un groupement

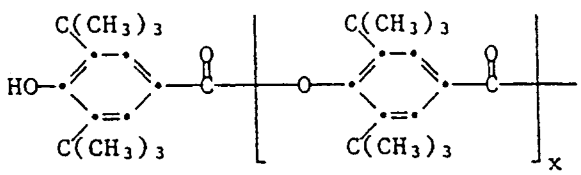

dans lequel x vaut 0 ou 1,
lorsque m vaut 2 et que D est un groupement alkyle,
$R_2$ est un radical acyle divalent d'un acide dicarboxylique cycloaliphatique ou aromatique ayant 8 à 14 atomes de carbone, ou d'un acide dicarbamique aliphatique, cycloaliphatique ou aromatique ayant de 8 à 14 atomes de carbone ; ou $R_2$ est un groupement

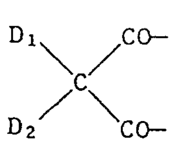

dans lequel $D_1$ représente un groupement alkyle en $C_1$-$C_4$ ou 3,5-di-t-butyl-4-hydroxybenzyle et $D_2$ est

identique à $D_1$ ou représente un atome d'hydrogène, et lorsque m vaut 2 et que D est un groupement phényle, amino, alkylamino, phénylamino ou alkoxy, $R_2$ est un radical acyle divalent d'un acide dicarboxylique aliphatique comportant de 2 à 8 atomes de carbone ou d'un acide dicarboxylique cycloaliphatique ou aromatique comportant de 8 à 14 atomes de carbone ou d'un acide dicarbamique aliphatique, cycloaliphatique ou aromatique comportant de 8 à 14 atomes de carbone, ou $R_2$ est un groupement

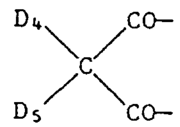

dans lequel $D_4$ et $D_5$ représentent indépendamment un atome d'hydrogène, un groupe alkyle en $C_1$-$C_8$, benzyle ou 3,5-di-t-butyl-4-hydroxybenzyle, et lorsque m vaut 4, $R_2$ est un radical acyle tétravalent d'acide 1,2,3,4-butanetétracarboxylique, d'acide 1,2,3,4-but-2-ènetétracarboxylique, d'acide 1,2,3,5- ou 1,2,4,5-pentanetétracarboxylique ;
p vaut 1, 2 ou 3,
$R_3$ représente un atome d'hydrogène, un groupement alkyle en $C_1$-$C_{12}$, cyclohexyle, aralkyle en $C_7$-$C_9$, alkanoyle en $C_2$-$C_{18}$, alkénoyle en $C_3$-$C_5$, ou benzoyle ;
lorsque p vaut 1,
$R_4$ représente un groupement alkyle en $C_1$-$C_{18}$, cyclohexyle, allyle, benzyle ou un groupe

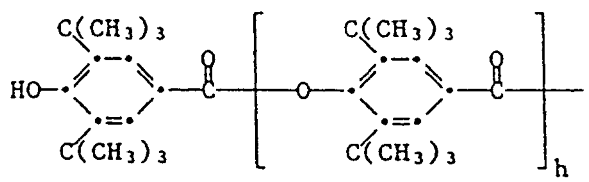

avec h valant 0 ou 1, ou représente un groupe

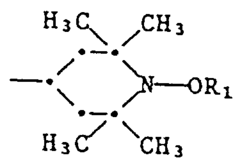

lorsque p vaut 2,
$R_4$ est un groupement alkylène en $C_1$-$C_{12}$, arylène en $C_6$-$C_{12}$, xylylène, un groupement $-CH_2CH(OH)-CH_2-$, ou, à condition que $R_3$ ne soit pas un groupement alkanoyle ou benzoyle, $R_4$ peut aussi être un radical acyle divalent d'un acide dicarbamique ou d'un acide dicarboxylique aliphatique, cycloaliphatique ou aromatique comprenant de 6 à 12 atomes de carbone,
ou $R_4$ représente un groupement de formule II,
dans laquelle $T_8$ et $T_9$ représentent indépendamment un atome d'hydrogène, un groupement alkyle de 1 à 12 atomes de carbone, ou $T_8$ et $T_9$ forment ensemble un groupement alkylène en $C_4$-$C_6$ ou 3-oxapentaméthylène,
lorsque p vaut 3, $R_4$ représente un groupement 2,4,6-triazinetriyle ;
n vaut 1 ou 2 et
$R_6$ représente un atome d'hydrogène, un groupement alkyle en $C_1$-$C_{12}$, allyle ou benzyle,
lorsque n vaut 1, $R_7$ représente un atome d'hydrogène, un groupement alkyle en $C_1$-$C_{12}$, cyclohexyle, allyle ou benzyle, et lorsque n vaut 2, $R_7$ représente un groupement alkylène en $C_2$-$C_{12}$ ;
k vaut de 5 à 50 ;
$T_5$ et $T_6$ représentent chacun un groupement méthyle,
$T_{10}$ et $T_{11}$ représentent indépendamment un groupement alkylène de 2 à 12 atomes de carbone, ou

$T_{11}$ représente un groupe de formule II ;
e vaut 4,
$T_{12}$ est un groupe

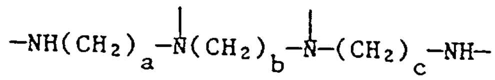

dans lequel a, b et c valent indépendamment 2 ou 3 ;
$E_1$ représente un groupe -CO- et $E_2$ représente un groupe -N($E_5$)- ; $E_3$ et $E_4$ représentent indépendamment un groupement alkyle en $C_1$-$C_{12}$ ou $E_3$ et $E_4$ forment ensemble un groupement polyméthylène en $C_5$-$C_{12}$, et $E_5$ représente un atome d'hydrogène ou un groupe alkyle en $C_1$-$C_{12}$ ;
$G_1$ est une liaison directe, un groupement alkylène en $C_2$-$C_8$ ou -NH-G'-NH dans lequel G' est un groupement alkylène en $C_2$-$C_8$, et $R_{12}$ représente un groupement alkanoyle en $C_2$-$C_{18}$ ou benzoyle.

**24.** Composé selon la revendication 17 de formule A', B', F', J' ou N', dans laquelle R est un atome d'hydrogène, $R_1$ est un groupement D-CO- et D est un groupement alkyle en $C_1$-$C_4$, alkoxy en $C_1$-$C_4$ , phényle, amino, alkylamino en $C_1$-$C_4$, ou phénylamino,
m vaut 1 ou 2 et
lorsque m vaut 1, $R_2$ est un groupement

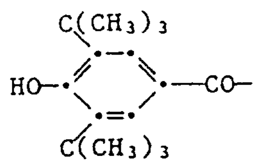

lorsque m vaut 2 et que D est un groupement alkyle, $R_2$ est un radical acyle divalent d'un acide phénylènedicarboxylique ou est un groupement -CO-C($D_1$)($D_2$)-CO- dans lequel $D_1$ représente un groupement alkyle en $C_1$-$C_4$ ou 3,5-di-t-butyl-4-hydroxybenzyle et $D_2$ est identique à $D_1$ ou représente un atome d'hydrogène, et
lorsque m vaut 2 et que D est un groupement alkoxy, phényle, amino ou alkylamino,
$R_2$ est un radical acyle divalent d'un acide dicarboxylique aliphatique ou aromatique comportant de 8 à 10 atomes de carbone ou d'un acide dicarbamique aliphatique comportant de 8 à 10 atomes de carbone, ou $R_2$ est un groupement

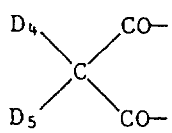

dans lequel $D_4$ représente un groupe alkyle ou 3,5-di-t-butyl-4-hydroxybenzyle, et $D_5$ est identique à $D_4$ ou représente un atome d'hydrogène ;
p vaut 1 ou 2, $R_3$ représente un groupement alkyle en $C_1$-$C_4$, acétyle ou benzoyle,
lorsque p vaut 1, $R_4$ représente un groupement alkyle en $C_1$-$C_{12}$ ou un groupe

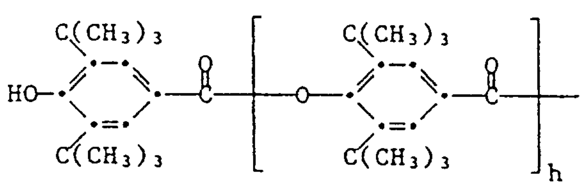

avec h valant 0 ou 1,

et lorsque p vaut 2, $R_4$ est un groupement alkylène en $C_4$-$C_8$ ;
k vaut de 5 à 50 ; $T_3$ représente un groupe éthylène et $Q_1$ représente O ;
$T_5$ et $T_6$ représentent un groupement méthyle,
$T_{10}$ représente un groupe hexaméthylène et $T_{11}$ représente un groupe de formule II
dans laquelle $T_8$ et $T_9$ représentent indépendamment un atome d'hydrogène, un groupement alkyle en $C_1$-$C_{12}$, ou $T_8$ et $T_9$ forment ensemble un groupement pentaméthylène ou 3-oxapentaméthylène ;
$R_{12}$ est un groupe acétyle ou benzoyle et $G_1$ est une liaison directe ou un groupement $-NH-(CH_2)_6-NH-$.

**25.** Utilisation d'un composé selon la revendication 17 en tant que stabilisant pour des produits organiques vis-à-vis de la dégradation actinique

**26.** Utilisation selon la revendication 25, comme stabilisant pour des polymères organiques.

**27.** Utilisation selon la revendication 25, comme stabilisant pour des couches photographiques.

**28.** Produit organique contenant une quantité efficace pour la stabilisation d'un composé selon la revendication 17.

**Revendications pour l'Etat contractant suivant : ES**

**1.** Une composition stabilisée de revêtement thermodurcissable catalysée par un acide ou durcissable à température ambiante contenant une quantité efficace pour la stabilisation d'un composé aminé à empêchement stérique contenant le groupement

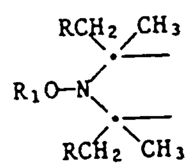

dans lequel R est un atome d'hydrogène ou un groupement méthyle et $R_1$ est un groupement

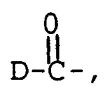

dans lequel D est un groupement alkyle en $C_1$-$C_{18}$, alkoxy en $C_1$-$C_{18}$, phényle, phényle substitué par un groupement hydroxy, alkyle en $C_1$-$C_4$ ou alkoxy en $C_1$-$C_4$, ou D est un groupement amino ou amino mono- ou di-substitué par un groupement alkyle en $C_1$-$C_{12}$ ou phényle.

**2.** La composition selon la Revendication 1, qui contient un composé aminé à empêchement stérique correspondant à l'une des formules A-N

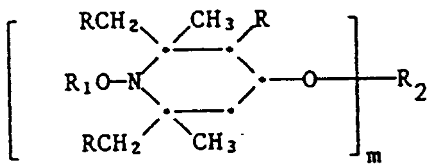

(A)

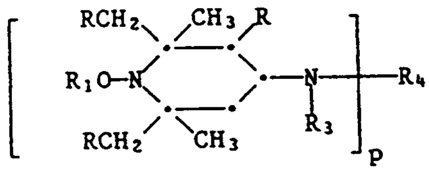

(B)

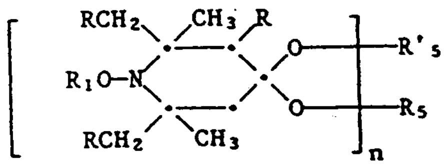

(C)

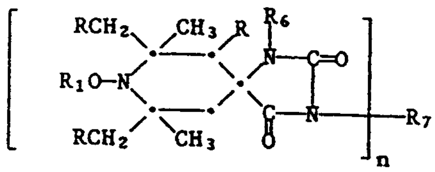

(D)

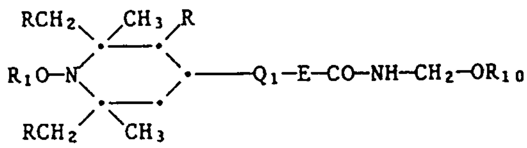

(E)

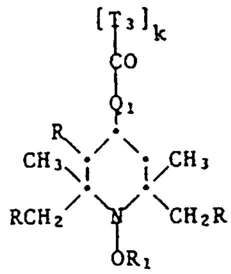

(F)

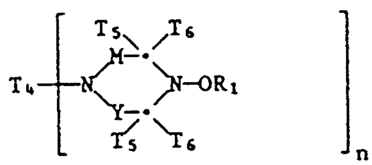

(G)

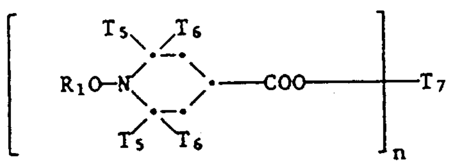

(H)

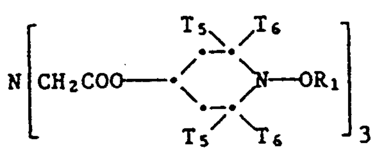

(I)

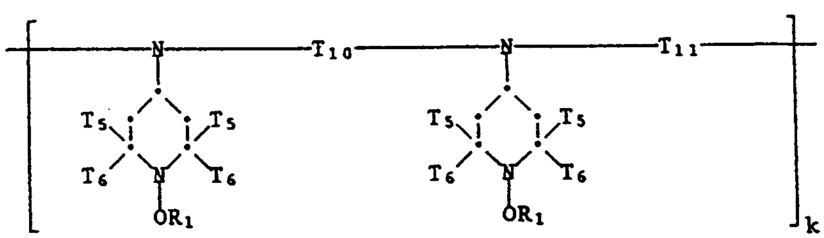

(J)

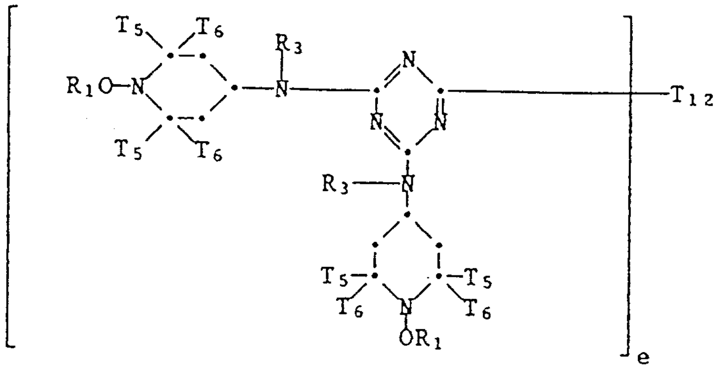

(K)

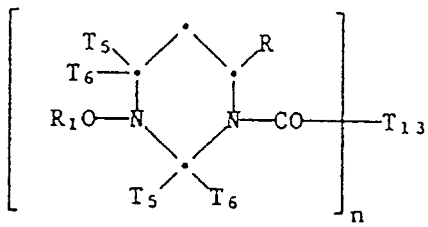

(L)

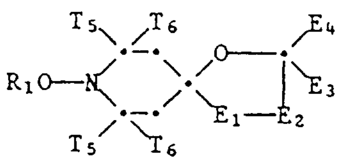

(M)

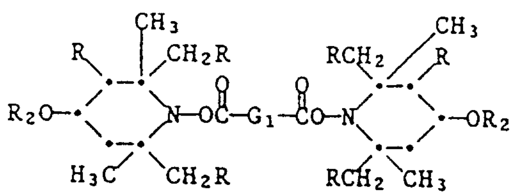

(N)

dans laquelle

R est un atome d'hydrogène ou un groupement méthyle,

$R_1$ est un groupement D-CO- dans lequel D est un groupement alkyle en $C_1$-$C_{18}$, alkoxy en $C_1$-$C_{18}$, phényle, phényle substitué par un groupement hydroxy, alkyle en $C_1$-$C_4$ ou alkoxy en $C_1$-$C_4$, ou un groupement amino ou amino mono- ou di-substitué par un groupement alkyle en $C_1$-$C_{12}$, ou phényle ;

m vaut 1-4,

lorsque m vaut 1,

$R_2$ est un atome d'hydrogène, un groupement alkyle en $C_1$-$C_{18}$, éventuellement interrompu par un ou plusieurs atomes d'oxygène, alkényle en $C_2$-$C_{12}$, aryle en $C_6$-$C_{10}$, aralkyle en $C_7$-$C_{18}$, glycidyle, un radical acyle monovalent d'un acide carboxylique aliphatique, cycloaliphatique, araliphatique ou aromatique, ou d'un acide carbamique, ou $R_2$ est un groupement

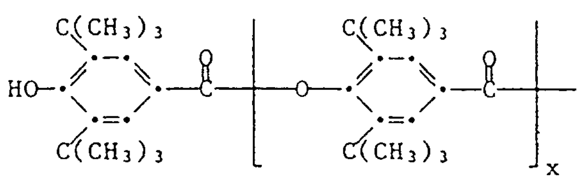

dans lequel x vaut 0 ou 1, ou est un groupement

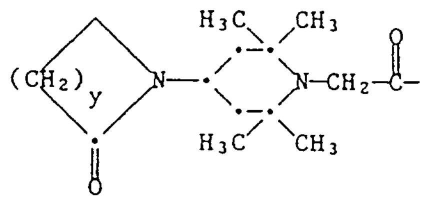

dans lequel y vaut 2-4 ;

lorsque m vaut 2,

$R_2$ est un groupement alkylène en $C_1$-$C_{12}$, alkénylène en $C_4$-$C_{12}$, xylylène, un radical acyle divalent d'un acide dicarboxylique aliphatique, cycloaliphatique, araliphatique ou aromatique ou d'un acide dicarbamique, ou $R_2$ est un groupement

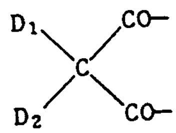

ou

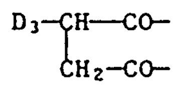

dans lequel $D_1$ et $D_2$ représentent indépendamment un atome d'hydrogène, un radical alkyle comprenant jusqu'à 8 atomes de carbone, un groupement phényle, benzyle ou 3,5-di-t-butyl-4-hydroxybenzyle et $D_3$ est un radical alkyle ou alkényle contenant jusqu'à 18 atomes de carbone ; lorsque m vaut 3, $R_2$ est un radical acyle trivalent d'un acide tricarboxylique aliphatique, cycloaliphatique ou aromatique ; lorsque m vaut 4, $R_2$ est un radical acyle tétravalent d'un acide tétracarboxylique aliphatique ou aromatique saturé ou insaturé ; p vaut 1, 2 ou 3,

$R_3$ représente un atome d'hydrogène, un groupement alkyle en $C_1$-$C_{12}$, cycloalkyle en $C_5$-$C_7$, aralkyle en $C_7$-$C_9$, alkanoyle en $C_2$-$C_{18}$, alkénoyle en $C_3$-$C_5$, ou benzoyle ;

lorsque p vaut 1,

$R_4$ représente un atome d'hydrogène, un groupement alkyle en $C_1$-$C_{18}$, cycloalkyle en $C_5$-$C_7$, alkényle en $C_2$-$C_8$ non substitué ou substitué par un groupement cyano, carbonyle ou carbamide, ou représente un groupement aryle, aralkyle, glycidyle, un groupement de formule $-CH_2-CH(OH)-Z$ ou de formule $-CONH-Z$ dans laquelle Z représente un atome d'hydrogène, un groupement méthyle ou phényle ;

ou $R_4$ est un groupement de formule I

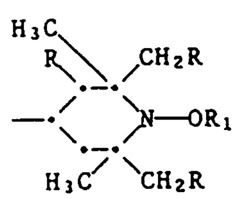

(I) ou

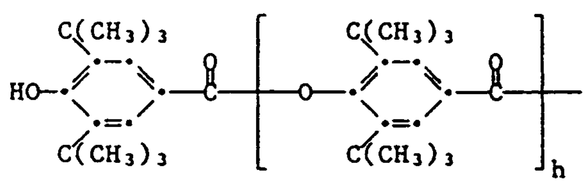

avec h valant 0 ou 1;

ou $R_3$ et $R_4$ pris ensemble sont un groupement alkylène possédant de 4 à 6 atomes de carbone, ou 1-oxo-alkylène ou le radical acyle divalent d'un acide 1,2- ou 1,3-dicarboxylique aliphatique ou aromatique ;

lorsque p vaut 2,
$R_4$ est un groupement alkylène en $C_1$-$C_{12}$, arylène en $C_6$-$C_{12}$, xylylène, un groupement $-CH_2CH(OH)-CH_2$, ou un groupement $-CH_2-CH(OH)-CH_2-O-X-O-CH_2-CH(OH)-CH_2-$ dans lequel X représente un groupement alkylène en $C_2$-$C_{10}$, arylène en $C_6$-$C_{15}$ ou cycloalkylène en $C_6$-$C_{12}$ ou, à condition que $R_3$ ne soit pas un groupement alkanoyle, alkénoyle ou benzoyle, $R_4$ peut aussi être un radical acyle divalent d'un acide dicarbamique ou dicarboxylique aliphatique, cycloaliphatique ou aromatique, ou peut être le groupe -CO- ; ou $R_4$ représente un groupement de formule II

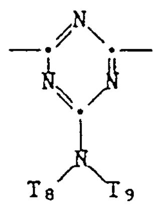
(II)

dans laquelle $T_8$ et $T_9$ représentent indépendamment un atome d'hydrogène, un groupement alkyle de 1 à 18 atomes de carbone, ou $T_8$ et $T_9$ représentent ensemble un groupement alkylène de 4 à 6 atomes de carbone ou 3-oxapentaméthylène, $T_8$ et $T_9$ représentant de préférence conjointement un groupement 3-oxapentaméthylène ;
lorsque p vaut 3, $R_4$ représente un groupement 2,4,6-triazinetriyle ;
n vaut 1 ou 2 et
lorsque n vaut 1,
$R_5$ et $R'_5$ représentent indépendamment un groupement alkyle en $C_1$-$C_{12}$, alkényle en $C_2$-$C_{12}$, aralkyle en $C_7$-$C_{12}$, ou $R_5$ représente aussi un atome d'hydrogène ou $R_5$ et $R'_5$ représentent ensemble un groupement alkylène en $C_2$-$C_8$, ou hydroxyalkylène ou (acyle en $C_4$-$C_{22}$)oxyalkylène ;
lorsque n vaut 2, $R_5$ et $R'_5$ représentent ensemble $(-CH_2)_2C(CH_2-)_2$ ;
$R_6$ représente un atome d'hydrogène, un groupement alkyle en $C_1$-$C_{12}$, allyle, benzyle, glycidyle ou alkoxyalkyle en $C_2$-$C_6$ ; lorsque n vaut 1,
$R_7$ représente un atome d'hydrogène, un groupement alkyle en $C_1$-$C_{12}$, alkényle en $C_3$-$C_5$, aralkyle en $C_7$-$C_9$, cycloalkyle en $C_5$-$C_7$, hydroxyalkyle en $C_2$-$C_4$, alkoxyalkyle en $C_2$-$C_6$, aryle en $C_6$-$C_{10}$, glycidyle, un groupement de formule $-(CH_2)_t-COO-Q$ ou de formule $-(CH_2)_t-O-CO-Q$ dans laquelle t vaut 1 ou 2, et Q représente un groupement alkyle en $C_1$-$C_4$, ou phényle ; ou lorsque n vaut 2,
$R_7$ représente un groupement alkylène en $C_2$-$C_{12}$, arylène en $C_6$-$C_{12}$, un groupement $-CH_2CH(OH)-CH_2-O-X-O-CH_2-CH(OH)-CH_2-$ dans lequel X est un groupement alkylène en $C_2$-$C_{10}$, arylène en $C_6$-$C_{15}$ ou cycloalkylène en $C_6$-$C_{12}$, ou un groupement $-CH_2CH(OZ')CH_2-(OCH_2-CH(OZ')CH_2)_2-$ dans lequel Z' est un atome d'hydrogène, un groupement (alkyle en $C_1$-$C_{18}$), allyle, benzyle, (alkanoyle en $C_2$-$C_{12}$), ou benzoyle ;
$Q_1$ représente $-N(R_8)-$ ou -O- ;
E est un groupement alkylène en $C_1$-$C_3$, le groupement $CH_2-CH(R_9)-O-$ dans lequel $R_9$ est un atome d'hydrogène, un groupement méthyle ou phényle, ou E est le groupement $-(CH_2)_3-NH-$ ou une liaison directe ;
$R_{10}$ est un atome d'hydrogène ou un groupement alkyle en $C_1$-$C_{18}$,
$R_8$ est un atome d'hydrogène, un groupement alkyle en $C_1$-$C_{18}$, cycloalkyle en $C_5$-$C_7$, aralkyle en $C_7$-$C_{12}$, cyanoéthyle, aryle en $C_6$-$C_{10}$, le groupement $-CH_2-CH(R_9)-OH$ dans lequel $R_9$ a la signification définie ci-dessus ; un groupement de formule I ou un groupement de formule

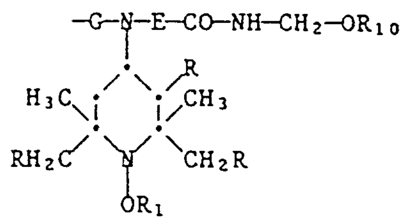

dans laquelle G peut être un groupement alkylène en $C_2$-$C_6$ ou arylène en $C_6$-$C_{12}$ ; ou $R_8$ est un

groupe $-E-CO-NH-CH_2-OR_{10}$ ;
$T_3$ est un groupement éthylène ou 1,2-propylène, ou est l'unité structurale répétitive dérivée d'un copolymère d'alpha-oléfine avec un acrylate ou un méthacrylate d'alkyle ;
k vaut de 2 à 100 ;
$T_4$ a la même signification que $R_4$ lorsque p vaut 1 ou 2,
$T_5$ représente un groupement méthyle,
$T_6$ est un groupement méthyle ou éthyle,
M et Y représentent indépendamment un groupement méthylène ou carbonyle ;
$T_7$ est identique à $R_7$ ;
$T_{10}$ et $T_{11}$ représentent indépendamment un groupement alkylène de 2 à 12 atomes de carbone, ou $T_{11}$ représente un groupe de formule II ;
e vaut 2, 3 ou 4 et
$T_{12}$ est un groupe $-N(R_5)-(CH_2)_d-N(R_5)-$ ou $-NH(CH_2)_a-N(CH_2)_b-N[(CH_2)_c-N]_fH$ dans lequel a, b et c valent indépendamment 2 ou 3, d vaut de 2 à 10 et f vaut 0 ou 1 ;
$T_{13}$ est le même que $R_4$ à condition que $R_{13}$ ne puisse pas être un hydrogène lorsque n vaut 1 ;
$E_1$ et $E_2$, étant différents, représentent chacun -CO- ou $-N(E_5)-$, dans lequel $E_5$ est un atome d'hydrogène, un groupement alkyle en $C_1$-$C_{12}$ ou alkoxycarbonylalkyle en $C_4$-$C_{22}$, $E_3$ est un groupement hydrogène, un groupement alkyle de 1 à 30 atomes de carbone, phényle, naphtyle, ledit groupement phényle ou ledit groupement naphtyle étant substitués par un atome de chlore ou par une groupement alkyle comprenant de 1 à 4 atomes de carbone, ou un groupement phénylalkyle comprenant de 7 à 12 atomes de carbone, ou ledit groupement phénylalkyle substitué par un groupement alkyle comprenant de 1 à 4 atomes de carbone,
$E_4$ représente un atome d'hydrogène, un groupement alkyle comprenant de 1 à 30 atomes de carbone, un groupement phényle, naphtyle, ou phénylalkyle comprenant de 7 à 12 atomes de carbone ou,
$E_3$ et $E_4$ représentent ensemble un groupement polyméthylène de 4 à 17 atomes de carbone, ou ledit polyméthylène substitué par jusqu'à 4 groupements alkyle comprenant de 1 à 4 atomes de carbone ;
$R_2$ de formule (N) est comme défini précédemment lorsque m vaut 1 ;
$G_1$ est une liaison directe, un groupement alkylène en $C_1$-$C_{12}$, phénylène ou -NH-G'-NH dans lequel G' est un groupement alkylène en $C_1$-$C_{12}$.

3. Composition selon la revendication 2 qui contient un composé de formule A, B, D, J, K ou M, dans laquelle R est un atome d'hydrogène, et $T_5$ et $T_6$ représentent un groupement méthyle.

4. Composition selon la revendication 2 qui contient un composé de formule A dans laquelle R est un atome d'hydrogène,
$R_1$ est un groupe D-CO- et D représente un groupement alkyle en $C_1$-$C_{12}$, alkoxy en $C_1$-$C_4$, phényle, amino ou amino monosubstitué par un groupement alkyle en $C_1$-$C_{12}$, ou phényle ;
m vaut 1, 2 ou 4 et lorsque m vaut 1,
$R_2$ est un radical acyle d'un acide carboxylique aliphatique en $C_2$-$C_{18}$, d'un acide carboxylique cycloaliphatique en $C_6$-$C_{12}$ ou d'un acide carboxylique aromatique en $C_7$-$C_{15}$ et lorsque m vaut 2, $R_2$ est un radical acyle divalent d'un acide dicarboxylique aliphatique en $C_2$-$C_{18}$ ou d'un acide dicarboxylique cycloaliphatique ou aromatique en $C_8$-$C_{14}$, ou d'un acide dicarbamique aliphatique, cycloaliphatique ou aromatique en $C_8$-$C_{14}$, ou $R_2$ est un groupement

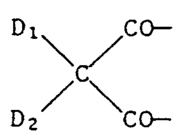

dans lequel $D_1$ représente un groupement alkyle en $C_1$-$C_8$ ou 3,5-di-tert-butyl-4-hydroxybenzyle et $D_2$ est identique à $D_1$ ou représente un atome d'hydrogène et lorsque m vaut 4, $R_2$ est un radical acyle tétravalent d'un acide butane- ou pentane-tétracarboxylique.

5. Composition selon la revendication 2 qui contient un composé de formule B dans laquelle R est un atome d'hydrogène,
$R_1$ est un groupe D-CO- et D représente un groupement alkyle en $C_1$-$C_{12}$, alkoxy en $C_1$-$C_4$, phényle, amino ou amino monosubstitué par un groupement alkyle en $C_1$-$C_{12}$, ou phényle ,

p vaut 1 ou 2, $R_3$ représente un atome d'hydrogène, un groupement alkyle en $C_1$-$C_{12}$, alkanoyle en $C_2$-$C_{12}$ et lorsque p vaut 1, $R_4$ est un groupement alkyle en $C_1$-$C_{12}$, cycloalkyle en $C_5$-$C_7$ ou un groupe de formule I, et lorsque p vaut 2, $R_4$ est un groupement alkylène en $C_2$-$C_8$, phénylène ou xylylène, et si $R_3$ n'est pas un groupement alkanoyle, $R_4$ peut aussi être un résidu acyle divalent d'un acide dicarboxylique aliphatique en $C_2$-$C_{10}$ ou d'un acide dicarboxylique aromatique en $C_6$ ou d'un acide dicarbamique aliphatique ou aromatique en $C_8$-$C_{15}$.

**6.** Composition selon la revendication 1, qui contient du di-(1-acétoxy-2,2,6,6-tétraméthylpipéridin-4-yl)-sébaçate.

**7.** Composition selon la revendication 3, qui contient de l'α,α-(di-1-éthoxy-2,2,6,6-tétraméthylpipéridin-4-yloxy)-p-xylène.

**8.** Composition selon la revendication 3, qui contient de la 1,4-dibenzyloxy-2,2,6,6-tétraméthylpipéridine.

**9.** Composition selon la revendication 3, qui contient du di-(1-benzyloxy-2,2,6,6-tétraméthylpipéridin-4-yl)-sébaçate.

**10.** Composition selon la revendication 3, qui contient de la 4-benzyloxy-1-éthoxy-2,2,6,6-tétraméthylpipéridine.

**11.** Composition selon la revendication 3, qui contient de la 1,4-di-(4-hydroxy-3,5-di-tert-butylbenzoyloxy)-2,2,6,6-tétraméthylpipéridine.

**12.** Composition selon la revendication 3, qui contient de l'α,α-(di-1-benzoyloxy-2,2,6,6-tétraméthylpipéridin-4-yloxy)-p-xylène.

**13.** Composition de revêtement selon la revendication 1, qui est un système durcissable à température ambiante à base de résine alkyde, de résine acrylique themoplastique, de résine alkyde acrylique, de résine de polyuréthane ou de résine polyester, ou desdites résines modifiées avec des silicones, isocyanates, époxydes, isocyanurates, cétimines ou oxazolidines, ou bien encore le système est à base d'ester cellulosique ou de résine époxyde.

**14.** Composition de revêtement selon la revendication 1, qui est un système thermodurcissable catalysé par un acide à base de résine réticulable à chaud de type acrylique, polyester, polyuréthane, polyamide ou alkyde.

**15.** Composition selon la revendication 1, qui est un vernis industriel de finition.

**16.** Composition selon la revendication 1, qui est un vernis de retouche pour les automobiles.